# EUROPEAN PATENT SPECIFICATION

(11) **EP 2 001 459 B1**
(45) Date of publication and mention of the grant of the patent: **11.07.2012**
(21) Application number: 07754313.0
(22) Date of filing: 28.03.2007
(51) Int. Cl.: A61K 39/21, C12N 7/00, C07K 14/16, C12N 15/53

(54) **METHODS AND COMPOSITIONS FOR INDUCING AN IMMUNE RESPONSE TO HIV AND MODELS FOR TESTING**
ZUSAMMENSETZUNGEN UND VERFAHREN ZUR INDUZIERUNG EINER IMMUNREAKTION GEGEN HIV UND TESTMODELLE
PROCÉDÉS ET COMPOSITIONS PERMETTANT L'INDUCTION D'UNE RÉPONSE IMMUNITAIRE VIS-A-VIS DU VIH ET MODÈLES POUR LE TEST

(30) Priority: 29.03.2006 US 787270 P
(43) Date of publication of application: 17.12.2008
(73) Proprietor: Dana-Farber Cancer Institute, Inc., Boston, MA 02115 (US)
(72) Inventor: RUPRECHT, Ruth, Boston, MA 02115 (US)
(74) Representative: Lloyd, John Scott
(86) International application number: PCT/US2007/007774
(87) International publication number: WO 2007/126959

(56) References cited:
- WO-A2-2005/028625
- BURES RENATA ET AL: "Regional clustering of shared neutralization determinants on primary isolates of clade C human immunodeficiency virus type 1 from South Africa" March 2002 (2002-03), JOURNAL OF VIROLOGY, VOL. 76, NR. 5, PAGE(S) 2233-2244 , XP002581888 ISSN: 0022-538X * the whole document *
- SONG R J ET AL: "Molecularly cloned SHIV-1157ipd3N4: a highly replication-competent, mucosally transmissible R5 simian-human immunodeficiency virus encoding HIV clade C env" September 2006 (2006-09), JOURNAL OF VIROLOGY, VOL. 80, NR. 17, PAGE(S) 8729-8738 , XP002581889 ISSN: 0022-538X
- LIAN Y. ET AL.: 'Evaluation of Envelope Vaccines Derived from the South African Subtype C Human Immunodeficiency Virus Type 1 TV1 Strain' JOURNAL OF VIROLOGY vol. 79, no. 21, November 2005, pages 13338 - 13349, XP002426835
- WYATT R. ET AL.: 'Functional and Immunologic Characterization of Human immunodeficiency Virus Type 1 Envelope Glycoproteins Containing Deletions of the Major Variable Regions' JOURNAL OF VIROLOGY vol. 67, no. 8, August 1993, pages 4557 - 4565, XP000943571
- NKOLOLA J.P. ET AL.: 'Progress towards an HIV-1 subtype C vaccine' VACCINE vol. 24, 2006, pages 391 - 401, XP005225977

### SEQUENCE LISTING

<110> DANA FARBER CANCER INSTITUTE, INC.
<120> METHODS AND COMPOSITIONS FOR INDUCING AN IMMUNE RESPONSE TO HIV AND MODELS FOR TESTING
<130> 207032-67925
<140>
   <141>
<150> 60/787,270
   <151> 2006-03-29
<160> 16
<170> PatentIn Ver. 3.3
   <210> 1
   <211> 2547
   <212> DNA
   <213> Artificial Sequence
<220>
   <223> Description of Artificial Sequence: Synthetic nucleotide construct
<400> 1
   atgcgcgtga aggagaagta ccagcacctg tggcgctggg gctggcgctg gggcaccatg 60
   ctgctgggca tgctgatgat ctgctccgcc accgagaagc tgtgggtgac cgtgtactac 120
   ggcgtgcccg tgtggaagga ggccaagacc accctgttct gcgcctccga cgccaaggcc 180
   tacgagaagg aggtgcacaa catctgggcc acccacgcct gcgtgcccac cgaccccaac 240
   ccccaggaga tcgtgctgga gaacgtgacc gagaacttca acatgtggaa ggacgacatg 300
   gtggaccaga tgcacgagga catcatctcc ctgtgggacc agtccctgaa gccctgcgtg 360
   aagctgaccc ccctgtgcgt gaccctgaag tgctccaact tcacccgcga gggcaacgtg 420
   acctacaagg aggagatgga caaggtgaag aactgctcct tcaacgtgac caccggcatc 480
   cgcgacaaga agcagaaggt gaacgccctg ttctaccgcc tggacatcac ccccctggac 540
   gagaacaaca acaactcctc cgagtaccgc ttgatcaact gcaagtcctc caccatcacc 600
   caggcctgcc ccaaggtgaa cttcgacccc atccccatcc actactgcgc ccccgccggc 660
   tacgccatcc tgaagtgcaa caacaagacc ttcaacggca ccggcccctg ccacaacgtg 720
   tccaccgtgc agtgcaccca cggcatcaag cccgtggtgt ccacccagct gctgctgaac 780
   ggctccctgg ccgagcgcga gatcatcatc cgctccgaga acctgaccga caacgtgaag 840
   accatcatcg tgcacttcaa cgagtccgtg gagatcaact gcacccgccc caacaacaac 900
   acccgcaagt ccatccgcat cggccccggc caggccttct acgccaccgg cgacatcatc 960
   ggcgacatcc gccaggccca ctgcaacatc tccaaggaga actggaacaa gaccctgcag 1020
   tgggtgcgcg gcaagctgga ggagcacttc cccaacaaga ccatcgtgtt caagccctcc 1080
   tccggcggcg acctggagat caccacccac tccttcaact gccgcggcga gttcttctac 1140
   tgcaacacct ccaagctgtt caacggcacc gacaactcca cccacatgga caccggcaac 1200
   gacaccgtga tcaccatccc ctgccgcatc aagcagatca tcaacatgtg gcaggaggtg 1260
   gggcgcgcca tgtacgcccc ccccatcgag ggcaacatca cctgcaagtc caacatcacc 1320
   ggcctgctgc tggtgcgcga cggcggccag gacaactcca ccaacaacac cgagaccttc 1380
   cgccccggcg gcggcgacat gcgcaacaac tggcgctccg agctgtacaa gtacaaggtg 1440
   gtggagatca agcccctggg catcgccccc accaaggcca agcgccgcgt ggtggagcgc 1500
   gagaagcgcg ccgtgggcat cggcgccgtg ttcctgggct tcctgggcgc cgccggctcc 1560
   accatgggcg ccgcctccat caccctgacc gtgcaggccc gccagctgct gtccggcatc 1620
   gtgcagcagc aggacaacct gctgcgcgcc atcgaggccc agcagcacat gctgcagctg 1680
   accgtgtggg gcatcaaaca gctgcaggcc cgcgtgctgg ccatcgagcg ctacctgcag 1740
   gaccagcagc tgctgggcat ctggggctgc tccggcaagc tgatctgcac caccgccgtg 1800
   ccctggaacg cctcctggtc caacaagtcc cagaccgaca tctgggagaa catgacctgg 1860
   atgcagtggg acaaggagat ctccaagcac accgacacca tctaccgcct gctggaggac 1920
   tcccagaacc agcaggagaa gaacgagaag gacctgctgg ccctggactc ctgggagaac 1980
   ctgtggaact ggttctccat caccaagtgg ctgtggtaca tcaagatctt catcatgatc 2040
   gtgggcggcc tgatcggcct gcgcatcatc ttcgccgtgc tgtccatcgt gtcccgcgtg 2100
   cgccagggct actcccccct gtccttccag acccacctgc ccaccccccg cggccccgac 2160
   cgccccgagg gcatcgagga ggagggcggc gagcgcgacc gcgaccgctc catccgcctg 2220
   gtgaacggct ccctggccct gatctgggac gacctgcgct ccctgtgcct gttctcctac 2280
   caccgcctgc gcgacctgct gctgatcgtg acccgcatcg tggagctgct gggccgccgc 2340
   ggctgggagg ccctgaagta ctggtggaac ctgctgcagt actggtccca ggagctgaag 2400
   aactccgccg tgtccctgct gaacgccacc gccatcgccg tggccgaggg caccgaccgc 2460
   gtgatcgagg tggtgcaggg cgcctgccgc gccatccgcc acatcccccg ccgcatgcgc 2520
   cagggcctgg agcgcatcct gctgtga                                     2547
<210> 2
   <211> 848
   <212> PRT
   <213> Artificial Sequence
<220>
   <223> Description of Artificial Sequence: Synthetic protein construct
<400> 2
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Lys Thr Thr Leu Phe Cys Ala Ser Asp Ala Lys Ala Tyr Glu Lys Glu
       50                  55                  60
   Val His Asn Ile Trp Ala Thr His Ala Cys Val Pro Thr Asp Pro Asn
   65                  70                  75                  80
   Pro Gln Glu Ile Val Leu Glu Asn Val Thr Glu Asn Phe Asn Met Trp
                   85                  90                  95
   Lys Asp Asp Met Val Asp Gln Met His Glu Asp Ile Ile Ser Leu Trp
               100                 105                 110
   Asp Gln Ser Leu Lys Pro Cys Val Lys Leu Thr Pro Leu Cys Val Thr
           115                 120                 125
   Leu Lys Cys Ser Asn Phe Thr Arg Glu Gly Asn Val Thr Tyr Lys Glu
       130                 135                 140
   Glu Met Asp Lys Val Lys Asn Cys Ser Phe Asn Val Thr Thr Gly Ile
   145                 150                 155                 160
   Arg Asp Lys Lys Gln Lys Val Asn Ala Leu Phe Tyr Arg Leu Asp Ile
                   165                 170                 175
   Thr Pro Leu Asp Glu Asn Asn Asn Asn Ser Ser Glu Tyr Arg Leu Ile
               180                 185                 190
   Asn Cys Lys Ser Ser Thr Ile Thr Gln Ala Cys Pro Lys Val Asn Phe
           195                 200                 205
   Asp Pro Ile Pro Ile His Tyr Cys Ala Pro Ala Gly Tyr Ala Ile Leu
       210                 215                 220
   Lys Cys Asn Asn Lys Thr Phe Asn Gly Thr Gly Pro Cys His Asn Val
   225                 230                 235                 240
   Ser Thr Val Gln Cys Thr His Gly Ile Lys Pro Val Val Ser Thr Gln
                   245                 250                 255
   Leu Leu Leu Asn Gly Ser Leu Ala Glu Arg Glu Ile Ile Ile Arg Ser
               260                 265                 270
   Glu Asn Leu Thr Asp Asn Val Lys Thr Ile Ile Val His Phe Asn Glu
           275                 280                 285
   Ser Val Glu Ile Asn Cys Thr Arg Pro Asn Asn Asn Thr Arg Lys Ser
       290                 295                 300
   Ile Arg Ile Gly Pro Gly Gln Ala Phe Tyr Ala Thr Gly Asp Ile Ile
   305                 310                 315                 320
   Gly Asp Ile Arg Gln Ala His Cys Asn Ile Ser Lys Glu Asn Trp Asn
                   325                 330                 335
   Lys Thr Leu Gln Trp Val Arg Gly Lys Leu Glu Glu His Phe Pro Asn
               340                 345                 350
   Lys Thr Ile Val Phe Lys Pro Ser Ser Gly Gly Asp Leu Glu Ile Thr
           355                 360                 365
   Thr His Ser Phe Asn Cys Arg Gly Glu Phe Phe Tyr Cys Asn Thr Ser
       370                 375                 380
   Lys Leu Phe Asn Gly Thr Asp Asn Ser Thr His Met Asp Thr Gly Asn
   385                 390                 395                 400
   Asp Thr Val Ile Thr Ile Pro Cys Arg Ile Lys Gln Ile Ile Asn Met
                   405                 410                 415
   Trp Gln Glu Val Gly Arg Ala Met Tyr Ala Pro Pro Ile Glu Gly Asn
               420                 425                 430
   Ile Thr Cys Lys Ser Asn Ile Thr Gly Leu Leu Leu Val Arg Asp Gly
           435                 440                 445
   Gly Gln Asp Asn Ser Thr Asn Asn Thr Glu Thr Phe Arg Pro Gly Gly
       450                 455                 460
   Gly Asp Met Arg Asn Asn Trp Arg Ser Glu Leu Tyr Lys Tyr Lys Val
   465                 470                 475                 480
   Val Glu Ile Lys Pro Leu Gly Ile Ala Pro Thr Lys Ala Lys Arg Arg
   485 490 495
   Val Val Glu Arg Glu Lys Arg Ala Val Gly Ile Gly Ala Val Phe Leu
   500 505 510
   Gly Phe Leu Gly Ala Ala Gly Ser Thr Met Gly Ala Ala Ser Ile Thr
   515 520 525
   Leu Thr Val Gln Ala Arg Gln Leu Leu Ser Gly Ile Val Gln Gln Gln
   530 535 540
   Asp Asn Leu Leu Arg Ala Ile Glu Ala Gln Gln His Met Leu Gln Leu
   545 550 555 560
   Thr Val Trp Gly Ile Lys Gln Leu Gln Ala Arg Val Leu Ala Ile Glu
   565 570 575
   Arg Tyr Leu Gln Asp Gln Gln Leu Leu Gly Ile Trp Gly Cys Ser Gly
   580 585 590
   Lys Leu Ile Cys Thr Thr Ala Val Pro Trp Asn Ala Ser Trp Ser Asn
   595 600 605
   Lys Ser Gln Thr Asp Ile Trp Glu Asn Met Thr Trp Met Gln Trp Asp
   610 615 620
   Lys Glu Ile Ser Lys His Thr Asp Thr Ile Tyr Arg Leu Leu Glu Asp
   625 630 635 640
   Ser Gln Asn Gln Gln Glu Lys Asn Glu Lys Asp Leu Leu Ala Leu Asp
   645 650 655
   Ser Trp Glu Asn Leu Trp Asn Trp Phe Ser Ile Thr Lys Trp Leu Trp
   660 665 670
   Tyr Ile Lys Ile Phe Ile Met Ile Val Gly Gly Leu Ile Gly Leu Arg
   675 680 685
   Ile Ile Phe Ala Val Leu Ser Ile Val Ser Arg Val Arg Gln Gly Tyr
   690 695 700
   Ser Pro Leu Ser Phe Gln Thr His Leu Pro Thr Pro Arg Gly Pro Asp
   705 710 715 720
   Arg Pro Glu Gly Ile Glu Glu Glu Gly Gly Glu Arg Asp Arg Asp Arg
   725 730 735
   Ser Ile Arg Leu Val Asn Gly Ser Leu Ala Leu Ile Trp Asp Asp Leu
   740 745 750
   Arg Ser Leu Cys Leu Phe Ser Tyr His Arg Leu Arg Asp Leu Leu Leu
   755 760 765
   Ile Val Thr Arg Ile Val Glu Leu Leu Gly Arg Arg Gly Trp Glu Ala
   770 775 780
   Leu Lys Tyr Trp Trp Asn Leu Leu Gln Tyr Trp Ser Gln Glu Leu Lys
   785 790 795 800
   Asn Ser Ala Val Ser Leu Leu Asn Ala Thr Ala Ile Ala Val Ala Glu
   805 810 815
   Gly Thr Asp Arg Val Ile Glu Val Val Gln Gly Ala Cys Arg Ala Ile
   820 825 830
   Arg His Ile Pro Arg Arg Met Arg Gln Gly Leu Glu Arg Ile Leu Leu
   835 840 845
<210> 3
   <211> 2313
   <212> DNA
   <213> Artificial Sequence
<220>
   <223> Description of Artificial Sequence: Synthetic nucleotide construct
<400> 3
   atgcgcgtga aggagaagta ccagcacctg tggcgctggg gctggcgctg gggcaccatg 60
   ctgctgggca tgctgatgat ctgctccgcc accgagaagc tgtgggtgac cgtgtactac 120
   ggcgtgcccg tgtggaagga ggccaagacc accctgttct gcgcctccga cgccaaggcc 180
   tacgagaagg aggtgcacaa catctgggcc acccacgcct gcgtgcccac cgaccccaac 240
   ccccaggaga tcgtgctgga gaacgtgacc gagaacttca acatgtggaa ggacgacatg 300
   gtggaccaga tgcacgagga catcatctcc ctgtgggacc agtccctgaa gccctgcgtg 360
   ggcgccggcg cctgccccaa ggtgaacttc gaccccatcc ccatccacta ctgcgccccc 420
   gccggctacg ccatcctgaa gtgcaacaac aagaccttca acggcaccgg cccctgccac 480
   aacgtgtcca ccgtgcagtg cacccacggc atcaagcccg tggtgtccac ccagctgctg 540
   ctgaacggct ccctggccga gcgcgagatc atcatccgct ccgagaacct gaccgacaac 600
   gtgaagacca tcatcgtgca cttcaacgag tccgtggaga tcaactgcac ccgccccaac 660
   aacaacaccc gcaagtccat ccgcatcggc cccggccagg ccttctacgc caccggcgac 720
   atcatcggcg acatccgcca ggcccactgc aacatctcca aggagaactg gaacaagacc 780
   ctgcagtggg tgcgcggcaa gctggaggag cacttcccca acaagaccat cgtgttcaag 840
   ccctcctccg gcggcgacct ggagatcacc acccactcct tcaactgccg cggcgagttc 900
   ttctactgca acacctccaa gctgttcaac ggcaccgaca actccaccca catggacacc 960
   ggcaacgaca ccgtgatcac catcccctgc cgcatcaagc agatcatcaa catgtggcag 1020
   gaggtggggc gcgccatgta cgcccccccc atcgagggca acatcacctg caagtccaac 1080
   atcaccggcc tgctgctggt gcgcgacggc ggccaggaca actccaccaa caacaccgag 1140
   accttccgcc ccggcggcgg cgacatgcgc aacaactggc gctccgagct gtacaagtac 1200
   aaggtggtgg agatcaagcc cctgggcatc gcccccacca aggccaagcg ccgcgtggtg 1260
   gagcgcgaga agcgcgccgt gggcatcggc gccgtgttcc tgggcttcct gggcgccgcc 1320
   ggctccacca tgggcgccgc ctccatcacc ctgaccgtgc aggcccgcca gctgctgtcc 1380
   ggcatcgtgc agcagcagga caacctgctg cgcgccatcg aggcccagca gcacatgctg 1440
   cagctgaccg tgtggggcat caaacagctg caggcccgcg tgctggccat cgagcgctac 1500
   ctgcaggacc agcagctgct gggcatctgg ggctgctccg gcaagctgat ctgcaccacc 1560
   gccgtgccct ggaacgcctc ctggtccaac aagtcccaga ccgacatctg ggagaacatg 1620
   acctggatgc agtgggacaa ggagatctcc aagcacaccg acaccatcta ccgcctgctg 1680
   gaggactccc agaaccagca ggagaagaac gagaaggacc tgctggccct ggactcctgg 1740
   gagaacctgt ggaactggtt ctccatcacc aagtggctgt ggtacatcaa gatcttcatc 1800
   atgatcgtgg gcggcctgat cggcctgcgc atcatcttcg ccgtgctgtc catcgtgtcc 1860
   cgcgtgcgcc agggctactc ccccctgtcc ttccagaccc acctgcccac cccccgcggc 1920
   cccgaccgcc ccgagggcat cgaggaggag ggcggcgagc gcgaccgcga ccgctccatc 1980
   cgcctggtga acggctccct ggccctgatc tgggacgacc tgcgctccct gtgcctgttc 2040
   tcctaccacc gcctgcgcga cctgctgctg atcgtgaccc gcatcgtgga gctgctgggc 2100
   cgccgcggct gggaggccct gaagtactgg tggaacctgc tgcagtactg gtcccaggag 2160
   ctgaagaact ccgccgtgtc cctgctgaac gccaccgcca tcgccgtggc cgagggcacc 2220
   gaccgcgtga tcgaggtggt gcagggcgcc tgccgcgcca tccgccacat cccccgccgc 2280
   atgcgccagg gcctggagcg catcctgctg tga                             2313
<210> 4
   <211> 770
   <212> PRT
   <213> Artificial Sequence
<220>
   <223> Description of Artificial Sequence: Synthetic protein construct
<400> 4
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Lys Thr Thr Leu Phe Cys Ala Ser Asp Ala Lys Ala Tyr Glu Lys Glu
       50                  55                  60
   Val His Asn Ile Trp Ala Thr His Ala Cys Val Pro Thr Asp Pro Asn
   65                  70                  75                  80
   Pro Gln Glu Ile Val Leu Glu Asn Val Thr Glu Asn Phe Asn Met Trp
                   85                  90                  95
   Lys Asp Asp Met Val Asp Gln Met His Glu Asp Ile Ile Ser Leu Trp
               100                 105                 110
   Asp Gln Ser Leu Lys Pro Cys Val Gly Ala Gly Ala Cys Pro Lys Val
           115                 120                 125
   Asn Phe Asp Pro Ile Pro Ile His Tyr Cys Ala Pro Ala Gly Tyr Ala
       130                 135                 140
   Ile Leu Lys Cys Asn Asn Lys Thr Phe Asn Gly Thr Gly Pro Cys His
   145                 150                 155                 160
   Asn Val Ser Thr Val Gln Cys Thr His Gly Ile Lys Pro Val Val Ser
                   165                 170                 175
   Thr Gln Leu Leu Leu Asn Gly Ser Leu Ala Glu Arg Glu Ile Ile Ile
               180                 185                 190
   Arg Ser Glu Asn Leu Thr Asp Asn Val Lys Thr Ile Ile Val His Phe
           195                 200                 205
   Asn Glu Ser Val Glu Ile Asn Cys Thr Arg Pro Asn Asn Asn Thr Arg
   210 215 220
   Lys Ser Ile Arg Ile Gly Pro Gly Gln Ala Phe Tyr Ala Thr Gly Asp
   225 230 235 240
   Ile Ile Gly Asp Ile Arg Gln Ala His Cys Asn Ile Ser Lys Glu Asn
   245 250 255
   Trp Asn Lys Thr Leu Gln Trp Val Arg Gly Lys Leu Glu Glu His Phe
   260 265 270
   Pro Asn Lys Thr Ile Val Phe Lys Pro Ser Ser Gly Gly Asp Leu Glu
   275 280 285
   Ile Thr Thr His Ser Phe Asn Cys Arg Gly Glu Phe Phe Tyr Cys Asn
   290 295 300
   Thr Ser Lys Leu Phe Asn Gly Thr Asp Asn Ser Thr His Met Asp Thr
   305 310 315 320
   Gly Asn Asp Thr Val Ile Thr Ile Pro Cys Arg Ile Lys Gln Ile Ile
   325 330 335
   Asn Met Trp Gln Glu Val Gly Arg Ala Met Tyr Ala Pro Pro Ile Glu
   340 345 350
   Gly Asn Ile Thr Cys Lys Ser Asn Ile Thr Gly Leu Leu Leu Val Arg
   355 360 365
   Asp Gly Gly Gln Asp Asn Ser Thr Asn Asn Thr Glu Thr Phe Arg Pro
   370 375 380
   Gly Gly Gly Asp Met Arg Asn Asn Trp Arg Ser Glu Leu Tyr Lys Tyr
   385 390 395 400
   Lys Val Val Glu Ile Lys Pro Leu Gly Ile Ala Pro Thr Lys Ala Lys
   405 410 415
   Arg Arg Val Val Glu Arg Glu Lys Arg Ala Val Gly Ile Gly Ala Val
   420 425 430
   Phe Leu Gly Phe Leu Gly Ala Ala Gly Ser Thr Met Gly Ala Ala Ser
   435 440 445
   Ile Thr Leu Thr Val Gln Ala Arg Gln Leu Leu Ser Gly Ile Val Gln
   450 455 460
   Gln Gln Asp Asn Leu Leu Arg Ala Ile Glu Ala Gln Gln His Met Leu
   465 470 475 480
   Gln Leu Thr Val Trp Gly Ile Lys Gln Leu Gln Ala Arg Val Leu Ala
   485 490 495
   Ile Glu Arg Tyr Leu Gln Asp Gln Gln Leu Leu Gly Ile Trp Gly Cys
   500 505 510
   Ser Gly Lys Leu Ile Cys Thr Thr Ala Val Pro Trp Asn Ala Ser Trp
   515 520 525
   Ser Asn Lys Ser Gln Thr Asp Ile Trp Glu Asn Met Thr Trp Met Gln
   530 535 540
   Trp Asp Lys Glu Ile Ser Lys His Thr Asp Thr Ile Tyr Arg Leu Leu
   545 550 555 560
   Glu Asp Ser Gln Asn Gln Gln Glu Lys Asn Glu Lys Asp Leu Leu Ala
   565 570 575
   Leu Asp Ser Trp Glu Asn Leu Trp Asn Trp Phe Ser Ile Thr Lys Trp
   580 585 590
   Leu Trp Tyr Ile Lys Ile Phe Ile Met Ile Val Gly Gly Leu Ile Gly
   595 600 605
   Leu Arg Ile Ile Phe Ala Val Leu Ser Ile Val Ser Arg Val Arg Gln
   610 615 620
   Gly Tyr Ser Pro Leu Ser Phe Gln Thr His Leu Pro Thr Pro Arg Gly
   625 630 635 640
   Pro Asp Arg Pro Glu Gly Ile Glu Glu Glu Gly Gly Glu Arg Asp Arg
   645 650 655
   Asp Arg Ser Ile Arg Leu Val Asn Gly Ser Leu Ala Leu Ile Trp Asp
   660 665 670
   Asp Leu Arg Ser Leu Cys Leu Phe Ser Tyr His Arg Leu Arg Asp Leu
   675 680 685
   Leu Leu Ile Val Thr Arg Ile Val Glu Leu Leu Gly Arg Arg Gly Trp
   690 695 700
   Glu Ala Leu Lys Tyr Trp Trp Asn Leu Leu Gln Tyr Trp Ser Gln Glu
   705 710 715 720
   Leu Lys Asn Ser Ala Val Ser Leu Leu Asn Ala Thr Ala Ile Ala Val
   725 730 735
   Ala Glu Gly Thr Asp Arg Val Ile Glu Val Val Gln Gly Ala Cys Arg
   740 745 750
   Ala Ile Arg His Ile Pro Arg Arg Met Arg Gln Gly Leu Glu Arg Ile
   755 760 765
   Leu Leu
   770
<210> 5
   <211> 2232
   <212> DNA
   <213> Artificial Sequence
<220>
   <223> Description of Artificial Sequence: Synthetic nucleotide construct
<400> 5
   atgcgcgtga aggagaagta ccagcacctg tggcgctggg gctggcgctg gggcaccatg 60
   ctgctgggca tgctgatgat ctgctccgcc accgagaagc tgtgggtgac cgtgtactac 120
   ggcgtgcccg tgtggaagga ggccaagacc accctgttct gcgcctccga cgccaaggcc 180
   tacgagaagg aggtgcacaa catctgggcc acccacgcct gcgtgcccac cgaccccaac 240
   ccccaggaga tcgtgctgga gaacgtgacc gagaacttca acatgtggaa ggacgacatg 300
   gtggaccaga tgcacgagga catcatctcc ctgtgggacc agtccctgaa gccctgcgtg 360
   ggcgccggcg cctgccccaa ggtgaacttc gaccccatcc ccatccacta ctgcgccccc 420
   gccggctacg ccatcctgaa gtgcaacaac aagaccttca acggcaccgg cccctgccac 480
   aacgtgtcca ccgtgcagtg cacccacggc atcaagcccg tggtgtccac ccagctgctg 540
   ctgaacggct ccctggccga gcgcgagatc atcatccgct ccgagaacct gaccgacaac 600
   gtgaagacca tcatcgtgca cttcaacgag tccgtggaga tcaactgcac cggcgccggc 660
   gcccactgca acatctccaa ggagaactgg aacaagaccc tgcagtgggt gcgcggcaag 720
   ctggaggagc acttccccaa caagaccatc gtgttcaagc cctcctccgg cggcgacctg 780
   gagatcacca cccactcctt caactgccgc ggcgagttct tctactgcaa cacctccaag 840
   ctgttcaacg gcaccgacaa ctccacccac atggacaccg gcaacgacac cgtgatcacc 900
   atcccctgcc gcatcaagca gatcatcaac atgtggcagg aggtggggcg cgccatgtac 960
   gcccccccca tcgagggcaa catcacctgc aagtccaaca tcaccggcct gctgctggtg 1020
   cgcgacggcg gccaggacaa ctccaccaac aacaccgaga ccttccgccc cggcggcggc 1080
   gacatgcgca acaactggcg ctccgagctg tacaagtaca aggtggtgga gatcaagccc 1140
   ctgggcatcg cccccaccaa ggccaagcgc cgcgtggtgg agcgcgagaa gcgcgccgtg 1200
   ggcatcggcg ccgtgttcct gggcttcctg ggcgccgccg gctccaccat gggcgccgcc 1260
   tccatcaccc tgaccgtgca ggcccgccag ctgctgtccg gcatcgtgca gcagcaggac 1320
   aacctgctgc gcgccatcga ggcccagcag cacatgctgc agctgaccgt gtggggcatc 1380
   aaacagctgc aggcccgcgt gctggccatc gagcgctacc tgcaggacca gcagctgctg 1440
   ggcatctggg gctgctccgg caagctgatc tgcaccaccg ccgtgccctg gaacgcctcc 1500
   tggtccaaca agtcccagac cgacatctgg gagaacatga cctggatgca gtgggacaag 1560
   gagatctcca agcacaccga caccatctac cgcctgctgg aggactccca gaaccagcag 1620
   gagaagaacg agaaggacct gctggccctg gactcctggg agaacctgtg gaactggttc 1680
   tccatcacca agtggctgtg gtacatcaag atcttcatca tgatcgtggg cggcctgatc 1740
   ggcctgcgca tcatcttcgc cgtgctgtcc atcgtgtccc gcgtgcgcca gggctactcc 1800
   cccctgtcct tccagaccca cctgcccacc ccccgcggcc ccgaccgccc cgagggcatc 1860
   gaggaggagg gcggcgagcg cgaccgcgac cgctccatcc gcctggtgaa cggctccctg 1920
   gccctgatct gggacgacct gcgctccctg tgcctgttct cctaccaccg cctgcgcgac 1980
   ctgctgctga tcgtgacccg catcgtggag ctgctgggcc gccgcggctg ggaggccctg 2040
   aagtactggt ggaacctgct gcagtactgg tcccaggagc tgaagaactc cgccgtgtcc 2100
   ctgctgaacg ccaccgccat cgccgtggcc gagggcaccg accgcgtgat cgaggtggtg 2160
   cagggcgcct gccgcgccat ccgccacatc cccgccgcca tgcgccaggg cctggagcgc 2220
   atcctgctgt ga                                                     2232
<210> 6
   <211> 743
   <212> PRT
   <213> Artificial Sequence
<220>
   <223> Description of Artificial Sequence: Synthetic protein construct
<400> 6
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
                   20                  25                  30
   Lys Leu Trp Val Thr Val Tyr Tyr Gly Val Pro Val Trp Lys Glu Ala
               35                  40                  45
   Lys Thr Thr Leu Phe Cys Ala Ser Asp Ala Lys Ala Tyr Glu Lys Glu
       50                  55                  60
   Val His Asn Ile Trp Ala Thr His Ala Cys Val Pro Thr Asp Pro Asn
   65                  70                  75                  80
   Pro Gln Glu Ile Val Leu Glu Asn Val Thr Glu Asn Phe Asn Met Trp
                   85                  90                  95
   Lys Asp Asp Met Val Asp Gln Met His Glu Asp Ile Ile Ser Leu Trp
               100                 105                 110
   Asp Gln Ser Leu Lys Pro Cys Val Gly Ala Gly Ala Cys Pro Lys Val
           115                 120                 125
   Asn Phe Asp Pro Ile Pro Ile His Tyr Cys Ala Pro Ala Gly Tyr Ala
       130                 135                 140
   Ile Leu Lys Cys Asn Asn Lys Thr Phe Asn Gly Thr Gly Pro Cys His
   145                 150                 155                 160
   Asn Val Ser Thr Val Gln Cys Thr His Gly Ile Lys Pro Val Val Ser
                   165                 170                 175
   Thr Gln Leu Leu Leu Asn Gly Ser Leu Ala Glu Arg Glu Ile Ile Ile
               180                 185                 190
   Arg Ser Glu Asn Leu Thr Asp Asn Val Lys Thr Ile Ile Val His Phe
           195                 200                 205
   Asn Glu Ser Val Glu Ile Asn Cys Thr Gly Ala Gly Ala His Cys Asn
       210                 215                 220
   Ile Ser Lys Glu Asn Trp Asn Lys Thr Leu Gln Trp Val Arg Gly Lys
   225                 230                 235                 240
   Leu Glu Glu His Phe Pro Asn Lys Thr Ile Val Phe Lys Pro Ser Ser
                   245                 250                 255
   Gly Gly Asp Leu Glu Ile Thr Thr His Ser Phe Asn Cys Arg Gly Glu
               260                 265                 270
   Phe Phe Tyr Cys Asn Thr Ser Lys Leu Phe Asn Gly Thr Asp Asn Ser
           275                 280                 285
   Thr His Met Asp Thr Gly Asn Asp Thr Val Ile Thr Ile Pro Cys Arg
       290                 295                 300
   Ile Lys Gln Ile Ile Asn Met Trp Gln Glu Val Gly Arg Ala Met Tyr
   305                 310                 315                 320
   Ala Pro Pro Ile Glu Gly Asn Ile Thr Cys Lys Ser Asn Ile Thr Gly
                   325                 330                 335
   Leu Leu Leu Val Arg Asp Gly Gly Gln Asp Asn Ser Thr Asn Asn Thr
               340                 345                 350
   Glu Thr Phe Arg Pro Gly Gly Gly Asp Met Arg Asn Asn Trp Arg Ser
           355                 360                 365
   Glu Leu Tyr Lys Tyr Lys Val Val Glu Ile Lys Pro Leu Gly Ile Ala
       370                 375                 380
   Pro Thr Lys Ala Lys Arg Arg Val Val Glu Arg Glu Lys Arg Ala Val
   385                 390                 395                 400
   Gly Ile Gly Ala Val Phe Leu Gly Phe Leu Gly Ala Ala Gly Ser Thr
                   405                 410                 415
   Met Gly Ala Ala Ser Ile Thr Leu Thr Val Gln Ala Arg Gln Leu Leu
               420                 425                 430
   Ser Gly Ile Val Gln Gln Gln Asp Asn Leu Leu Arg Ala Ile Glu Ala
           435                 440                 445
   Gln Gln His Met Leu Gln Leu Thr Val Trp Gly Ile Lys Gln Leu Gln
       450                 455                 460
   Ala Arg Val Leu Ala Ile Glu Arg Tyr Leu Gln Asp Gln Gln Leu Leu
   465                 470                 475                 480
   Gly Ile Trp Gly Cys Ser Gly Lys Leu Ile Cys Thr Thr Ala Val Pro
                   485                 490                 495
   Trp Asn Ala Ser Trp Ser Asn Lys Ser Gln Thr Asp Ile Trp Glu Asn
               500                 505                 510
   Met Thr Trp Met Gln Trp Asp Lys Glu Ile Ser Lys His Thr Asp Thr
           515                 520                 525
   Ile Tyr Arg Leu Leu Glu Asp Ser Gln Asn Gln Gln Glu Lys Asn Glu
       530                 535                 540
   Lys Asp Leu Leu Ala Leu Asp Ser Trp Glu Asn Leu Trp Asn Trp Phe
   545                 550                 555                 560
   Ser Ile Thr Lys Trp Leu Trp Tyr Ile Lys Ile Phe Ile Met Ile Val
                   565                 570                 575
   Gly Gly Leu Ile Gly Leu Arg Ile Ile Phe Ala Val Leu Ser Ile Val
               580                 585                 590
   Ser Arg Val Arg Gln Gly Tyr Ser Pro Leu Ser Phe Gln Thr His Leu
           595                 600                 605
   Pro Thr Pro Arg Gly Pro Asp Arg Pro Glu Gly Ile Glu Glu Glu Gly
       610                 615                 620
   Gly Glu Arg Asp Arg Asp Arg Ser Ile Arg Leu Val Asn Gly Ser Leu
   625 630 635 640
   Ala Leu Ile Trp Asp Asp Leu Arg Ser Leu Cys Leu Phe Ser Tyr His
   645 650 655
   Arg Leu Arg Asp Leu Leu Leu Ile Val Thr Arg Ile Val Glu Leu Leu
   660 665 670
   Gly Arg Arg Gly Trp Glu Ala Leu Lys Tyr Trp Trp Asn Leu Leu Gln
   675 680 685
   Tyr Trp Ser Gln Glu Leu Lys Asn Ser Ala Val Ser Leu Leu Asn Ala
   690 695 700
   Thr Ala Ile Ala Val Ala Glu Gly Thr Asp Arg Val Ile Glu Val Val
   705 710 715 720
   Gln Gly Ala Cys Arg Ala Ile Arg His Ile Pro Arg Arg Met Arg Gln
   725 730 735
   Gly Leu Glu Arg Ile Leu Leu
   740
<210> 7
   <211> 2169
   <212> DNA
   <213> Artificial Sequence
<220>
   <223> Description of Artificial Sequence: Synthetic nucleotide construct
<400> 7
   atgcgcgtga aggagaagta ccagcacctg tggcgctggg gctggcgctg gggcaccatg 60
   ctgctgggca tgctgatgat ctgctccgcc accgagaagc tgtgggtgac cgtgtactac 120
   ggcgtgcccg tgtggaagga ggccaagacc accctgttct gcgcctccga cgccaaggcc 180
   tacgagaagg aggtgcacaa catctgggcc acccacgcct gcgtgcccac cgaccccaac 240
   ccccaggaga tcgtgctgga gaacgtgacc gagaacttca acatgtggaa ggacgacatg 300
   gtggaccaga tgcacgagga catcatctcc ctgtgggacc agtccctgaa gccctgcgtg 360
   ggcgccggcg cctgccccaa ggtgaacttc gaccccatcc ccatccacta ctgcgccccc 420
   gccggctacg ccatcctgaa gtgcaacaac aagaccttca acggcaccgg cccctgccac 480
   aacgtgtcca ccgtgcagtg cacccacggc atcaagcccg tggtgtccac ccagctgctg 540
   ctgaacggct ccctggccga gcgcgagatc atcatccgct ccgagaacct gaccgacaac 600
   gtgaagacca tcatcgtgca cttcaacgag tccgtggaga tcaactgcac ccgcgccggc 660
   gcccactgca acatctccaa ggagaactgg aacaagaccc tgcagtgggt gcgcggcaag 720
   ctggaggagc acttccccaa caagaccatc gtgttcaagc cctcctccgg cggcgacctg 780
   gagatcacca cccactcctt caactgccgc ggcgagttct tctactgcaa cggcgccggc 840
   ccctgccgca tcaagcagat catcaacatg tggcaggagg tggggcgcgc catgtacgcc 900
   ccccccatcg agggcaacat cacctgcaag tccaacatca ccggcctgct gctggtgcgc 960
   gacggcggcc aggacaactc caccaacaac accgagacct tccgccccgg cggcggcgac 1020
   atgcgcaaca actggcgctc cgagctgtac aagtacaagg tggtggagat caagcccctg 1080
   ggcatcgccc ccaccaaggc caagcgccgc gtggtggagc gcgagaagcg cgccgtgggc 1140
   atcggcgccg tgttcctggg cttcctgggc gccgccggct ccaccatggg cgccgcctcc 1200
   atcaccctga ccgtgcaggc ccgccagctg ctgtccggca tcgtgcagca gcaggacaac 1260
   ctgctgcgcg ccatcgaggc ccagcagcac atgctgcagc tgaccgtgtg gggcatcaaa 1320
   cagctgcagg cccgcgtgct ggccatcgag cgctacctgc aggaccagca gctgctgggc 1380
   atctggggct gctccggcaa gctgatctgc accaccgccg tgccctggaa cgcctcctgg 1440
   tccaacaagt cccagaccga catctgggag aacatgacct ggatgcagtg ggacaaggag 1500
   atctccaagc acaccgacac catctaccgc ctgctggagg actcccagaa ccagcaggag 1560
   aagaacgaga aggacctgct ggccctggac tcctgggaga acctgtggaa ctggttctcc 1620
   atcaccaagt ggctgtggta catcaagatc ttcatcatga tcgtgggcgg cctgatcggc 1680
   ctgcgcatca tcttcgccgt gctgtccatc gtgtcccgcg tgcgccaggg ctactccccc 1740
   ctgtccttcc agacccacct gcccaccccc cgcggccccg accgccccga gggcatcgag 1800
   gaggagggcg gcgagcgcga ccgcgaccgc tccatccgcc tggtgaacgg ctccctggcc 1860
   ctgatctggg acgacctgcg ctccctgtgc ctgttctcct accaccgcct gcgcgacctg 1920
   ctgctgatcg tgacccgcat cgtggagctg ctgggccgcc gcggctggga ggccctgaag 1980
   tactggtgga acctgctgca gtactggtcc caggagctga agaactccgc cgtgtccctg 2040
   ctgaacgcca ccgccatcgc cgtggccgag ggcaccgacc gcgtgatcga ggtggtgcag 2100
   ggcgcctgcc gcgccatccg ccacatcccc cgccgcatgc gccagggcct ggagcgcatc 2160
   ctgctgtga                                                         2169
<210> 8
   <211> 722
   <212> PRT
   <213> Artificial Sequence
<220>
   <223> Description of Artificial Sequence: Synthetic protein construct
<400> 8
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Lys Thr Thr Leu Phe Cys Ala Ser Asp Ala Lys Ala Tyr Glu Lys Glu
       50                  55                  60
   Val His Asn Ile Trp Ala Thr His Ala Cys Val Pro Thr Asp Pro Asn
   65                  70                  75                  80
   Pro Gln Glu Ile Val Leu Glu Asn Val Thr Glu Asn Phe Asn Met Trp
                   85                  90                  95
   Lys Asp Asp Met Val Asp Gln Met His Glu Asp Ile Ile Ser Leu Trp
               100                 105                 110
   Asp Gln Ser Leu Lys Pro Cys Val Gly Ala Gly Ala Cys Pro Lys Val
           115                 120                 125
   Asn Phe Asp Pro Ile Pro Ile His Tyr Cys Ala Pro Ala Gly Tyr Ala
       130                 135                 140
   Ile Leu Lys Cys Asn Asn Lys Thr Phe Asn Gly Thr Gly Pro Cys His
   145                 150                 155                 160
   Asn Val Ser Thr Val Gln Cys Thr His Gly Ile Lys Pro Val Val Ser
                   165                 170                 175
   Thr Gln Leu Leu Leu Asn Gly Ser Leu Ala Glu Arg Glu Ile Ile Ile
   180 185 190
   Arg Ser Glu Asn Leu Thr Asp Asn Val Lys Thr Ile Ile Val His Phe
   195 200 205
   Asn Glu Ser Val Glu Ile Asn Cys Thr Gly Ala Gly Ala His Cys Asn
   210 215 220
   Ile Ser Lys Glu Asn Trp Asn Lys Thr Leu Gln Trp Val Arg Gly Lys
   225 230 235 240
   Leu Glu Glu His Phe Pro Asn Lys Thr Ile Val Phe Lys Pro Ser Ser
   245 250 255
   Gly Gly Asp Leu Glu Ile Thr Thr His Ser Phe Asn Cys Arg Gly Glu
   260 265 270
   Phe Phe Tyr Cys Asn Gly Ala Gly Pro Cys Arg Ile Lys Gln Ile Ile
   275 280 285
   Asn Met Trp Gln Glu Val Gly Arg Ala Met Tyr Ala Pro Pro Ile Glu
   290 295 300
   Gly Asn Ile Thr Cys Lys Ser Asn Ile Thr Gly Leu Leu Leu Val Arg
   305 310 315 320
   Asp Gly Gly Gln Asp Asn Ser Thr Asn Asn Thr Glu Thr Phe Arg Pro
   325 330 335
   Gly Gly Gly Asp Met Arg Asn Asn Trp Arg Ser Glu Leu Tyr Lys Tyr
   340 345 350
   Lys Val Val Glu Ile Lys Pro Leu Gly Ile Ala Pro Thr Lys Ala Lys
   355 360 365
   Arg Arg Val Val Glu Arg Glu Lys Arg Ala Val Gly Ile Gly Ala Val
   370 375 380
   Phe Leu Gly Phe Leu Gly Ala Ala Gly Ser Thr Met Gly Ala Ala Ser
   385 390 395 400
   Ile Thr Leu Thr Val Gln Ala Arg Gln Leu Leu Ser Gly Ile Val Gln
   405 410 415
   Gln Gln Asp Asn Leu Leu Arg Ala Ile Glu Ala Gln Gln His Met Leu
   420 425 430
   Gln Leu Thr Val Trp Gly Ile Lys Gln Leu Gln Ala Arg Val Leu Ala
   435 440 445
   Ile Glu Arg Tyr Leu Gln Asp Gln Gln Leu Leu Gly Ile Trp Gly Cys
   450 455 460
   Ser Gly Lys Leu Ile Cys Thr Thr Ala Val Pro Trp Asn Ala Ser Trp
   465 470 475 480
   Ser Asn Lys Ser Gln Thr Asp Ile Trp Glu Asn Met Thr Trp Met Gln
   485 490 495
   Trp Asp Lys Glu Ile Ser Lys His Thr Asp Thr Ile Tyr Arg Leu Leu
   500 505 510
   Glu Asp Ser Gln Asn Gln Gln Glu Lys Asn Glu Lys Asp Leu Leu Ala
   515 520 525
   Leu Asp Ser Trp Glu Asn Leu Trp Asn Trp Phe Ser Ile Thr Lys Trp
   530 535 540
   Leu Trp Tyr Ile Lys Ile Phe Ile Met Ile Val Gly Gly Leu Ile Gly
   545 550 555 560
   Leu Arg Ile Ile Phe Ala Val Leu Ser Ile Val Ser Arg Val Arg Gln
   565 570 575
   Gly Tyr Ser Pro Leu Ser Phe Gln Thr His Leu Pro Thr Pro Arg Gly
   580 585 590
   Pro Asp Arg Pro Glu Gly Ile Glu Glu Glu Gly Gly Glu Arg Asp Arg
   595 600 605
   Asp Arg Ser Ile Arg Leu Val Asn Gly Ser Leu Ala Leu Ile Trp Asp
   610 615 620
   Asp Leu Arg Ser Leu Cys Leu Phe Ser Tyr His Arg Leu Arg Asp Leu
   625 630 635 640
   Leu Leu Ile Val Thr Arg Ile Val Glu Leu Leu Gly Arg Arg Gly Trp
   645 650 655
   Glu Ala Leu Lys Tyr Trp Trp Asn Leu Leu Gln Tyr Trp Ser Gln Glu
   660 665 670
   Leu Lys Asn Ser Ala Val Ser Leu Leu Asn Ala Thr Ala Ile Ala Val
   675 680 685
   Ala Glu Gly Thr Asp Arg Val Ile Glu Val Val Gln Gly Ala Cys Arg
   690 695 700
   Ala Ile Arg His Ile Pro Arg Arg Met Arg Gln Gly Leu Glu Arg Ile
   705 710 715 720
   Leu Leu
<210> 9
   <211> 3925
   <212> DNA
   <213> Artificial Sequence
<220>
   <223> Description of Artificial Sequence: Synthetic nucleotide construct
<400> 9
   gcatgctgta gagcaagaaa tggagccagt agatcctaga ctagagccct ggaagcatcc 60
   aggaagcagg cctaaaactg cttgtaccaa ttgctattgt aaaaagtgtt gctttcattg 120
   ccaagtttgt ttcataacaa aagccctagg catctcctat ggcaggaaga agcggagaca 180
   gcgacgaaga gctcatcaga acagtcagac tcatcaagct tctctatcaa agcagtaagt 240
   agtacatgta atgcaatcta tacaaataga aatagtagca ttagtagtag caataataat 300
   agcaatagtt gtgtggtcca tagtaatcat agaatatagg aaaatattaa gacaaagaaa 360
   aatagacagg ttaattaata gactaataga aagagcagaa gacagtggca atgagagtga 420
   aggagaaata tcagcacttg tggagatggg ggtggagatg gggcatcatg ctccttggga 480
   tgttgatgat ctgtagtgct acagaaaaat tgtgggtcac agtctattat ggggtacctg 540
   tatggaaaga agcaaaaact actttattct gtgcatcaaa tgctaaagca tatgagaaag 600
   aagtacataa catctgggct acacatgcct gtgtacccac agaccccaac ccacaagaaa 660
   tagttttggg aaatgtaaca gaaaatttta acatgtggaa aaatgacatg gtggatcaga 720
   tgcatgagga tataatcagt ttatgggatc aaagcctaaa gccatgtgta aagttgactt 780
   cactctgtgt cactttaaag tgtagtaatt ttaccgggaa gagtaatgtt acctacaaag 840
   gggatatgga agtaaaaaat tgctctttca atgtaaccac agaaataaga gataagaagc 900
   agaaagtgta tgctcttttt tatagacttg atataacacc acttgatgac aactctagtg 960
   agtatatatt aataaattgc aattcctcaa ccataacaca agcctgtcca aaggtcaatt 1020
   ttgacccaat tcctatacat tattgtgctc cagctggtta tgcgattcta aagtgtaata 1080
   ataagacatt taatgggaca ggaccatgcc ataatgtcag tacagtacaa tgtacacatg 1140
   gaattaagcc agtggtatca actcaactac tgttaaacgg tagcctagca gaaggggaga 1200
   taataattag atctgaaaat ctgacagaca atgtcaaaac aataatagta cactttaatg 1260
   aatctgtaga aattacttgt acaagaccca acaataatac aagaaaaagt ataagcatag 1320
   gaccaggaca agcaatctat gccacaggtg atataatagg agacataaga caagcacact 1380
   gtaacattag taaagaaaat tggaacaaaa ctttacaatg ggtaagggga aaattaaaag 1440
   aacacttccc taataaaaca atagtattta aaccatcctc aggaggggat ctagaaatta 1500
   caacacatag ctttaattgt agaggagaat ttttctattg caacacatca aaactgttta 1560
   atagtacaga caatagtaca cacatgggta cagaaaataa tacaatcatc acaatcccat 1620
   gtagaataaa acaaattata aacatgtggc aggaggtagg acgagcaatg tatgcccccc 1680
   ccatagaagg aaacataaca tgtaaatcaa atatcacagg actactactg gtacgtgatg 1740
   gaggatggga caacagtaca aatgacacag aaacattcag gcctggagga ggagatatga 1800
   gggacaattg gagaagtgaa ttatataaat ataaggtggt agaagtcaag ccattgggaa 1860
   tagcacccac taaggcaaaa aggagagtgg tggagagaga aaaaagagca gtgggaatag 1920
   gagctgtgtt ccttgggttc ttgggagcag caggaagcac tatgggcgcg gcgtcaataa 1980
   cgctgacggt acaggccaga caactgttgt ctggtatagt gcagcagcaa gacaatttgc 2040
   tgagagctat agaggcgcaa caacatatgt tgcaactcac agtctggggc attaagcagc 2100
   tccaggcgag agtcctggct atagaaagat acctacagga tcaacagctc ctagggattt 2160
   ggggctgctc tggaaaactc atctgcacca ctgctgtgcc ttggaacgac agttggagta 2220
   ataaatctca aacagatatt tgggagaaca tgacctggat gcagtgggat agagaaatta 2280
   gtagacacac agacacaata tacaggttgc ttgaagactc acaaaaccag caggagaaaa 2340
   atgaaaaaga tttattagca ttggacagtt ggaaaaattt gtggaattgg tttagcataa 2400
   caaggtggct gtggtatata aaaatattca taatgatagt aggaggcctg ataggtttga 2460
   gaataatttt tgctgtgctc tcgatagtga atagagttag gcagggatac tcaccattat 2520
   cgtttcagac ccacctccca cttccgaggg gagccgacag gcccgaagga atagaagaag 2580
   aaggtggaga gagagacaga gacagatcca ttcgattagt gaccggatcc ttagcactta 2640
   tctgggacga tctgcggagc ctgtgcctct tcagctacca ccgcttgaga gacttactct 2700
   tgattgtaac gaggactgtg gaactcctgg gacgcagagg gtgggaagcc ctcaaatatt 2760
   ggtggaatct cctactgtat tggagtcagg aactaaagaa tagtgctgtt agcttgctca 2820
   acgccacagc catagcagta agacaatatg ggtggagcta tttccatgag gcggtccagg 2880
   ccgtctggag atctgcgaca gagactcttg cgggcgcgtg gggagactta tgggagattc 2940
   ttaggagagg tggaagatgg atactcgcaa tccccaggag gattagacaa gggctcgagc 3000
   tcactctctt gtgagggaca gaaatacaat cagggacagc atatgaatac tccatggaga 3060
   aacccagctg aagagggaga aaaattagca tacagaaaac aaaatatgga tgatatagat 3120
   gaggaagatg atgacttggt aggggtatca gtgaggccaa aagttctcct aagaacaatg 3180
   agttacaaat tggcaataga catgtctcat tttataaaag aaaagggggg actggaaggg 3240
   atttattaca gtgcaagaag acatagaatc ttagacatat acttagaaaa ggaagaaggc 3300
   atcataccag attggcagga ttacacctca ggaccaggaa ttagataccc aaagacattt 3360
   ggctggctat ggaaattagt ccctgtagat gtatcagatg aggcacagga ggatgaggag 3420
   cattacttaa tgcatccagc tcaaacttcc cagtgggatg acccttgggg agaggttcta 3480
   gcatggaagt ttgatccaac tctggcctac acttatgagg catatgttag atacccagaa 3540
   gagtttggaa gcaagtcagg cctgtcagag gaagaggtta gaagaaggct aaccgcaaga 3600
   ggccttctta acatggctga caagaaggaa actcgctgaa acagcaggga ctttccacaa 3660
   ggggatgtta cggggaggta ctggggagga gccggtcggg aacgcccact ttcttgatgt 3720
   ataaatatca ctgcatttcg ctctgtattc agtcgctctg cggagaggct ggcagattga 3780
   gccctgggag gttctctcca gcactagcag gtagagcctg ggtgttccct gctagactct 3840
   caccagcact tggccggtgc tgggcagagt gactccacgc ttgcttactt aaagccctct 3900
   tcaataaagc tgccatttag aagta 3925
<210> 10
   <211> 867
   <212> PRT
   <213> Artificial Sequence
<220>
   <223> Description of Artificial Sequence: Synthetic protein construct
<400> 10
   Met Arg Val Lys Glu Lys Tyr Gln His Leu Trp Arg Trp Gly Trp Arg
   1               5                   10                  15
   Trp Gly Ile Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Lys Thr Thr Leu Phe Cys Ala Ser Asn Ala Lys Ala Tyr Glu Lys Glu
       50                  55                  60
   Val His Asn Ile Trp Ala Thr His Ala Cys Val Pro Thr Asp Pro Asn
   65                  70                  75                  80
   Pro Gln Glu Ile Val Leu Gly Asn Val Thr Glu Asn Phe Asn Met Trp
                   85                  90                  95
   Lys Asn Asp Met Val Asp Gln Met His Glu Asp Ile Ile Ser Leu Trp
               100                 105                 110
   Asp Gln Ser Leu Lys Pro Cys Val Lys Leu Thr Ser Leu Cys Val Thr
           115                 120                 125
   Leu Lys Cys Ser Asn Phe Thr Gly Lys Ser Asn Val Thr Tyr Lys Gly
       130                 135                 140
   Asp Met Glu Val Lys Asn Cys Ser Phe Asn Val Thr Thr Glu Ile Arg
   145                 150                 155                 160
   Asp Lys Lys Gln Lys Val Tyr Ala Leu Phe Tyr Arg Leu Asp Ile Thr
                   165                 170                 175
   Pro Leu Asp Asp Asn Ser Ser Glu Tyr Ile Leu Ile Asn Cys Asn Ser
               180                 185                 190
   Ser Thr Ile Thr Gln Ala Cys Pro Lys Val Asn Phe Asp Pro Ile Pro
           195                 200                 205
   Ile His Tyr Cys Ala Pro Ala Gly Tyr Ala Ile Leu Lys Cys Asn Asn
       210                 215                 220
   Lys Thr Phe Asn Gly Thr Gly Pro Cys His Asn Val Ser Thr Val Gln
   225                 230                 235                 240
   Cys Thr His Gly Ile Lys Pro Val Val Ser Thr Gln Leu Leu Leu Asn
                   245                 250                 255
   Gly Ser Leu Ala Glu Gly Glu Ile Ile Ile Arg Ser Glu Asn Leu Thr
               260                 265                 270
   Asp Asn Val Lys Thr Ile Ile Val His Phe Asn Glu Ser Val Glu Ile
           275                 280                 285
   Thr Cys Thr Arg Pro Asn Asn Asn Thr Arg Lys Ser Ile Ser Ile Gly
       290                 295                 300
   Pro Gly Gln Ala Ile Tyr Ala Thr Gly Asp Ile Ile Gly Asp Ile Arg
   305                 310                 315                 320
   Gln Ala His Cys Asn Ile Ser Lys Glu Asn Trp Asn Lys Thr Leu Gln
                   325                 330                 335
   Trp Val Arg Gly Lys Leu Lys Glu His Phe Pro Asn Lys Thr Ile Val
               340                 345                 350
   Phe Lys Pro Ser Ser Gly Gly Asp Leu Glu Ile Thr Thr His Ser Phe
           355                 360                 365
   Asn Cys Arg Gly Glu Phe Phe Tyr Cys Asn Thr Ser Lys Leu Phe Asn
       370                 375                 380
   Ser Thr Asp Asn Ser Thr His Met Gly Thr Glu Asn Asn Thr Ile Ile
   385                 390                 395                 400
   Thr Ile Pro Cys Arg Ile Lys Gln Ile Ile Asn Met Trp Gln Glu Val
                   405                 410                 415
   Gly Arg Ala Met Tyr Ala Pro Pro Ile Glu Gly Asn Ile Thr Cys Lys
               420                 425                 430
   Ser Asn Ile Thr Gly Leu Leu Leu Val Arg Asp Gly Gly Trp Asp Asn
           435                 440                 445
   Ser Thr Asn Asp Thr Glu Thr Phe Arg Pro Gly Gly Gly Asp Met Arg
       450                 455                 460
   Asp Asn Trp Arg Ser Glu Leu Tyr Lys Tyr Lys Val Val Glu Val Lys
   465                 470                 475                 480
   Pro Leu Gly Ile Ala Pro Thr Lys Ala Lys Arg Arg Val Val Glu Arg
                   485                 490                 495
   Glu Lys Arg Ala Val Gly Ile Gly Ala Val Phe Leu Gly Phe Leu Gly
   500 505 510
   Ala Ala Gly Ser Thr Met Gly Ala Ala Ser Ile Thr Leu Thr Val Gln
   515 520 525
   Ala Arg Gln Leu Leu Ser Gly Ile Val Gln Gln Gln Asp Asn Leu Leu
   530 535 540
   Arg Ala Ile Glu Ala Gln Gln His Met Leu Gln Leu Thr Val Trp Gly
   545 550 555 560
   Ile Lys Gln Leu Gln Ala Arg Val Leu Ala Ile Glu Arg Tyr Leu Gln
   565 570 575
   Asp Gln Gln Leu Leu Gly Ile Trp Gly Cys Ser Gly Lys Leu Ile Cys
   580 585 590
   Thr Thr Ala Val Pro Trp Asn Asp Ser Trp Ser Asn Lys Ser Gln Thr
   595 600 605
   Asp Ile Trp Glu Asn Met Thr Trp Met Gln Trp Asp Arg Glu Ile Ser
   610 615 620
   Arg His Thr Asp Thr Ile Tyr Arg Leu Leu Glu Asp Ser Gln Asn Gln
   625 630 635 640
   Gln Glu Lys Asn Glu Lys Asp Leu Leu Ala Leu Asp Ser Trp Lys Asn
   645 650 655
   Leu Trp Asn Trp Phe Ser Ile Thr Arg Trp Leu Trp Tyr Ile Lys Ile
   660 665 670
   Phe Ile Met Ile Val Gly Gly Leu Ile Gly Leu Arg Ile Ile Phe Ala
   675 680 685
   Val Leu Ser Ile Val Asn Arg Val Arg Gln Gly Tyr Ser Pro Leu Ser
   690 695 700
   Phe Gln Thr His Leu Pro Leu Pro Arg Gly Ala Asp Arg Pro Glu Gly
   705 710 715 720
   Ile Glu Glu Glu Gly Gly Glu Arg Asp Arg Asp Arg Ser Ile Arg Leu
   725 730 735
   Val Thr Gly Ser Leu Ala Leu Ile Trp Asp Asp Leu Arg Ser Leu Cys
   740 745 750
   Leu Phe Ser Tyr His Arg Leu Arg Asp Leu Leu Leu Ile Val Thr Arg
   755 760 765
   Thr Val Glu Leu Leu Gly Arg Arg Gly Trp Glu Ala Leu Lys Tyr Trp
   770 775 780
   Trp Asn Leu Leu Leu Tyr Trp Ser Gln Glu Leu Lys Asn Ser Ala Val
   785 790 795 800
   Ser Leu Leu Asn Ala Thr Ala Ile Ala Val Arg Gln Tyr Gly Trp Ser
   805 810 815
   Tyr Phe His Glu Ala Val Gln Ala Val Trp Arg Ser Ala Thr Glu Thr
   820 825 830
   Leu Ala Gly Ala Trp Gly Asp Leu Trp Glu Ile Leu Arg Arg Gly Gly
   835 840 845
   Arg Trp Ile Leu Ala Ile Pro Arg Arg Ile Arg Gln Gly Leu Glu Leu
   850 855 860
   Thr Leu Leu
   865
<210> 11
   <211> 263
   <212> PRT
   <213> Artificial Sequence
<220>
   <223> Description of Artificial Sequence: Synthetic protein construct
<400> 11
   Met Gly Gly Ala Ile Ser Met Arg Arg Ser Arg Pro Ser Gly Asp Leu
   1 5 10 15
   Arg Gln Arg Leu Leu Arg Ala Arg Gly Glu Thr Tyr Gly Arg Phe Leu
   20 25 30
   Gly Glu Val Glu Asp Gly Tyr Ser Gln Ser Pro Gly Gly Leu Asp Lys
   35 40 45
   Gly Ser Ser Ser Leu Ser Cys Glu Gly Gln Lys Tyr Asn Gln Gly Gln
   50 55 60
   His Met Asn Thr Pro Trp Arg Asn Pro Ala Glu Glu Gly Glu Lys Leu
   65 70 75 80
   Ala Tyr Arg Lys Gln Asn Met Asp Asp Ile Asp Glu Glu Asp Asp Asp
   85 90 95
   Leu Val Gly Val Ser Val Arg Pro Lys Val Leu Leu Arg Thr Met Ser
   100 105 110
   Tyr Lys Leu Ala Ile Asp Met Ser His Phe Ile Lys Glu Lys Gly Gly
   115 120 125
   Leu Glu Gly Ile Tyr Tyr Ser Ala Arg Arg His Arg Ile Leu Asp Ile
   130 135 140
   Tyr Leu Glu Lys Glu Glu Gly Ile Ile Pro Asp Trp Gln Asp Tyr Thr
   145 150 155 160
   Ser Gly Pro Gly Ile Arg Tyr Pro Lys Thr Phe Gly Trp Leu Trp Lys
   165 170 175
   Leu Val Pro Val Asp Val Ser Asp Glu Ala Gln Glu Asp Glu Glu His
   180 185 190
   Tyr Leu Met His Pro Ala Gln Thr Ser Gln Trp Asp Asp Pro Trp Gly
   195 200 205
   Glu Val Leu Ala Trp Lys Phe Asp Pro Thr Leu Ala Tyr Thr Tyr Glu
   210 215 220
   Ala Tyr Val Arg Tyr Pro Glu Glu Phe Gly Ser Lys Ser Gly Leu Ser
   225 230 235 240
   Glu Glu Glu Val Arg Arg Arg Leu Thr Ala Arg Gly Leu Leu Asn Met
   245 250 255
   Ala Asp Lys Lys Glu Thr Arg
   260
<210> 12
   <211> 597
   <212> PRT
   <213> Artificial Sequence
<220>
   <223> Description of Artificial Sequence: Synthetic consensus protein
<400> 12
   Met Arg Val Trp Trp Trp Gly Leu Gly Leu Met Ile Leu Trp Val Thr
   1 5 10 15
   Val Tyr Tyr Gly Val Pro Val Trp Lys Glu Ala Lys Thr Thr Leu Phe
   20 25 30
   Cys Ala Ser Asp Ala Lys Ala Tyr Glu Glu Val His Asn Ile Trp Ala
   35 40 45
   Thr His Ala Cys Val Pro Thr Asp Pro Asn Pro Gln Glu Val Leu Glu
   50 55 60
   Asn Val Thr Glu Asn Phe Asn Met Trp Asp Met Val Asp Gln Met His
   65 70 75 80
   Glu Asp Ile Ile Ser Leu Trp Asp Gln Ser Leu Lys Pro Cys Val Ala
   85 90 95
   Cys Pro Lys Val Asn Phe Asp Pro Ile Pro Ile His Tyr Cys Ala Pro
   100 105 110
   Ala Gly Tyr Ala Ile Leu Lys Cys Asn Asn Lys Thr Phe Asn Gly Gly
   115 120 125
   Pro Cys Asn Val Ser Thr Val Gln Cys Thr His Gly Ile Lys Pro Val
   130 135 140
   Val Ser Thr Gln Leu Leu Leu Asn Gly Ser Leu Ala Glu Ile Ile Ile
   145 150 155 160
   Arg Ser Glu Asn Leu Thr Asn Val Lys Thr Ile Ile Val His Val Ile
   165 170 175
   Cys Thr Gly Gly Ala His Cys Asn Ile Ser Trp Asn Thr Leu Gln Val
   180 185 190
   Lys Leu Glu His Phe Pro Asn Thr Val Phe Lys Pro Ser Ser Gly Gly
   195 200 205
   Asp Leu Glu Ile Thr Thr His Ser Phe Asn Cys Arg Gly Glu Phe Phe
   210 215 220
   Tyr Cys Asn Pro Cys Ile Lys Gln Ile Ile Asn Met Trp Gln Val Gly
   225 230 235 240
   Arg Ala Met Tyr Ala Pro Pro Ile Gly Asn Ile Thr Cys Lys Ser Asn
   245 250 255
   Ile Thr Gly Leu Leu Leu Arg Asp Gly Gly Asn Thr Glu Phe Arg Pro
   260 265 270
   Gly Gly Gly Asp Met Arg Asn Trp Arg Ser Glu Leu Tyr Lys Tyr Lys
   275 280 285
   Val Val Ile Pro Leu Gly Ile Ala Pro Thr Lys Ala Lys Arg Lys Val
   290 295 300
   Val Glu Arg Lys Arg Ala Val Gly Ile Gly Ala Phe Leu Gly Phe Leu
   305 310 315 320
   Gly Ala Ala Gly Ser Thr Met Gly Ala Ala Ser Thr Leu Thr Val Gln
   325 330 335
   Ala Arg Gln Leu Leu Ser Gly Ile Val Gln Gln Gln Asn Leu Leu Arg
   340 345 350
   Ala Ile Glu Ala Gln Gln His Met Leu Gln Leu Thr Val Trp Gly Ile
   355 360 365
   Lys Gln Leu Gln Ala Arg Val Leu Ala Ile Glu Arg Tyr Leu Gln Asp
   370 375 380
   Gln Gln Leu Leu Gly Ile Trp Gly Cys Ser Gly Lys Leu Ile Cys Thr
   385 390 395 400
   Thr Val Pro Trp Asn Ser Trp Ser Lys Ser Asp Ile Trp Asn Met Thr
   405 410 415
   Trp Met Gln Trp Asp Lys Glu Ile Thr Thr Ile Tyr Leu Leu Asp Ser
   420 425 430
   Gln Gln Gln Glu Lys Asn Glu Lys Leu Leu Ala Leu Asp Ser Trp Asn
   435 440 445
   Leu Trp Asn Trp Phe Ile Thr Trp Leu Trp Tyr Ile Lys Ile Phe Ile
   450 455 460
   Met Ile Val Gly Gly Leu Ile Gly Leu Arg Ile Ile Phe Ala Val Leu
   465 470 475 480
   Ser Val Arg Val Arg Gln Gly Tyr Ser Pro Leu Ser Phe Gln Thr Pro
   485 490 495
   Pro Arg Gly Pro Asp Arg Ile Glu Glu Glu Gly Gly Glu Asp Arg Asp
   500 505 510
   Arg Ser Ile Arg Leu Val Gly Leu Ala Leu Trp Asp Asp Leu Arg Ser
   515 520 525
   Leu Cys Leu Phe Ser Tyr His Arg Leu Arg Asp Leu Ile Val Arg Glu
   530 535 540
   Leu Leu Gly Arg Gly Trp Glu Ala Leu Lys Tyr Leu Gln Tyr Trp Glu
   545 550 555 560
   Leu Lys Ser Ala Val Ser Leu Leu Thr Ala Ile Ala Val Ala Glu Gly
   565 570 575
   Thr Asp Arg Val Ile Glu Val Gln Cys Arg Ala Ile Ile Pro Arg Arg
   580 585 590
   Arg Gln Gly Glu Leu
   595
<210> 13
   <211> 839
   <212> PRT
   <213> Artificial Sequence
<220>
   <223> Description of Artificial Sequence: Synthetic construct
<400> 13
   Met Arg Val Arg Gly Ile Gln Arg Asn Tyr Pro Gln Trp Trp Ile Trp
   1 5 10 15
   Gly Ile Leu Gly Phe Leu Met Ile Tyr Asn Gly Met Gly Ser Leu Trp
   20 25 30
   Val Thr Val Tyr Tyr Gly Val Pro Val Trp Lys Glu Ala Lys Thr Thr
   35 40 45
   Leu Phe Cys Ala Ser Asp Ala Lys Ala Tyr Glu Arg Glu Val His Asn
   50 55 60
   Ile Trp Ala Thr His Ala Cys Val Pro Thr Asp Pro Asn Pro Gln Glu
   65 70 75 80
   Leu Val Leu Glu Asn Val Thr Glu Asn Phe Asn Met Trp Glu Asn Asp
   85 90 95
   Met Val Asp Gln Met His Glu Asp Ile Ile Ser Leu Trp Asp Gln Ser
   100 105 110
   Leu Lys Pro Cys Val Lys Leu Thr Pro Leu Cys Val Thr Leu Asn Cys
   115 120 125
   Thr Asp Val Lys Ser Ala Asn Ser Thr Ser Glu Asp Met Arg Asn Cys
   130 135 140
   Ser Phe Asn Val Thr Thr Glu Ile Lys Asp Arg Lys Lys Leu Glu Gln
   145 150 155 160
   Ala Leu Phe Tyr Arg Leu Asp Ile Val Pro Leu Lys Asn Ser Ser Ser
   165 170 175
   Ser Asn Phe Ser Glu Tyr Arg Leu Ile Asn Cys Asn Thr Ser Thr Val
   180 185 190
   Ser Gln Ala Cys Pro Lys Val Asn Phe Asp Pro Ile Pro Ile His Tyr
   195 200 205
   Cys Ala Pro Ala Gly Tyr Ala Ile Leu Lys Cys Asn Asn Lys Thr Phe
   210 215 220
   Asn Gly Ser Gly Pro Cys Asn Asn Val Ser Thr Val Gln Cys Thr His
   225 230 235 240
   Gly Ile Lys Pro Val Val Ser Thr Gln Leu Leu Leu Asn Gly Ser Leu
   245 250 255
   Ala Glu Glu Asp Ile Ile Ile Arg Ser Glu Asn Leu Thr Asn Asn Val
   260 265 270
   Lys Thr Ile Ile Val His Leu Lys Asp Tyr Val Lys Ile Val Cys Thr
   275 280 285
   Arg Pro Asn Asn Asn Thr Arg Lys Ser Met Arg Ile Gly Pro Gly Gln
   290 295 300
   Ala Phe Tyr Ala Thr Gly Glu Ile Ile Gly Asn Ile Arg Glu Ala His
   305 310 315 320
   Cys Asn Ile Ser Gly Ser Lys Trp Asn Asn Thr Leu Gln Arg Val Lys
   325 330 335
   Lys Lys Leu Gly Glu His Phe Pro Asn Asn Thr Thr Val Asp Phe Lys
   340 345 350
   Pro Ser Ser Gly Gly Asp Leu Glu Ile Thr Thr His Ser Phe Asn Cys
   355 360 365
   Arg Gly Glu Phe Phe Tyr Cys Asn Thr Ser Lys Leu Phe Asn Gly Thr
   370 375 380
   Ser Glu Ser Asn Ser Thr Ile Thr Leu Pro Cys Lys Ile Lys Gln Ile
   385 390 395 400
   Ile Asn Met Trp Gln Gly Val Gly Arg Ala Met Tyr Ala Pro Pro Ile
                   405                 410                 415
   Ala Gly Asn Ile Thr Cys Lys Ser Asn Ile Thr Gly Leu Leu Leu Thr
               420                 425                 430
   Arg Asp Gly Gly Asn Gly Asn Gly Thr Glu Ile Phe Arg Pro Gly Gly
           435                 440                 445
   Gly Asp Met Arg Asp Asn Trp Arg Ser Glu Leu Tyr Lys Tyr Lys Val
       450                 455                 460
   Val Lys Ile Glu Pro Leu Gly Ile Ala Pro Thr Lys Ala Lys Arg Arg
   465                 470                 475                 480
   Val Val Arg Arg Gly Lys Arg Ala Val Gly Ile Gly Ala Leu Phe Leu
                   485                 490                 495
   Gly Phe Leu Gly Ala Ala Gly Ser Thr Met Gly Ala Ala Ser Leu Thr
               500                 505                 510
   Leu Thr Val Gln Ala Arg Gln Leu Leu Ser Gly Ile Val Gln Gln Gln
           515                 520                 525
   Asn Asn Leu Leu Arg Ala Ile Glu Ala Gln Gln His Met Leu Gln Leu
       530                 535                 540
   Thr Val Trp Gly Ile Lys Gln Leu Gln Ala Arg Val Leu Ala Ile Glu
   545                 550                 555                 560
   Arg Tyr Leu Gln Asp Gln Gln Leu Leu Gly Ile Trp Gly Cys Ser Gly
                   565                 570                 575
   Lys Leu Ile Cys Thr Thr Asp Val Pro Trp Asn Ser Ser Trp Ser Ser
               580                 585                 590
   Lys Ser Tyr Glu Asp Ile Trp Thr Asn Met Thr Trp Met Gln Trp Asp
           595                 600                 605
   Lys Glu Ile Asn Asn Tyr Thr Asn Thr Ile Tyr Gln Leu Leu Val Asp
       610                 615                 620
   Ser Gln Thr Gln Gln Glu Lys Asn Glu Lys Glu Leu Leu Ala Leu Asp
   625                 630                 635                 640
   Ser Trp Lys Asn Leu Trp Asn Trp Phe Asn Ile Thr Asn Trp Leu Trp
                   645                 650                 655
   Tyr Ile Lys Ile Phe Ile Met Ile Val Gly Gly Leu Ile Gly Leu Arg
               660                 665                 670
   Ile Ile Phe Ala Val Leu Ser Met Val Asn Arg Val Arg Gln Gly Tyr
           675                 680                 685
   Ser Pro Leu Ser Phe Gln Thr Leu Thr Pro Asn Pro Arg Gly Pro Asp
       690                 695                 700
   Arg Leu Gly Arg Ile Glu Glu Glu Gly Gly Glu Gln Asp Arg Asp Arg
   705 710 715 720
   Ser Ile Arg Leu Val Ser Gly Phe Leu Ala Leu Ala Trp Asp Asp Leu
   725 730 735
   Arg Ser Leu Cys Leu Phe Ser Tyr His Arg Leu Arg Asp Cys Ile Leu
   740 745 750
   Ile Val Ala Arg Ala Ala Glu Leu Leu Gly Arg Ser Ser Leu Arg Gly
   755 760 765
   Leu Gln Lys Gly Trp Glu Ala Leu Lys Tyr Leu Gly Ser Leu Val Gln
   770 775 780
   Tyr Trp Gly Leu Glu Leu Lys Lys Ser Ala Val Ser Leu Leu Asp Thr
   785 790 795 800
   Thr Ala Thr Ala Val Ala Glu Gly Thr Asp Arg Val Ile Glu Leu Val
   805 810 815
   Gln Arg Ile Cys Arg Ala Ile Cys Asn Ile Pro Arg Arg Ile Arg Gln
   820 825 830
   Gly Phe Glu Ala Ala Leu Gln
   835
<210> 14
   <211> 1727
   <212> DNA
   <213> Artificial Sequence
<220>
   <223> Description of Artificial Sequence: Synthetic consensus nucleotide
<400> 14
   caggagcgag cgctcggtgg cgatgcccac gggcctgacg cgtgtggcga cctcccagcc 60
   aaatcccacg cacggagtgc tacgcgacca cccgcaacct cccgcctgcc ccaaggtgaa 120
   cttcgacccc atccccatcc actactgcgc ccccgccggc tacgccatcc tgaagtgcaa 180
   caacaagacc ttcaacggcc cggcccctgc acaacgtgtc caccgtgcag tgcacccacg 240
   gcatcaagcc cgtggtgtcc acccagctgc tgctgaacgg ctccctggcc gaggaatcat 300
   catccgctcc gagaacctga ccacaacgtg aagaccatca tcgtgcacta agatcgtgag 360
   atctgcaccg ccccgcccac tgcaacatct ccaatggaac aaaccctgca gggtgaagct 420
   gggagcactt ccccaacaaa ccatcgttca agccctcctc cggcggcgac ctggagatca 480
   ccacccactc cttcaactgc cgcggcgagt tcttctactg caaccccccc tgcatcaagc 540
   agatcatcaa catgtggcag ggtgggcgcg ccatgtacgc cccccccatc gggcaacatc 600
   acctgcaagt ccaacatcac cggcctgctg ctgcgcgacg gcggcaccaa ccaccgagac 660
   ttccgccccg gcggcggcga catgcgcaca actggcgctc cgagctgtac aagtacaagg 720
   tggtgagatc agcccctggg catcgccccc accaaggcca agcgccgcgt ggtggagcgc 780
   gaagcgcgcc gtgggcatcg gcgcctgttc ctgggcttcc tgggcgccgc cggctccacc 840
   atgggcgccg cctcctaccc tgaccgtgca ggcccgccag ctgctgtccg gcatcgtgca 900
   gcagcagaca acctgctgcg cgccatcgag gcccagcagc acatgctgca gctgaccgtg 960
   tggggcatca acagctgcag gcccgcgtgc tggccatcga gcgctacctg caggaccagc 1020
   agctgctggg catctggggc tgctccggca agctgatctg caccaccgcg tgccctggaa 1080
   ccctcctggt cccaagtcca gacatctgga acatgacctg gatgcagtgg gacaaggaga 1140
   tccaaacacc acaccatcta ccctgctggg gactcccaga ccagcaggag aagaacgaga 1200
   aggactgctg gccctggact cctggagaac ctgtggaact ggttccatca ccaatggctg 1260
   tggtacatca agatcttcat catgatcgtg ggcggcctga tcggcctgcg catcatcttc 1320
   gccgtgctgt ccatgtgccg cgtgcgccag ggctactccc ccctgtcctt ccagaccccc 1380
   cacccccgcg gccccgaccg ccggcatcga ggaggagggc ggcgagcgac cgcgaccgct 1440
   ccatccgcct ggtgcggctc ctggccctgc tgggacgacc tgcgctccct gtgcctgttc 1500
   tcctaccacc gcctgcgcga ctctgatcgt gcccgccgga gctgctgggc cgccggctgg 1560
   gaggccctga agtacggcct gtgcagtact ggccggagct gaagaatccg ccttccctgc 1620
   tgacccaccg ccatcgccgt ggccgagggc accgaccgct atcgagtgtc aggcctgccg 1680
   cgccatcgca catcccccgc cgcatcgcca gggctgagcc ctgcgta                1727
<210> 15
   <211> 2520
   <212> DNA
   <213> Artificial Sequence
<220>
   <223> Description of Artificial Sequence: Synthetic nucleotide construct
<400> 15
   atgcgcgtgc gcggcatcca gcgcaactac ccccagtggt ggatctgggg catcctgggc 60
   ttcctgatga tctacaacgg catgggctcc ctgtgggtga ccgtgtacta cggcgtgccc 120
   gtgtggaagg aggccaagac caccctgttc tgcgcctccg acgccaaggc ctacgagcgc 180
   gaggtgcaca acatctgggc cacccacgcc tgcgtgccca ccgaccccaa cccccaggag 240
   ctggtgctgg agaacgtgac cgagaacttc aacatgtggg agaacgacat ggtggaccag 300
   atgcacgagg acatcatctc cctgtgggac cagtccctga agccctgcgt gaagctgacc 360
   cccctgtgcg tgaccctgaa ctgcaccgac gtgaagtccg ccaactccac ctccgaggac 420
   atgcgcaact gctccttcaa cgtgaccacc gagatcaagg accgcaagaa gctggagcag 480
   gccctgttct accgcctgga catcgtgccc ctgaagaact cctcctcctc caacttctcc 540
   gagtaccgcc tgatcaactg caacacctcc accgtgtccc aggcctgccc caaggtgaac 600
   ttcgacccca tccccatcca ctactgcgcc cccgccggct acgccatcct gaagtgcaac 660
   aacaagacct tcaacggctc cggcccctgc aacaacgtgt ccaccgtgca gtgcacccac 720
   ggcatcaagc ccgtggtgtc cacccagctg ctgctgaacg gctccctggc cgaggaggac 780
   atcatcatcc gctccgagaa cctgaccaac aacgtgaaga ccatcatcgt gcacctgaag 840
   gactacgtga agatcgtgtg cacccgcccc aacaacaaca cccgcaagtc catgcgcatc 900
   ggccccggcc aggccttcta cgccaccggc gagatcatcg gcaacatccg cgaggcccac 960
   tgcaacatct ccggctccaa gtggaacaac accctgcagc gcgtgaagaa gaagctgggc 1020
   gagcacttcc ccaacaacac caccatcgac ttcaagccct cctccggcgg cgacctggag 1080
   atcaccaccc actccttcaa ctgccgcggc gagttcttct actgcaacac ctccaagctg 1140
   ttcaacggca cctccgagtc caactccacc atcaccctgc cctgcaagat caagcagatc 1200
   atcaacatgt ggcagggcgt gggccgcgcc atgtacgccc cccccatcgc cggcaacatc 1260
   acctgcaagt ccaacatcac cggcctgctg ctgacccgcg acggcggcaa cggcaacggc 1320
   accgagatct tccgccccgg cggcggcgac atgcgcgaca actggcgctc cgagctgtac 1380
   aagtacaagg tggtgaagat cgagcccctg ggcatcgccc ccaccaaggc caagcgccgc 1440
   gtggtggagc gcggcaagcg cgccgtgggc atcggcgccc tgttcctggg cttcctgggc 1500
   gccgccggct ccaccatggg cgccgcctcc ctgaccctga ccgtgcaggc ccgccagctg 1560
   ctgtccggca tcgtgcagca gcagaacaac ctgctgcgcg ccatcgaggc ccagcagcac 1620
   atgctgcagc tgaccgtgtg gggcatcaag cagctgcagg cccgcgtgct ggccatcgag 1680
   cgctacctgc aggaccagca gctgctgggc atctggggct gctccggcaa gctgatctgc 1740
   accaccgacg tgccctggaa ctcctcctgg tcctccaagt cctacgagga catctggacc 1800
   aacatgacct ggatgcagtg ggacaaggag atcaacaact acaccaacac catctaccag 1860
   ctgctggtgg actcccagac ccagcaggag aagaacgaga aggagctgct ggccctggac 1920
   tcctggaaga acctgtggaa ctggttcaac atcaccaact ggctgtggta catcaagatc 1980
   ttcatcatga tcgtgggcgg cctgatcggc ctgcgcatca tcttcgccgt gctgtccatg 2040
   gtgaaccgcg tgcgccaggg ctactccccc ctgtccttcc agaccctgac ccccaacccc 2100
   cgcggccccg accgcctggg ccgcatcgag gaggagggcg gcgagcagga ccgcgaccgc 2160
   tccatccgcc tggtgtccgg cttcctggcc ctggcctggg acgacctgcg ctccctgtgc 2220
   ctgttctcct accaccgcct gcgcgactgc atcctgatcg tggcccgcgc cgccgagctg 2280
   ctgggccgct cctccctgcg cggcctgcag aagggctggg aggccctgaa gtacctgggc 2340
   tccctggtgc agtactgggg cctggagctg aagaagtccg ccatctccct gctggacacc 2400
   accgccatcg ccgtggccga gggcaccgac cgcatcatcg agctgatcca gcgcatctgc 2460
   cgcgccatct gcaacatccc ccgccgcatc cgccagggct tcgaggccgc cctgcagtaa 2520
<210> 16
   <211> 263
   <212> PRT
   <213> Artificial Sequence
<220>
   <223> Description of Artificial Sequence: Synthetic protein construct
<400> 16
   Met Gly Gly Ala Ile Ser Met Arg Arg Ser Arg Pro Ser Gly Asp Leu
   1               5                   10                  15
   Arg Gln Arg Leu Leu Arg Ala Arg Gly Glu Thr Tyr Gly Arg Leu Leu
               20                  25                  30
   Gly Glu Val Glu Asp Gly Tyr Ser Gln Ser Pro Gly Gly Leu Asp Lys
           35                  40                  45
   Gly Leu Ser Ser Leu Ser Cys Glu Gly Gln Lys Tyr Asn Gln Gly Gln
       50                  55                  60
   Tyr Met Asn Thr Pro Trp Arg Asn Pro Ala Glu Glu Arg Glu Lys Leu
   65                  70                  75                  80
   Ala Tyr Arg Lys Gln Asn Met Asp Asp Ile Asp Glu Glu Asp Asp Asp
                   85                  90                  95
   Leu Val Gly Val Ser Val Arg Pro Lys Val Pro Leu Arg Thr Met Ser
               100                 105                 110
   Tyr Lys Leu Ala Ile Asp Met Ser His Phe Ile Lys Glu Lys Gly Gly
           115                 120                 125
   Leu Glu Gly Ile Tyr Tyr Ser Ala Arg Arg His Arg Ile Leu Asp Ile
       130                 135                 140
   Tyr Leu Glu Lys Glu Glu Gly Ile Ile Pro Asp Trp Gln Asp Tyr Thr
   145                 150                 155                 160
   Ser Gly Pro Gly Ile Arg Tyr Pro Lys Thr Phe Gly Trp Leu Trp Lys
                   165                 170                 175
   Leu Val Pro Val Asn Val Ser Asp Glu Ala Gln Glu Asp Glu Glu His
               180                 185                 190
   Tyr Leu Met His Pro Ala Gln Thr Ser Gln Trp Asp Asp Pro Trp Gly
           195                 200                 205
   Glu Val Leu Ala Trp Lys Phe Asp Pro Thr Leu Ala Tyr Thr Tyr Glu
       210                 215                 220
   Ala Tyr Val Arg Tyr Pro Glu Glu Phe Gly Ser Lys Ser Gly Leu Ser
   225                 230                 235                 240
   Glu Glu Glu Val Arg Arg Arg Leu Thr Ala Arg Gly Leu Leu Asn Met
                   245                 250                 255
   Ala Asp Lys Lys Glu Thr Arg
               260

## Description

### BACKGROUND

Acquired immune deficiency syndrome (AIDS) is a major health threat throughout the world. HIV is the leading cause of AIDS. To date there are no effective vaccines for AIDS. For these reasons there are great efforts to create vaccines and suitable models for testing the vaccines. One target of therapeutic vaccines in the envelope protein of HIV (gp160). During infection, gp160 is cleaved by host cell proteases to from the gp120 and gp41. Barnett et al. (US2002/0146683) describes numerous HIV Env polypeptides in which at least one of the native B-sheet configurations has been modified. The constructs are taught to be used in vaccine compositions. Vajdy et al. (US2005/0175631) teaches pharmaceutical compositions for the treatment of AIDS in which the compositions have an HIV antigen and a mucosal adjuvant. WO 2005/028625 describes clade C HN viruses and applications thereof.

### SUMMARY OF THE INVENTION

Methods and compositions for raising an immune response in a subject by administering an HIV antigen are described. The HIV antigens include HIV clade C polynucleotides and polypeptides. recombinant HIV viral particles and compositions. The recombinant HIV viral particles are particularly useful in animal models for studying HIV.

The aspects and embodiments of the present invention are presented in the accompanying claims.

### BRIEF DESCRIPTION OF THE DRAWINGS

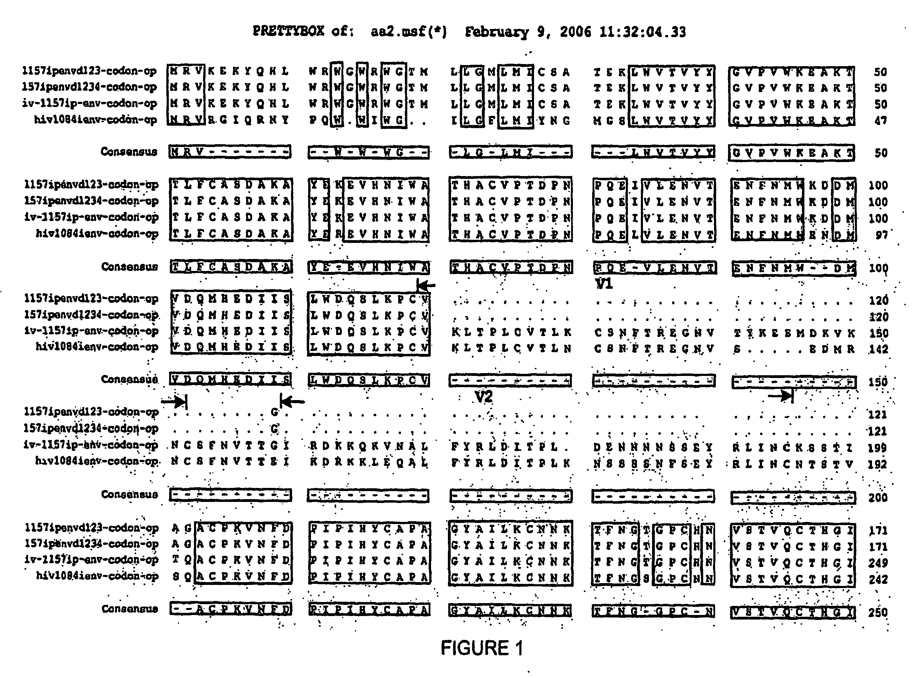

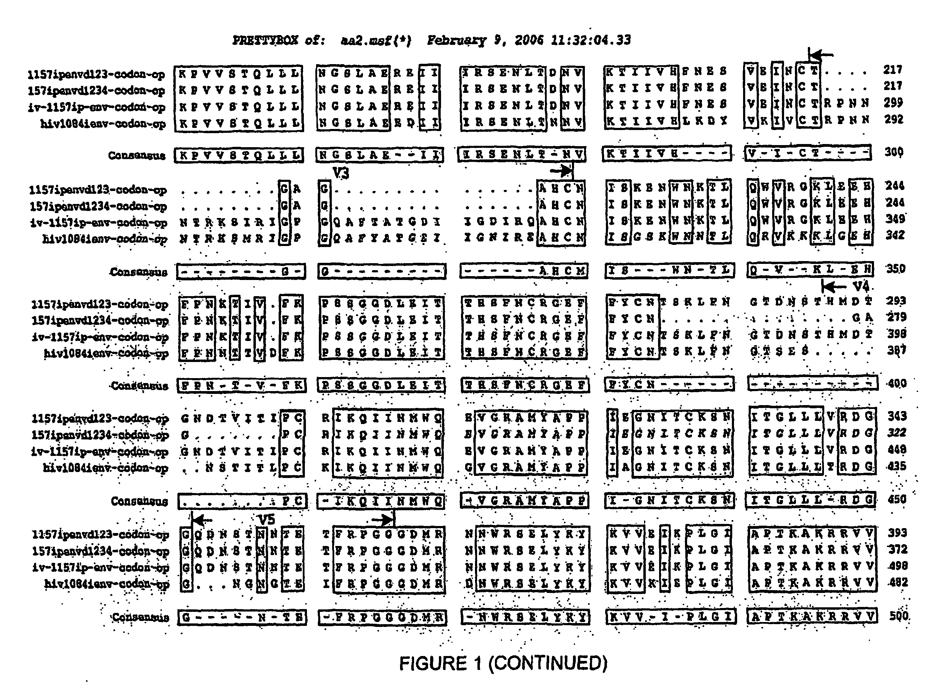

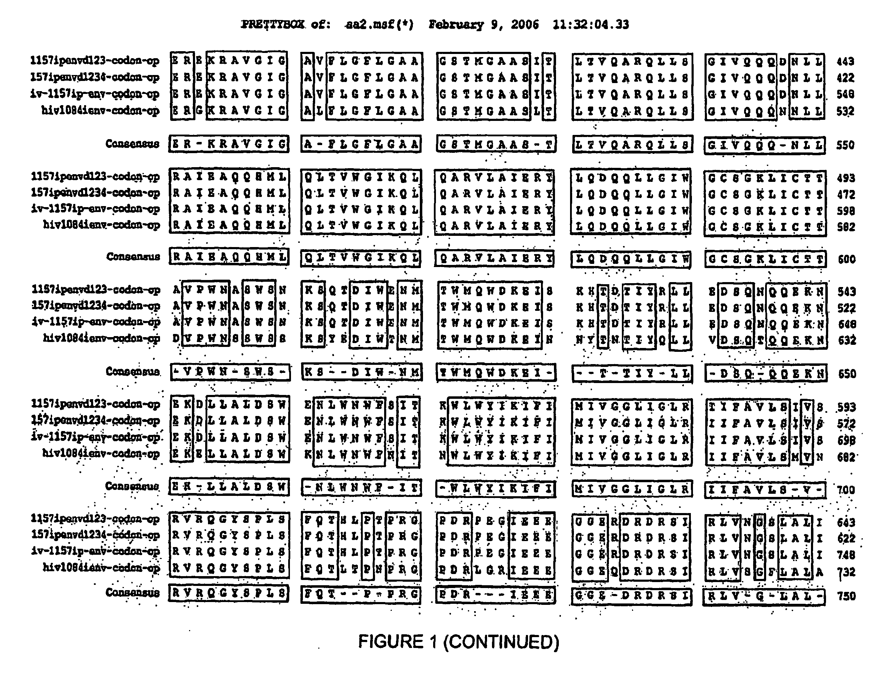

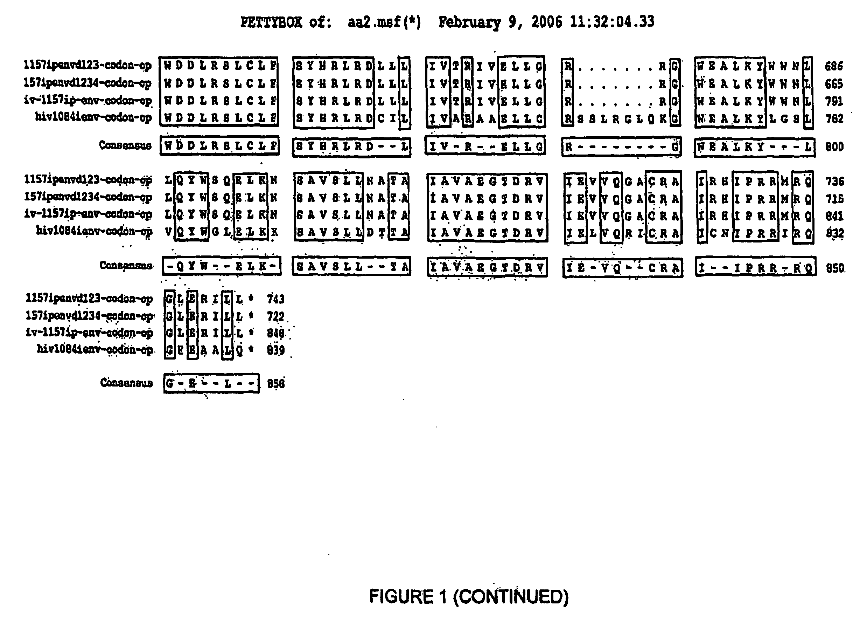

Figure 1 depicts an alignment of the amino acid sequence of the HIV envelope consensus region (SEQ ID NO:12) with the envelope regions of strains 1084i (SEQ ID NO:13), full-length 1157ip (SEQ ID NO:2), 1157ip with deletions in envelope variable regions 1, 2 and 3 (SEQ ID NO: 6), and 1157ip with deletions in envelope variable regions 1, 2, 3 and 4 (SEQ ID NO: 8).

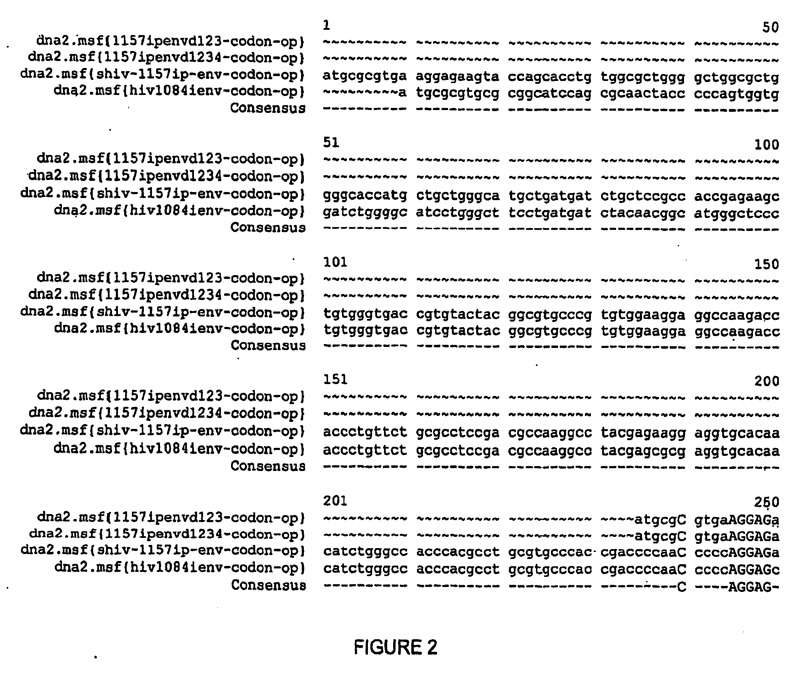

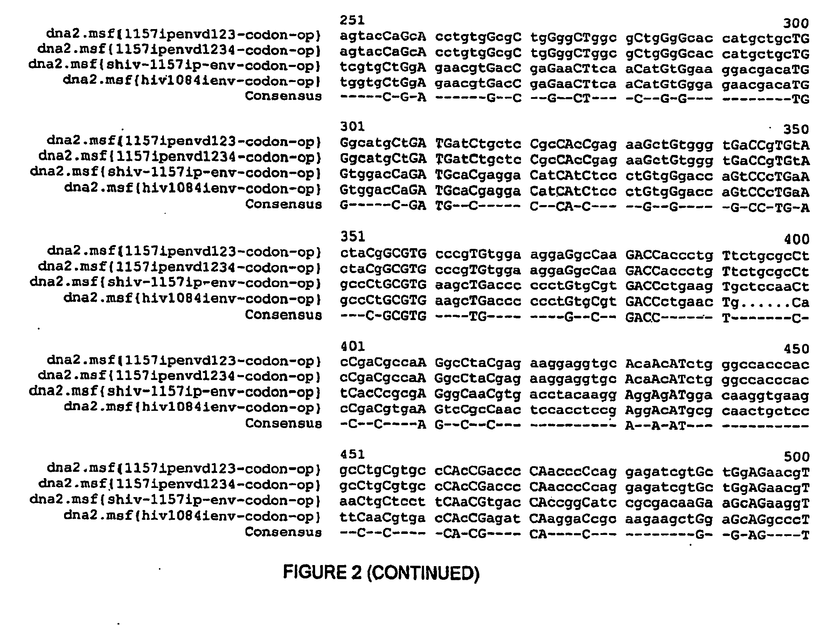

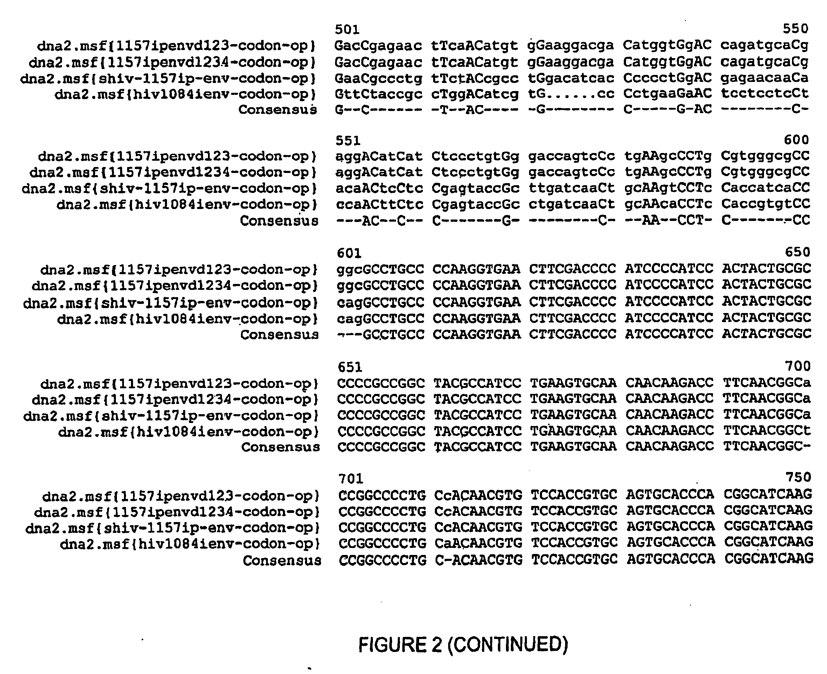

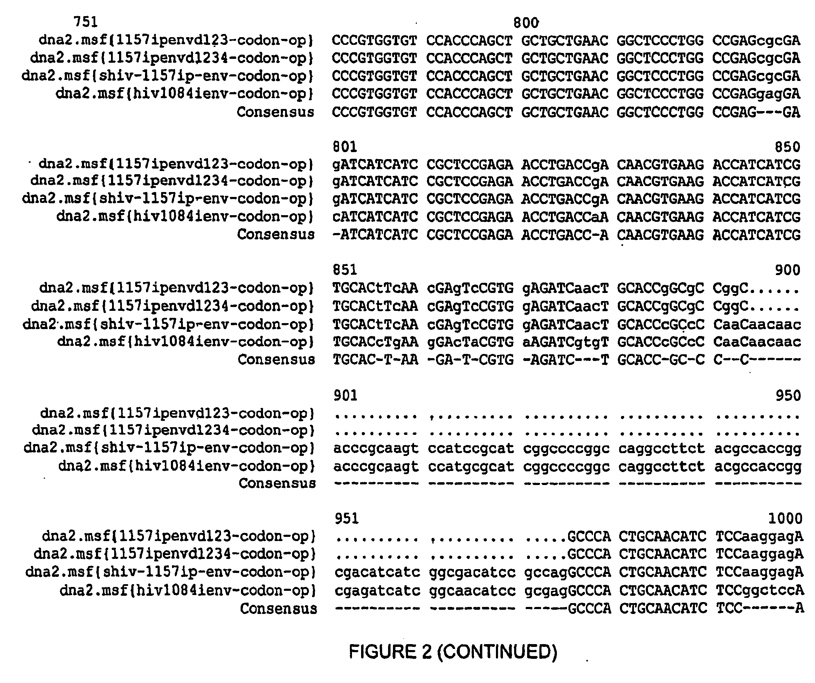

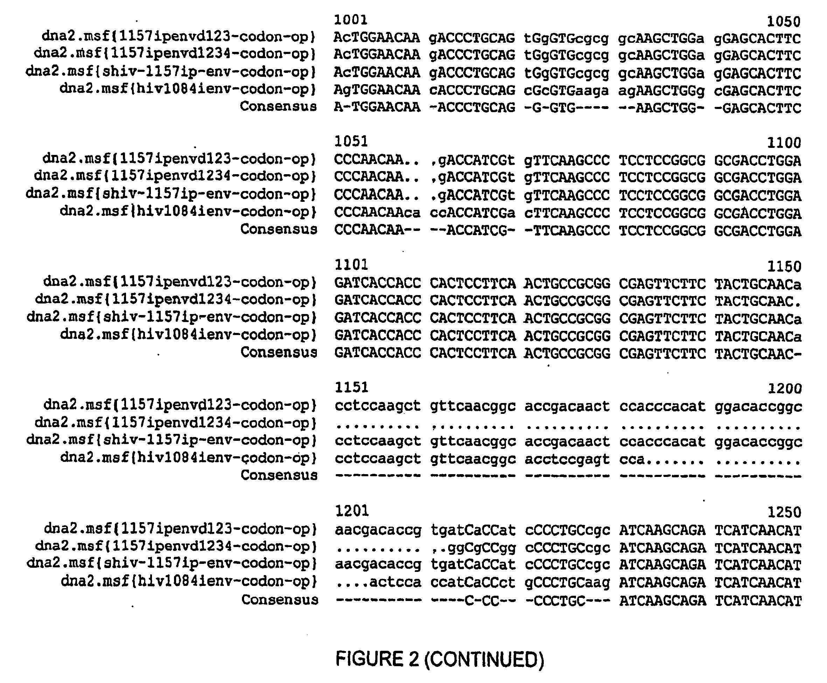

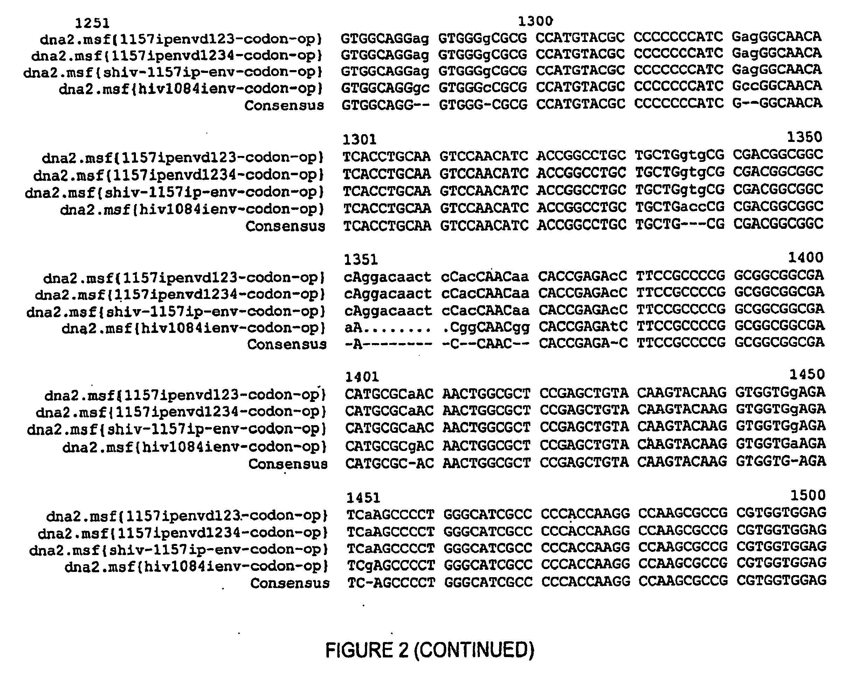

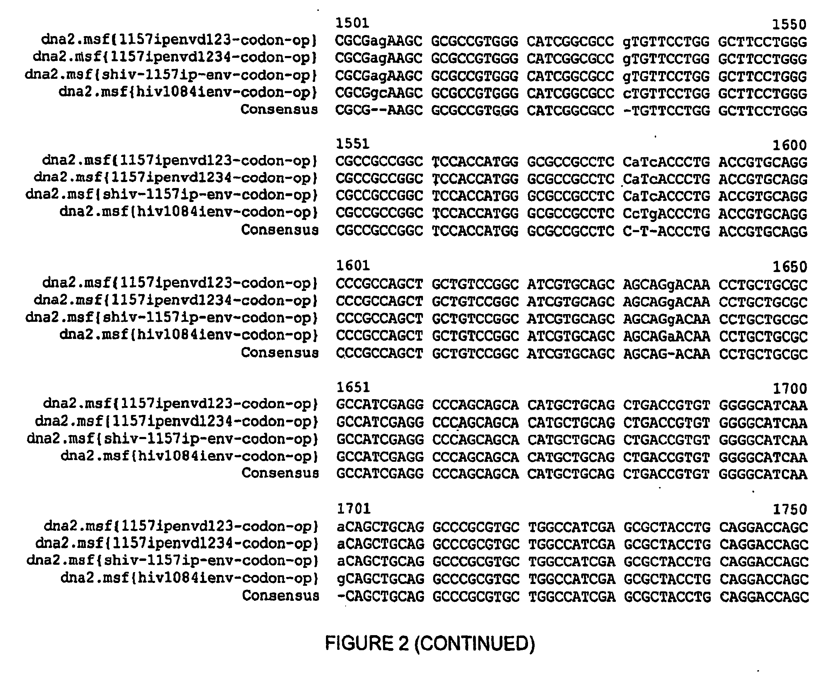

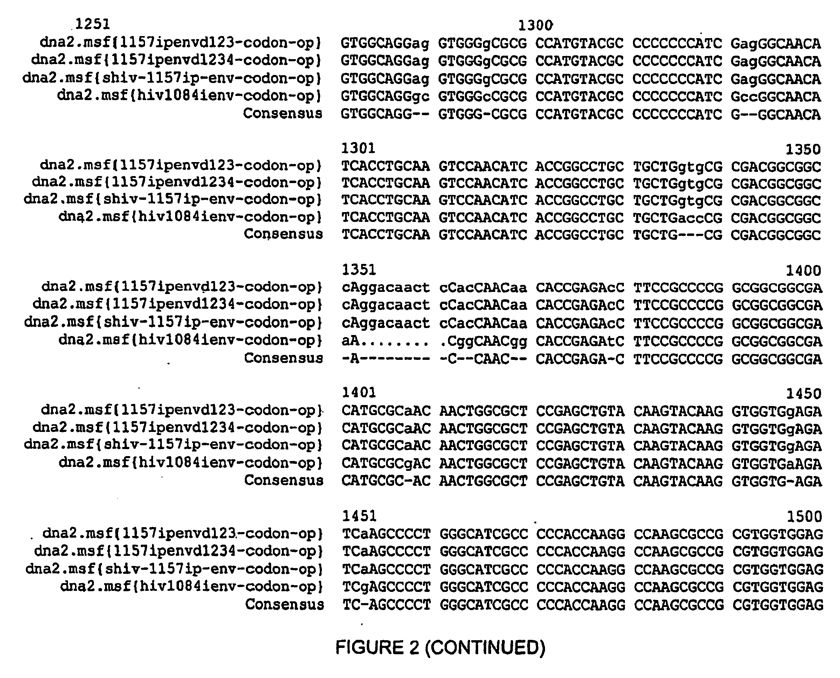

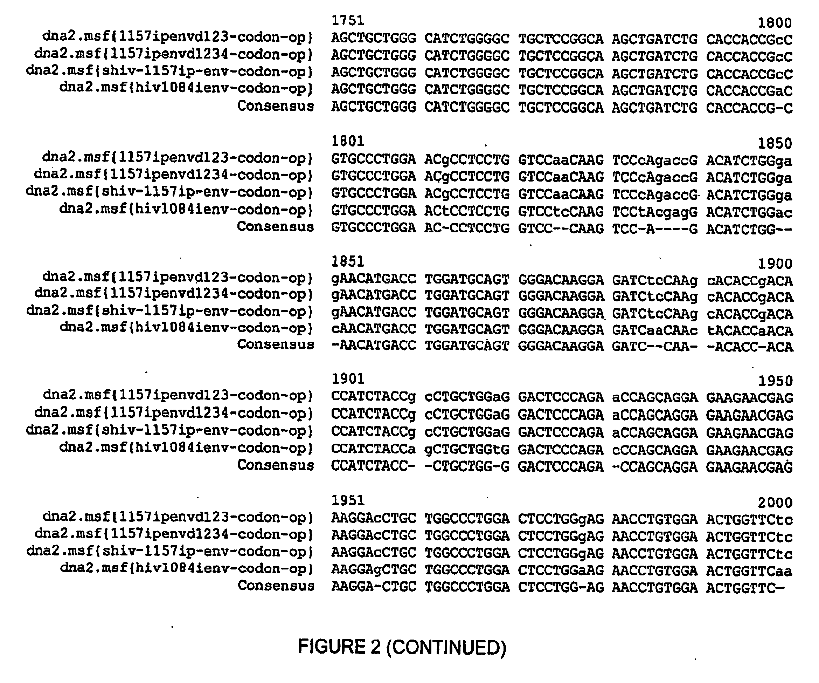

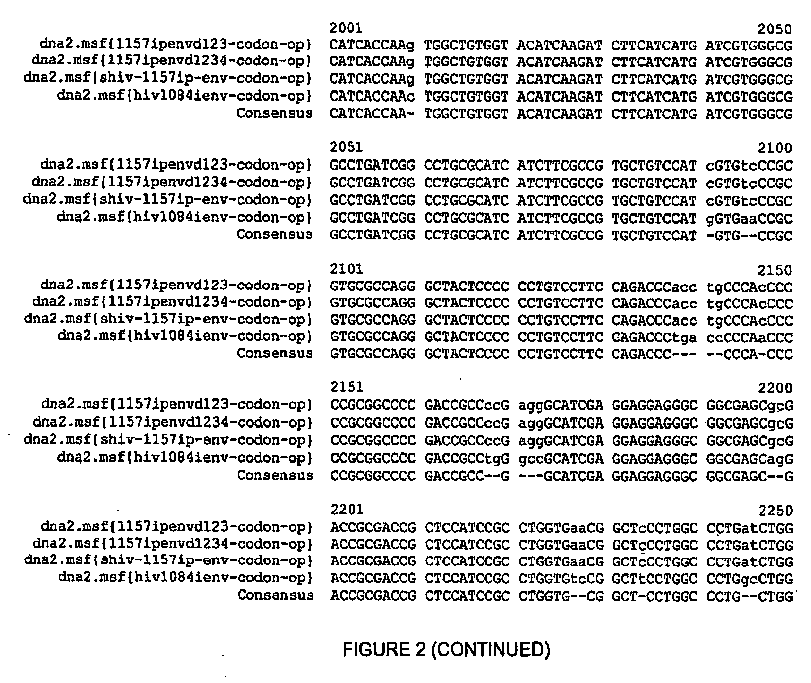

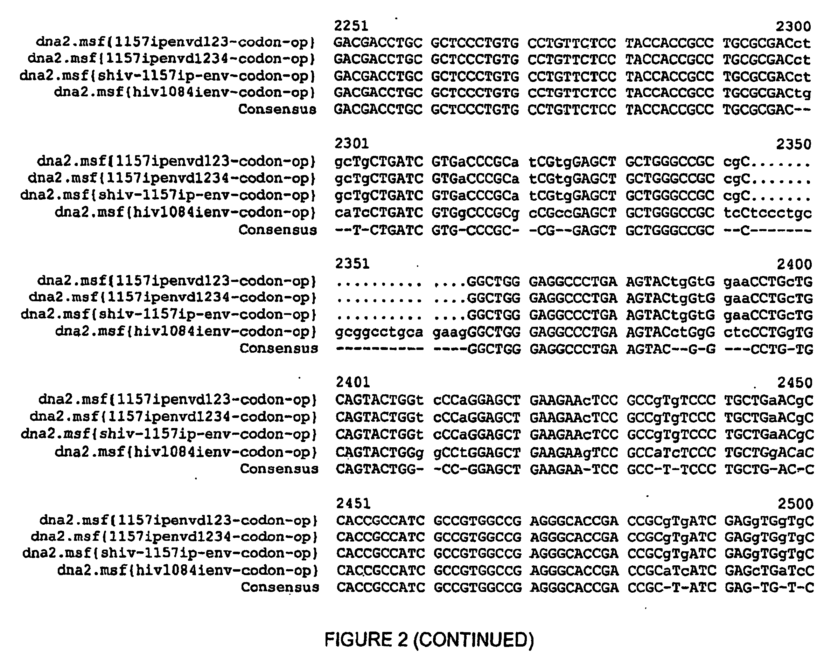

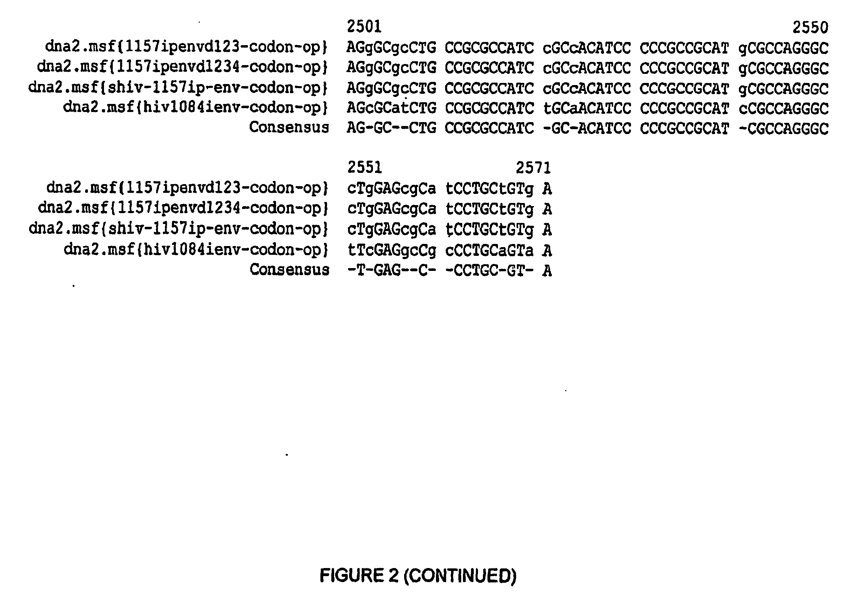

Figure 2 depicts an alignment of the nucleic acid sequence of the HIV envelope consensus region (SEQ ID NO: 14) with the envelope regions of strains 1084i (SEQ ID NO: 15), full-length 1157ip (SEQ ID NO:1), 1157ip with deletions in envelope variable regions 1, 2 and 3 (SEQ ID NO: 5), and 1157ip with deletions in envelope variable regions 1, 2, 3 and 4 (SEQ ID NO: 7).
Figure 3 illustrates neutralizing antibody activity against SHIV-1157ip and SHiV89.6P.
Figure 4 illustrates CD4+T cell counts and vial RNA loads in Rhesus Monkeys.
Figure 5 illustrates construction of SHIV-1157ipd3.
Figure 6 illustrates luciferase expression levels in constructs with increasing numbers of NF-κB sites and replication of SHIV-1157ipd3 v. SKIV-1157ipd3N4.
Figure 7a illustrates coreceptor usage of SHIV-1157ipd3 and SHIV-1157ipd3N4.
Figures 7b and c illustrate virus neutralization with several broadly reactive human nmAbs: b12, 2G12, 2F5 and 4E10, and b12, 2G12, 2F5, 4E10 and 4X, respectively.

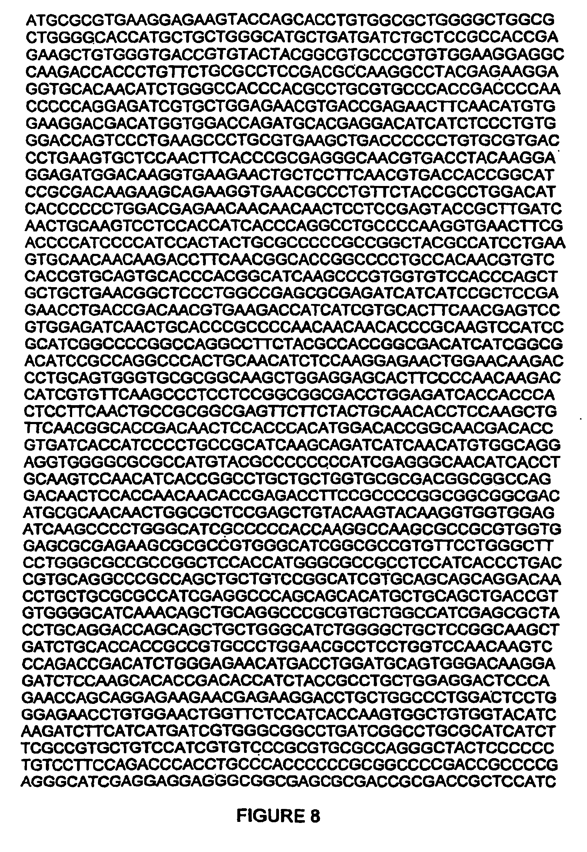

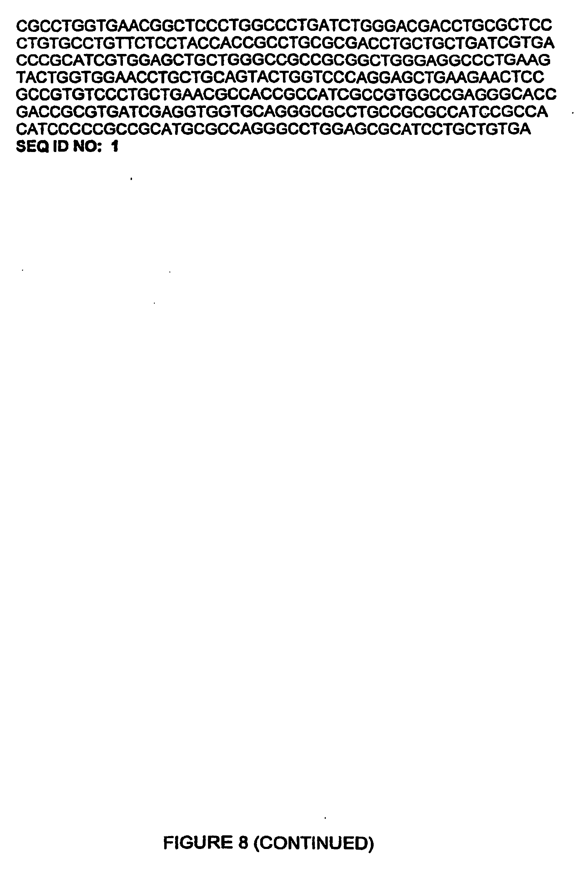

Figure 8 depicts the nucleotide sequence of the envelope region of the construct designated 1157ip env (SEQ ID NO: 1).

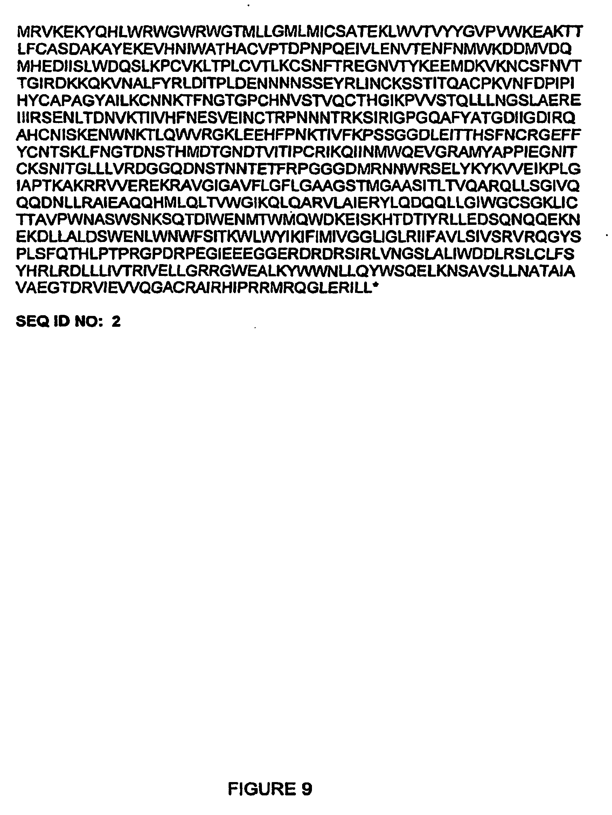

Figure 9 depicts the amino acid sequence of the envelope region of the construct designated 1157ip env. (SEQ ID NO: 2).

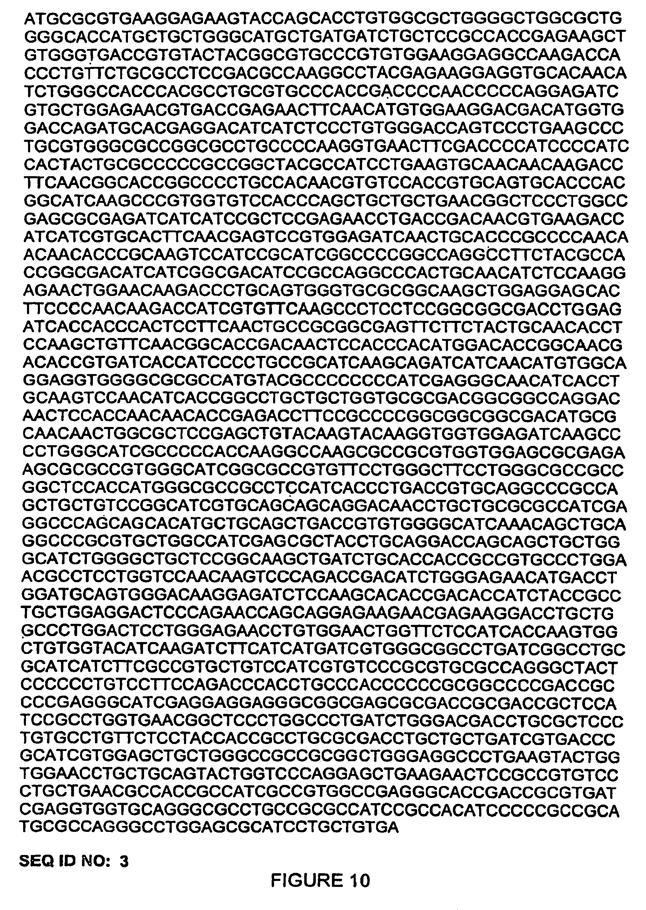

Figure 10 depicts the nucleotide sequence of the envelope region of the construct designated 1157ipdv12 (SEQ ID NO: 3).

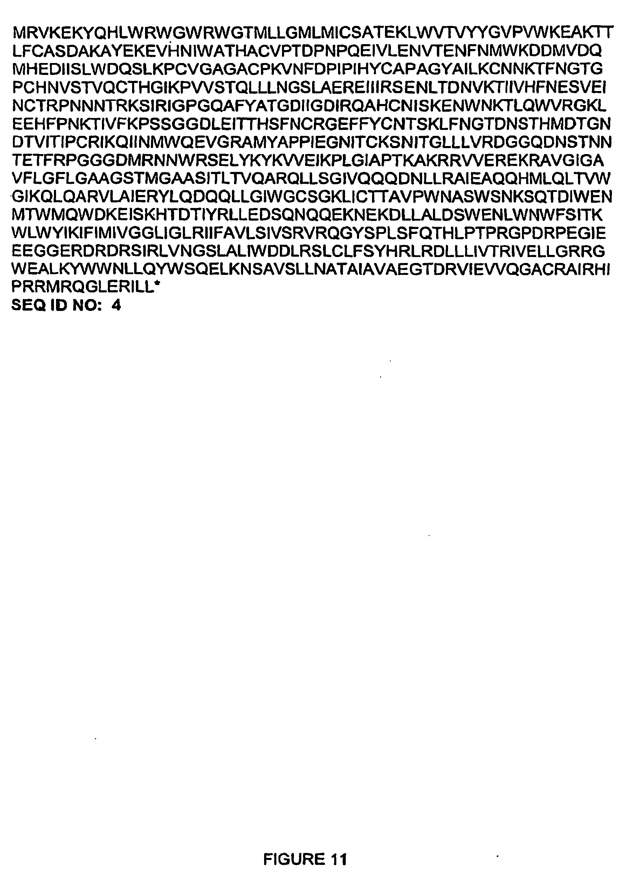

Figure 11 depicts the amino acid sequence of the envelope region of the construct designated 1157ipdv12 (SEQ ID NO: 4).

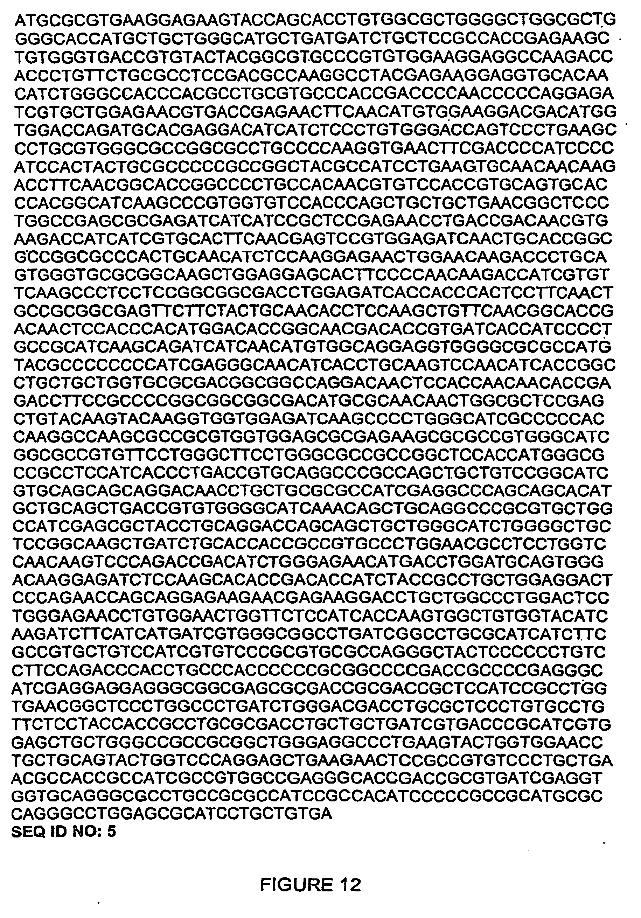

Figure 12 depicts the nucleotide sequence of the envelope region of the construct designated 1157ipdv123 (SEQ ID NO: 5).

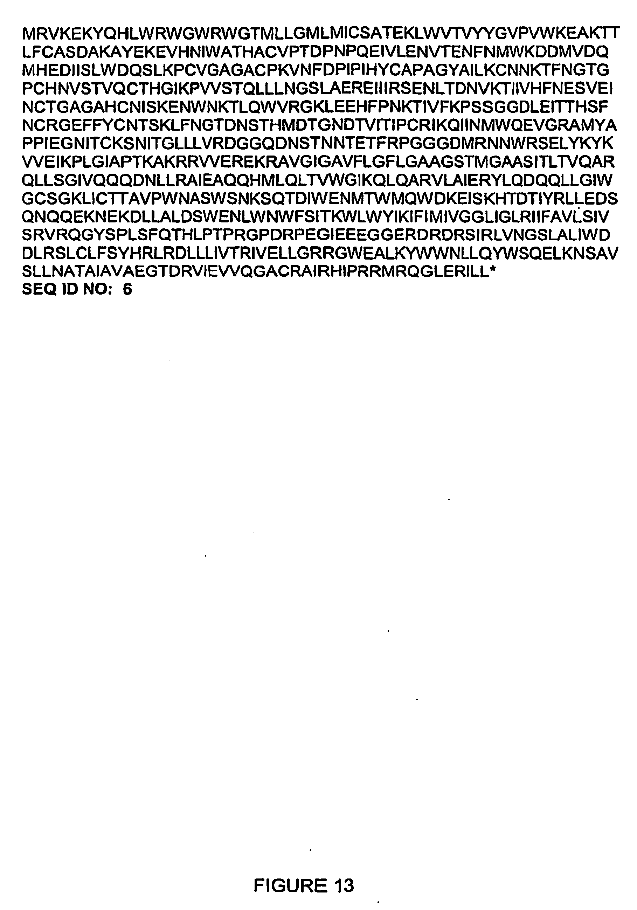

Figure 13 depicts the amino acid sequence of the envelope region of the construct designated 1157ipdv123 (SEQ ID NO: 6).

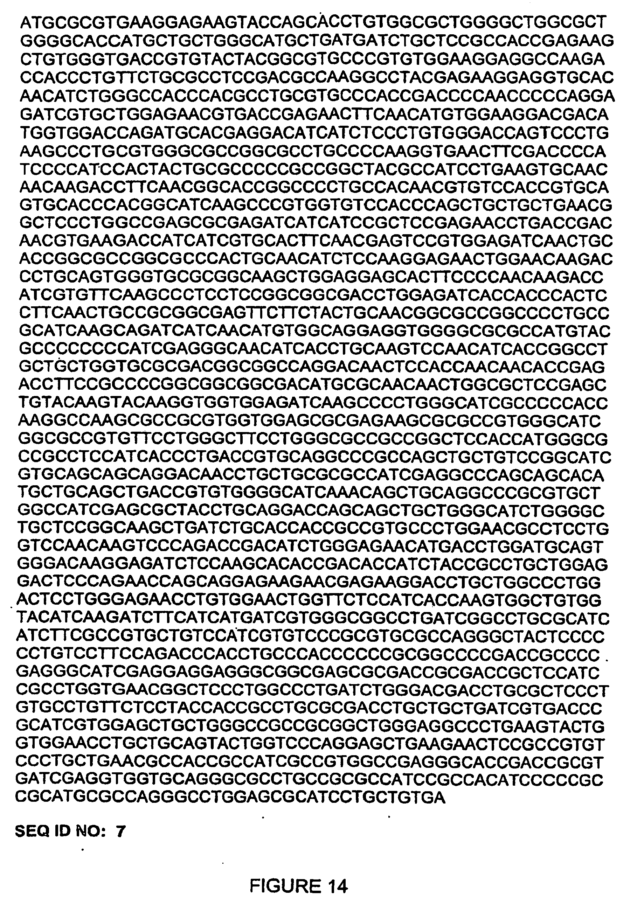

Figure 14 depicts the nucleotide sequence of the envelope region of the construct designated 1157ipdv 1234 (SEQ ID NO: 7).

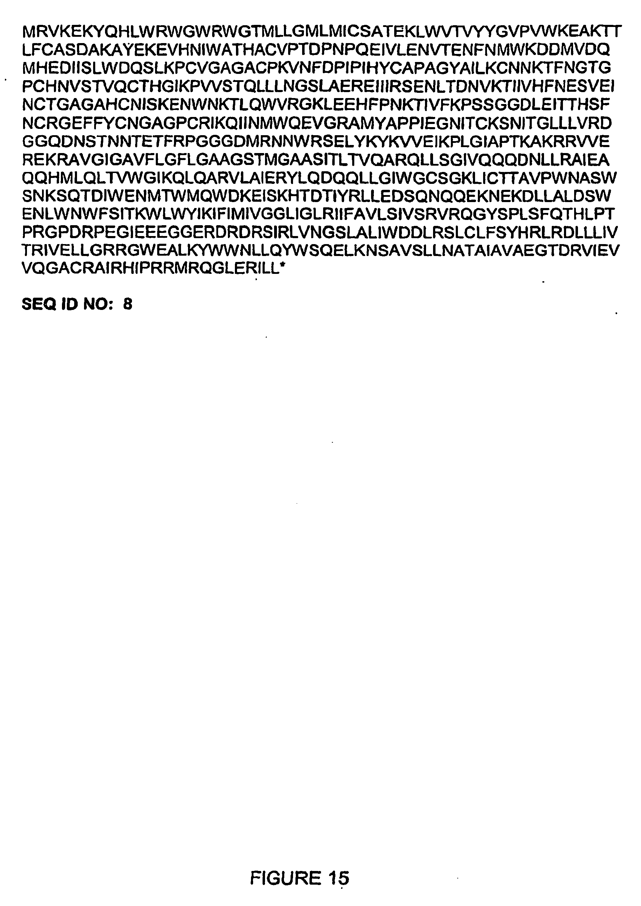

Figure 15 depicts the amino acid sequence of the envelope region of the construct designated 1157ipdv1234 (SEQ ID NO: 8).

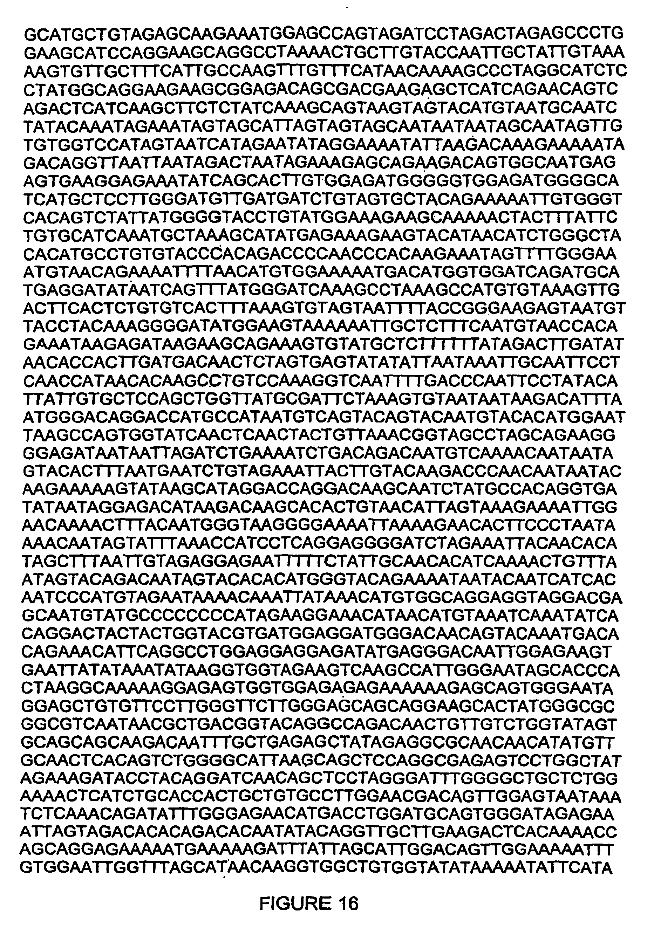

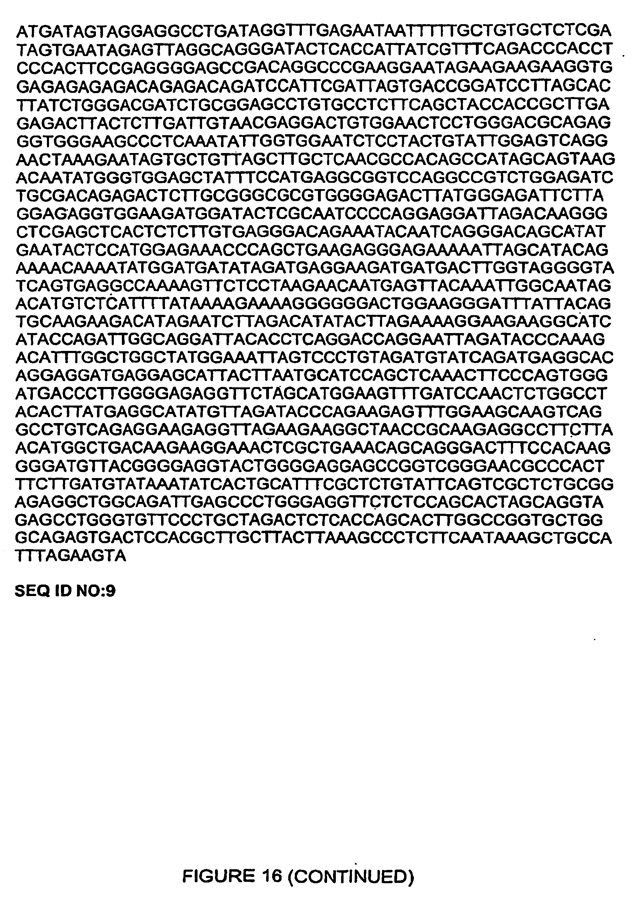

Figure 16 depicts the nucleotide sequence of the envelope region of the construct designated 1157ipd3 (SEQ ID NO: 9).

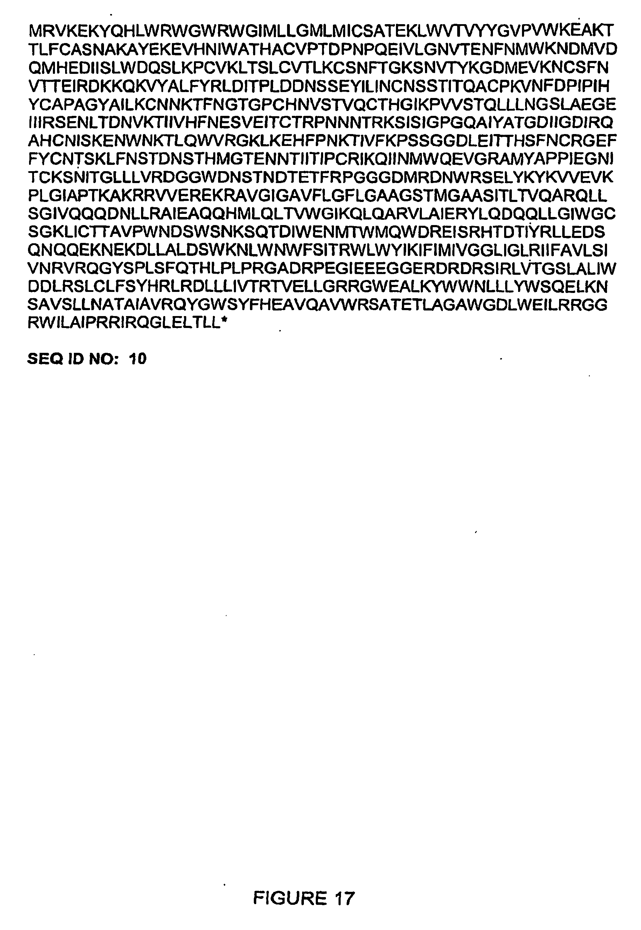

Figure 17 depicts the amino acid sequence of the envelope region of the construct designated 1157ipd3 (SEQ ID NO: 10).

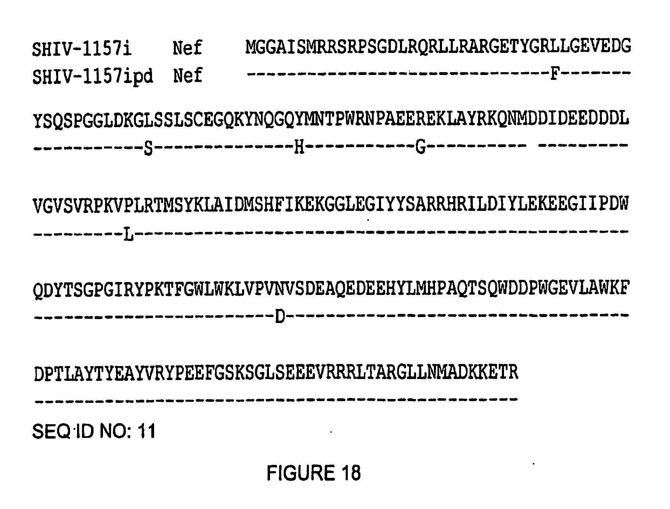

Figure 18 depicts the amino acid sequence of the Nef region of the construct designated 1157ipd (SEQ ID NO: 11).
Figure 19 illustration of the cloning strategy for SHIV-1157i.
Figure 20 illustration of viremia in vaccinated and control macaques.

### DETAILED DESCRIPTION

### Definitions

As used herein, a "polynucleotide" refers to a covalently linked sequence of nucleotides (i.e., ribonucleotides for RNA and deoxyribonucleotides for DNA) in which the 3' position of the pentose of one nucleotide is joined by a phosphodiester linkage to the 5' position of the pentose of the next nucleotide. The term "polynucleotide" includes single- and double-stranded polynucleotides. "Polynucleotide" also embraces a short polynucleotide, often referred to as an oligonucleotide (e.g., a primer or a probe). A polynucleotide has a "5'-terminus" and a "3'-terminus" because polynucleotide phosphodiester linkages occur between the 5' carbon and 3' carbon of the pentose ring of the substituent mononucleotides. The end of a polynucleotide at which a new linkage would be to a 5' carbon is its 5' terminal nucleotide. The end of a polynucleotide at which a new linkage would be to a 3' carbon is its 3' terminal nucleotide. As used herein, a polynucleotide sequence, even if internal to a larger polynucleotide (e.g., a sequence region within a polynucleotide), also can be said to have 5'- and 3'- ends.

The term "polynucleotide" as it is employed herein embraces chemically, enzymatically, or metabolically modified forms of a polynucleotide. The term also captures sequences that include any of the known base analogs of DNA and RNA, and includes modifications such as deletions, additions and substitutions (generally conservative in nature), to the native sequence, so long as the nucleic acid molecule encodes a therapeutic or antigenic protein. Modifications of polynucleotides may have any number of effects including, for example, facilitating expression of the polypeptide product in a host cell.

The polynucleotides used in the present invention include polynucleotides encoding for an immunogenic fragment or derivative thereof. Such immunogenic fragments or derivatives thereof include fragments encoding for a B-cell epitope or a T-cell epitope. The polynucleotides used in the present invention also include polynucleotides encoding for an env gene or recombinant HIV viral particle.

As used herein a "polypeptide" is a molecular chain of amino acids and does not refer to a specific length of the product. Thus, peptides, oligopeptides dimers, multimers, and the like are included within the definition of polypeptide. This term is also intended to include polypeptide that have been subjected to post-expression modifications such as, for example, glycosylations, acetylations, phosphorylations and the like. Furthermore, for purposes of the present invention, a "polypeptide" refers to a protein that includes modifications, such as deletions, additions and substitutions (generally conservative in nature), to the native sequence, as long as the protein maintains the desired activity.

As used herein, "isolated" is meant, when referring to a polynucleotide or a polypeptide, that the indicated molecule is separate and discrete from the whole organism with which the molecule is found in nature or, when the polynucleotide or polypeptide is not found in nature, is sufficiently free of other biological macromolecules so that the polynucleotide or polypeptide can be used for its intended purpose.

As used herein, "antigen" refers to a molecule containing one or more epitopes (either linear, conformational or both) that will stimulate a host's immune system to make a humoral and/or cellular antigen-specific response. The term is used interchangeably with the term "immunogen." Normally, a B-cell epitope will include at least about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes.

Also for purposes of the present disclosure, an "antigen" refers to a protein that includes modifications, such as deletions, additions and substitution (generally conservative in nature), to the native sequence, so long as the protein maintains the ability to elicit an immunological response, as defined herein. Antigens include the polypeptides of SEQ ID NOs: 2, 4, 6, 8,10 and 11 and variants thereof.

As used herein, the term "inducing an immune response" to an antigen or composition is the development in a subject of a humoral and/or a cellular immune response to an antigen present in the composition of interest. A "humoral immune response" refers to an immune response mediated by antibody molecules, while a "cellular immune response" is one mediated by T-lymphocytes and/or other white blood cells. The antigen of interest may also elicit an antibody-mediated immune response. Hence, an immunological response may include one or more of the following effects: the production of antibodies by B-cells; and/or the activation of suppressor, cytotoxic, or helper T-cells and/or T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

A cellular immune response involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, specific effector cells, such as B and plasma cells as well as cytotoxic T cells, against cells displaying peptide antigens in association with MHC molecules on their surface. A "cellular immune response" also refers to the production of cytokines, chemokines and other such molecule produced by activated T-cells and/or other white blood cells, including those derived from CD4+ and CD8+ T-cells. In addition, a chemokine response may be induced by various white blood or endothelial cells in response to an administered antigen.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

A cell-mediated immunological response maybe determined by a number of assays known in the art including, lymphoproliferation (lymphocyte activation) assays, CTL cytotoxic cell assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Cell-mediated immune response may also be determined by the measurement of intracellular cytokines or cytokine secretion by T-cell populations (e.g., by ELISPOT technique), or by measurement of epitope specific T-cells (e.g., by the tetramer technique)(reviewed by McMichael, A. J., and O'Callaghan, C. A., J. Exp. Med. 187(9):1367-1371, 1998; Mcheyzer-Williams, M. G., et al, Immunol. Rev. 150:5-21, 1996; Lalvani, A., et al, J. Exp. Med. 186:859-865, 1997).

As used herein, the terms "Env polypeptide" or "envelope polypeptide" refer to a molecule derived from an envelope protein, e.g. from HIV Env (SEQ ID NO:2 or variants thereof). The envelope protein of HIV-1 is a glycoprotein of about 160 kd (gp160). During virus infection of the host cell, gyp160 is cleaved by host cell proteases to form gp120 and the integral membrane protein, gp41. The gp41 portion is anchored in (and spans) the membrane bilayer of virion, while the gp120 segment protrudes into the surrounding environment. As there is no covalent attachment between gp120 and gyp41, free gp120 is released from the surface of virions and infected cells. Env polypeptides may also include gp 140 polypeptides. Env polypeptides can exist as monomers, dimers or multimers.

As used herein, a "gp120 polypeptide" is meant a molecule derived from a gp120 region of the Env polypeptide. The primary amino acid sequence of gp120 is approximately 511 amino acids. The amino acid sequence of gp120 contains five relatively conserved domains interspersed with five variable domains. The variable domains contain extensive amino acid substitutions, insertions and deletions. A "gp120 polypeptide" includes both single subunits or multimers.

An "Env polypeptide" or "gp120 polypeptide" as defined herein is not limited to a polypeptide having the exact sequence described herein, but includes proteins having

Additionally, the term "Env polypeptide" (e.g., "gp 120 polypeptide") encompasses proteins which include additional modifications to the native sequence, such as additional internal deletions, additions and substitutions. These modifications may be deliberate, as through site-directed mutagenesis, or may be accidental, such as through naturally occurring mutational events. Thus, for example, the Env polypeptide to be used in vaccine compositions, the modifications must be such that immunological activity (i.e., the ability to elicit an antibody response to the polypeptide) is not lost.

As used herein, the term "modified Env polypeptide" is an Env polypeptide (e.g., gp120 as defined above), which has been manipulated to delete or replace all or a part of variable regions V1, V1/V2, V1-V3 or V1-V4. Generally, modified Env (e.g., gp120) polypeptides have enough of the variable regions removed to expose the CD4 binding site, but leave enough of the structure to allow correct folding. Although not all possible variable region modifications have been exemplified herein, it is be understood that other modifications can be envisaged. The modified Env polypeptide may comprise the amino acid sequence of SEQ ID NO: 4, 6, 8 or 10. The modified Env polypeptide is encoded by the nucleotide sequence of SEQ ID NO: 3, 5, 7 or 9 or a variant thereof. .

A variant of a "modified Env polypeptide" or "Env polypeptide" as used herein, refers to an amino acid sequence that is altered by one or more amino acids. The variant may have "conservative" changes, wherein a substituted amino acid has similar structural or chemical properties, e.g., replacement of leucine with isoleucine. More rarely, a variant may have "nonconservative" changes, e.g., replacement of a glyrine with a tryptophan. Similar minor variations may also include amino acid deletions or insertions, or both as long as the variant is capable of inducing an immune response. Guidance in determining which amino acid residues may be substituted, inserted, or deleted without abolishing biological or immunological activity may be found using computer programs well known in the art, for example, DNASTAR software. Variants of the present invention include "modified Env polypeptides" or "Env polypeptides" that are at least 80%, preferably 85%, more preferably 90%, even more preferably 95% and most preferably 96%, 97%, 98% or 99% homologous to a reference amino acid sequence.

"HIV" is Human Immunodeficiency Virus, a virus that causes immunodeficiency by attacking CD4+ cells in the body. The present invention is primarily focused on HIV clade C. HIV is organized into groups and subtypes (clades). Guidance on the application of the invention to different clades may also be found in HIV Sequence Compendium 2002 Kuiken C, Foley B, Freed E, Hahn B, Marx P, McCutchan F, Mellors J, Wolinsky S, and Korber B, editors. Published by the Theoretical Biology and Biophysics Group, Los Alamos National Laboratory, LA-UR number 03-3564. In particular, consensus sequence data for HIV-1 clades A, B, C and D and a number of isolates can be found on pages 490 to 550; consensus sequence data for HIV-2 clades A, B, C and D and a number of isolates can be found one pages 554 to 578.

In one embodiment of the disclosure the HIV clade c env polypeptide is encoded by the polynucleotide sequence of SEQ ID NO:1, 3, 5, 7, or 9 or a variant thereof. In other embodiments of the disclosure the HIV clade C env polypeptide has the amino acid sequence of SEQ ID NO:2, 4, 6, 8, or 10 or a variant thereof.

Methods and compositions for raising an immune response in a subject by administering an HIV antigen are described. The HIV antigens include HIV clade C polynucleotides and polypeptides. Methods and compositions for treating or preventing an HIV infection are also described.

The terms "prevent," "preventing," and "prevention," as used herein, refer to a decrease in the occurrence of HIV in an animal. The prevention may be complete, e.g., the total absence of HIV in a subject. The prevention may also be partial, such that the occurrence of HIV in a subject is less than that which would have occurred without the present invention.

A Method of inducing an immune response in a subject comprising administering an HIV clade C envelope polypeptide in an amount sufficient to induce an immune response in the subject is also described. In one embodiment, the polypeptide comprises the amino acid sequence of SEQ ID NO:2. The HIV clade C envelop polypeptide may be modified so that the V1 region is deleted. The HIV clade C envelope polypeptide may be modified so that the V1/V2 regions are deleted. In yet a further possibility the polypeptide may comprise the amino acid sequence of SEQ ID NO:4. The HIV clade C envelope polypeptide may be modified so that the V1 , V2, and V3 regions are deleted. In yet a further possibility the polypeptide may comprise the amino acid sequence of SEQ ID NO:6. The HIV clade C envelope polypeptide may be modified so that the V1, V2, V3 and V4 regions are deleted. The polypeptide may comprise the amino acid sequence of SEQ ID NO:8. The method may further include administering a second HIV antigen. The second HIV antigen includes gp120 and gp160.

A method of inducing an immune response in a subject comprising administering a polynucleotide encoding a HIV clade C envelope polypeptide in an amount sufficient to induce an immune response in the subject is also described. The polynucleotide may comprise the nucleotide sequence of SEQ ID NO:1. The polynucleotide region encoding the V1 region of the HIV clade C envelop polypeptide may be modified so that the V1 region is deleted. The polynucleotide region encoring the V1/V2 regions of the HIV clade C envelop polypeptide may be modified so that the V1/V2 regions are deleted. The polynucleotide may comprise the nucleotide sequence of SEQ ID NO:3. The polynucleotide regions encoding the V1 V2 and V3 regions of the HIV clade C envelop polypeptide may be modifed so that the V1, V2 and V3 regions are deleted. The polynucleotide may comprise the nucleotide sequence of SEQ ID NO:5. The polynucleotide region encoding the V1, V2, V3 and V4 regions of the HIV clade C envelop polypeptide are may be modified so that the V1, V2, V3 and V4 regions are deleted. The polynucleotide may comprise the nucleotide sequence of SEQ ID NO:7. The method may further include administering a second HIV antigen. The second HIV antigen includes gp120 and gp160.

Isolated and modified HIV clade C envelope polypeptides are disclosed. An isolated HIV envelope polypeptide is also disclosed. In one embodiment of the invention, the polypeptide comprises the amino acid sequence of SEQ ID NO:2.

A modified HIV clade C envelope polypeptide in which the V1 region is deleted and a modified. HIV is described clade C envelope polypeptide in which the V1/V2 regions are deleted is described. In one embodiment of the invention, the polypeptide comprises the amino acid sequence of SEQ ID NO:4. A modified HIV clade C envelope polypepode in which the V1, V2 and V3 regions are deleted is also described. In one embodiment of the invention the polypeptide comprise the amino acid sequence of SEQ ID NO:6. A modified HIV clade C envelope polypeptide in which the V1, V2, V3 and V4 regions are deleted is described. In one embodiment or the invention, the polypeptide comprises the amino acid sequence of SEQ ID NO:8.

Isolated and modified polynucleotides encoding an HIV envelope polypeptide are described. An isolated polynucleotide encoding an HIV clade C envelope polypeptide is also described In one embodiment of the invention the polynucleotide comprises the nucleotide sequence of SEQ ID NO:1. The polynucleotide region encoding the V**1** region of the HIV clade C envelop polypeptide may be modified so that the V1 region is deleted. The polynucleotide regions encoding the V1/V2 regions of the HIV clade C envelop polypeptide may be modified so that the V1/V2 regions are deleted. In yet a further, embodiment of the invention the polynucleotide comprises the nucleotide sequence of SEQ ID NO:3. The polynucleotide regions encoding the V1, V2 and V3 regions of the HIV clade C envelop polypeptide may be modified so that the V1, V2 and V3 regions are deleted. In yet a further, embodiment of the invention the polynucleotide comprises the nucleotide sequence of SEQ ID NO:5. The polynucleotide regions encoding the V1, V2, V3 and V4 regions of the HIV clade C envelop polypeptide may be modified so that the V1, V2, V3 and V4 regions are deleted. In yet a further, embodiment of the invention the polynucleotide comprises the nucleotide sequence of SEQ ID NO:7.

The invention also provides for vectors and compositions which include the HIV clade C envelope polynucleotides and polypeptides of the invention.

In another aspect, the invention provides for an HIV recombinant viral particle that has a 1157 ipd envelope region having the nucleotide sequence of SEQ ID NO: 9. In another aspect, the invention provides an HIV recombinant viral particle which includes a 1157 ipd3 envelope region having the amino acid nucleotide sequence of SEQ ID NO: 10. In either of the last two aspects the HIV recombinant viral particle may further include the Nef amino acid sequence of SEQ ID NO:11. In further embodiments of the invention, the recombinant viral particle has two or more NF-kB regions. The HIV recombinant viral particles may be include in a composition and may be used in a method for assessing the efficacy of a candidate AIDS vaccine in a subject. The method includes the steps of administering a candidate AIDS vaccine to a non-human primate, infecting the non-human primate with the viral particle and detecting disease progression.

### Polypeptides and polynucleotides of the invention

A polynucleotide of the present invention may be employed for producing a polypeptide by recombinant techniques. Such a polypeptide may be useful, for example, as an antigen for the induction of an immune response or as a reagent for the affinity purification of such an antibody.

In one embodiment, the polypeptides are generated using recombinant techniques, well known in the art. In this regard, oligonucleotide probes can be devised based on the known sequences of the Env polypeptide genome and used to probe genomic or cDNA libraries for Env genes. The gene can then be further isolated using standard techniques and, e.g., restriction enzymes employed to truncate the gene at desired portions of the full-length sequence. Similarly, the Env gene(s) can be isolated directly from cells and tissues containing the same, using known techniques, such as phenol extraction and the sequence further manipulated to produce the desired truncations. See, e.g., Sambrook et al., supra, for a description of techniques used to obtain and isolate DNA.

The genes encoding the Env (e.g., full length and variable region deletion) polypeptides can be produced synthetically, based on the known sequences, e.g., sequences disclosed herein. The nucleotide sequence can be designed with the appropriate codons for the particular amino acid sequence desired. The complete sequence is generally assembled from overlapping oligonucleotides prepared by standard methods and assembled into a complete coding sequence. See, e.g., Edge (1981) Nature 292:756; Nambair et al. (1984) Science 223:1299; Jay et al. (1984) J. Biol. Chem. 259:6311; Stemmer et al. (1995) Gene 164:49-53.

Recombinant techniques are readily used to clone a gene encoding an Env polypeptide gene which can then be mutagenized in vitro by the replacement of the appropriate base pair(s) to result in the codon for the desired amino acid. Such a change can include as little as one base pair, effecting a change in a single amino acid, or can encompass several base pair changes. Alternatively, the mutations can be effected using a mismatched primer which hybridizes to the parent nucleotide sequence (generally cDNA corresponding to the RNA sequence), at a temperature below the melting temperature of the mismatched duplex. The primer can be made specific by keeping primer length and base composition within relatively narrow limits and by keeping the mutant base centrally located. See, e.g., Innis et al, (1990) PCR Applications: Protocols for Functional Genomics; Zoller and Smith, Methods Enzymol. (1983) 100:468. Primer extension is effected using DNA polymerase, the product cloned and clones containing the mutated DNA, derived by segregation of the primer extended strand, selected. Selection can be accomplished using the mutant primer as a hybridization probe. The technique is also applicable for generating multiple point mutations. See, e.g., Dalbie-McFarland et al. Proc. Natl. Acad. Sci USA (1982) 79:6409.

Once coding sequences for the desired proteins have been isolated or synthesized, they can be cloned into any suitable vector or replicon for expression. A wide variety of vectors encoding modified polypeptides can be generated by creating expression constructs which operably link the polynucleotides of the invention. The construct is then introduced into a host cell for polypeptide expression. Non-limiting examples of such combinations are discussed in the Examples.

Numerous cloning vectors are known to those of skill in the art, and the selection of an appropriate cloning vector is a matter of choice. Examples of recombinant DNA vectors for cloning and host cells which they can transform include the bacteriophage lambda. (E. coli), pBR322 (E. coli), pACYC177 (E. coli), pKT230 (gram-negative bacteria), pGV1106 (gram-negative bacteria), pLAFR1 (gram-negative bacteria), pME290 (non-E. coli gram-negative bacteria), pHV14 (E. coli and Bacillus subtilis), pBD9 (Bacillus), pIJ61 (Streptomyces), pUC6 (Streptomyces), YIp5 (Saccharomyces), YCp19 (Saccharomyces) and bovine papilloma virus (mammalian cells). See, generally, DNA Cloning: Vols. I & II, supra; Sambrook et al., supra; B. Perbal, supra.

Insect cell expression systems, such as baculovirus systems, can also be used and are known to those of skill in the art and described in, e.g., Summers and Smith, Texas Agricultural Experiment Station Bulletin No. 1555 (1987). Materials and methods for baculovirus/insect cell expression systems are commercially available in kit form from, inter alia, Invitrogen, San Diego Calif. ("MaxBac" kit).

Viral systems, such as a vaccinia based infection/transfection system, as described in Tomei et al., J. Virol. (1993) 67:4017-4026 and Selby et al., J. Gen. Virol. (1993) 74:1103-1113, will also find use with the present invention. In this system, cells are first transfected in vitro with a vaccinia virus recombinant that encodes the bacteriophage T7 RNA polymerase. Following infection, cells are transfected with the DNA of interest, driven by a T7 promoter. The polymerase expressed in the cytoplasm from the vaccinia virus recombinant transcribes the transfected DNA into RNA which is then translated into protein by the host translational machinery. The method provides for high level, transient, cytoplasmic production of large quantities of RNA and its translation product(s).

The gene can be placed under the control of a promoter, ribosome binding site (for bacterial expression) and, optionally, an operator (collectively referred to herein as "control" elements), so that the DNA sequence encoding the desired HIV clad C envelope polypeptide is transcribed into RNA in the host cell transformed by a vector containing this expression construction. The coding sequence may or may not contain a signal peptide or leader sequence. With the present invention, both the naturally occurring signal peptides or heterologous sequences can be used. Leader sequences can be removed by the host in post-translational processing. See, e.g., U.S. Pat. Nos. 4,431,739; 4,425,437; 4,338,397. Such sequences include, but are not limited to, the TPA leader, as well as the honey bee mellitin signal sequence.

Other regulatory sequences may also be desirable which allow for regulation of expression of the protein sequences relative to the growth of the host cell. Such regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

The control sequences and other regulatory sequences may be ligated to the coding sequence prior to insertion into a vector. Alternatively, the coding sequence can be cloned directly into an expression vector which already contains the control sequences and an appropriate restriction site.

In some cases it may be necessary to modify the coding sequence so that it may be attached to the control sequences with the appropriate orientation; i.e., to maintain the proper reading frame. Mutants or analogs may be prepared by the deletion of a portion of the sequence encoding the protein, by insertion of a sequence, and/or by substitution of one or more nucleotides within the sequence. Techniques for modifying nucleotide sequences, such as site-directed mutagenesis, are well known to those skilled in the art. See, e.g., Sambrook et al., supra; DNA Cloning, Vols. I and II, supra; Nucleic Acid Hybridization, supra.

The expression vector is then used to transform an appropriate host cell. A number of mammalian cell lines are known in the art and include immortalized cell lines available from the American Type Culture Collection (ATCC), such as, but not limited to, Chinese hamster ovary (CHO) cells, HeLa cells, baby hamster kidney (BHK) cells, monkey kidney cells (COS), human hepatocellular carcinoma cells (e.g., Hep G2), Vero293 cells, as well as others. Similarly, bacterial hosts such as E. coli, Bacillus subtilis, and Streptococcus spp., will find use with the present expression constructs. Yeast hosts useful in the present invention include inter alia, Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe and Yarrowia lipolytica. Insect cells for use with baculovirus expression vectors include, inter alia, Aedes aegypti, Autographa californica, Bombyx mori, Drosophila melanogaster, Spodoptera frugiperda, and Trichoplusia ni.

Depending on the expression system and host selected, the proteins of the present invention are produced by growing host cells transformed by an expression vector described above under conditions whereby the protein of interest is expressed. The selection of the appropriate growth conditions is within the skill of the art.

In one embodiment, the transformed cells secrete the polypeptide product into the surrounding media. Certain regulatory sequences can be included in the vector to enhance secretion of the protein product, for example using a tissue plasminogen activator (TPA) leader sequence, a gamma-interferon signal sequence or other signal peptide sequences from known secretory proteins. The secreted polypeptide product can then be isolated by various techniques described herein, for example, using standard purification techniques such as but not limited to, hydroxyapatite resins, column chromatography, ion-exchange chromatography, size-exclusion chromatography, electrophoresis, HPLC, immunoadsorbent techniques, affinity chromatography, immunoprecipitation, and the like.

Alternatively, the transformed cells are disrupted, using chemical, physical or mechanical means, which lyse the cells yet keep the HIV clad C envelope polypeptide substantially intact. Intracellular proteins can also be obtained by removing components from the cell wall or membrane, e.g., by the use of detergents or organic solvents, such that leakage of the Env polypeptides occurs. Such methods are known to those of skill in the art and are described in, e.g., Protein Purification Applications: A Practical Approach, (E. L. V. Harris and S. Angal, Eds., 1990).

For example, methods of disrupting cells for use with the present invention include but are not limited to: sonication or ultrasonication; agitation; liquid or solid extrusion; heat treatment; freeze-thaw; desiccation; explosive decompression; osmotic shock; treatment with lytic enzymes including proteases such as trypsin, neuraminidase and lysozyme; alkali treatment; and the use of detergents and solvents such as bile salts, sodium dodecylsulphate, Triton, NP40 and CHAPS. The particular technique used to disrupt the cells is largely a matter of choice and will depend on the cell type in which the polypeptide is expressed, culture conditions and any pre-treatment used.

Following disruption of the cells, cellular debris is removed, generally by centrifugation, and the intracellularly produced Env polypeptides are further purified, using standard purification techniques such as but not limited to, column chromatography, ion-exchange chromatography, size-exclusion chromatography, electrophoresis, HPLC, immunoadsorbent techniques, affinity chromatography, immunoprecipitation, and the like.

For example, one method for obtaining the intracellular HIV clade c envelope polypeptides of the present invention involves affinity purification, such as by immunoaffinity chromatography using anti-Env specific antibodies, or by lectin affinity chromatography. Particularly preferred lectin resins are those that recognize mannose moieties such as but not limited to resins derived from Galanthus nivalis agglutinin (GNA), Lens culinaris agglutinin (LCA or lentil lectin), Pisum sativum agglutinin (PSA or pea lectin), Narcissus pseudonarcissus agglutinin (NPA) and Allium ursinum agglutinin (AUA). The choice of a suitable affinity resin is within the skill in the art. After affinity purification, the Env polypeptides can be further purified using conventional techniques well known in the art, such as by any of the techniques described above.

Relatively small polypeptides, i.e., up to about 50 amino acids in length, can be conveniently synthesized chemically, for example by any of several techniques that are known to those skilled in the peptide art. In general, these methods employ the sequential addition of one or more amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complementary (amino or carboxyl) group suitably protected, under conditions that allow for the formation of an amide linkage. The protecting group is then removed from the newly added amino acid residue and the next amino acid (suitably protected) is then added, and so forth. After the desired amino acids have been linked in the proper sequence, any remaining protecting groups (and any solid support, if solid phase synthesis techniques are used) are removed sequentially or concurrently, to render the final polypeptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide. See, e.g., J. M. Stewart and J. D. Young, Solid Phase Peptide Synthesis (Pierce Chemical Co., Rockford, Ill. 1984) and G. Barany and R. B. Merrifield, The Peptides: Analysis. Synthesis. Biology, editors E. Gross and J. Meienhofer, Vol. 2, (Academic Press, New York, 1980), pp. 3-254, for solid phase peptide synthesis techniques; and M. Bodansky, Principles of Peptide Synthesis, (Springer-Verlag, Berlin 1984) and E. Gross and J. Meienhofer, Eds., The Peptides: Analysis, Synthesis, Biology, Vol. 1, for classical solution synthesis.

The polypeptides of the present invention can also be chemically prepared by other methods such as by the method of simultaneous multiple peptide synthesis. See, e.g., Houghten Proc. Natl. Acad. Sci. USA (1985) 82:5131-5135; U.S. Pat. No. 4,631,211.

### Pharmaceutical preparations

In one embodiment the antigens of the invention are present in pharmaceutical preparations. Generally they are in the form of a polypeptide which can be prepared by the various methods describe herein and known in the art. In some embodiments, the antigens are provided as polynucleotides encoding the antigen.

The HIV clade C envelope polypeptide and the polynucleotides encoding them are useful, for example, as vaccine compositions. Compositions of the invention can be used as vaccine compositions, for example, to enhance or induce an immune response of a subject to a HIV. Immune responses which can be enhanced or induced include both humoral and cell-mediated responses. Compositions comprising particular preparations of HIV clade C envelope molecules can be tested for the ability to induce an immune response using assays well known in the art. For example, a composition can be injected into a laboratory animal, such as a rat or mouse, and the animal can be monitored for the appearance of immune reactants, such as antibodies, directed against the Env polypeptide. Alternatively, assays such as cytotoxic T lymphocyte assays can be performed to determine whether a particular composition of Env molecule is immunogenic.

For use as a vaccine, a composition of the invention comprises a HIV clade C envelope polypeptide or a modified HIV clade C envelope polypeptide as defined in the claims. Vaccine compositions of the invention typically comprise a physiologically compatible carrier. Physiologically compatible carriers are well known to those in the art. Such carriers include, but are not limited to, a simple low salt solution which permits preservation of the integrity of the Env polypeptide, e.g., 10 mM NaCl, 0.1 mM EDTA, or to large, slowly metabolized macromolecules, such as proteins, polysaccharides, polylactic acids, polyglycolic acids, polymeric amino acids, amino acid copolymers, and inactive virus particles. Pharmaceutically acceptable salts can also be used in Env compositions, for example, mineral salts such as hydrochlorides, hydrobromides, phosphates, or sulfates, as well as salts of organic acids such as acetates, proprionates, malonates, or benzoates. Compositions of the invention can also contain liquids, such as water, saline, glycerol, and ethanol, as well as substances such as wetting agents, emulsifying agents, or pH buffering agents.

Various methods can be used to administer an HIV clade C envelope molecule vaccine composition to a human, including intravenous, intramuscular, or subcutaneous injection. Vaccine compositions of the invention are administered at a dose effective to induce an immune response against the Env polypeptide. The particular dosages of the composition used to enhance an immune response will vary, for example, according to the Env compositions being used and the mammal to which the composition is administered. Generally, about 5ug to about 50ug of Env polypeptide per kg of patient body weight, about 50ug to about 5 mg/kg, about 100ug to about 500ug/kg, or about 200 to about 250ug/kg will be administered to an adult human. Such ranges by no means preclude use of a higher or lower amount of a component, as might be warranted in a particular application. For example, the actual dose and schedule may vary depending on whether the compositions are administered in combination with other pharmaceutical compositions, or depending on individual differences in pharmacokinetics, drug disposition, and metabolism.

The antigens and/or adjuvants of the invention can also be delivered using one or more gene vectors, administered via nucleic acid immunization or the like using standard gene delivery protocols. Methods for gene delivery are known in the art. See, e.g., U.S. Pat. Nos. 5,399,346, 5,580,859, 5,589,466. The constructs can be delivered either subcutaneously, epidermally, intradermally, intramuscularly, intravenous, mucosally (such as nasally, rectally and vaginally), intraperitoneally, orally or combinations thereof.

An exemplary replication-deficient gene delivery vehicle that may be used in the practice of the present invention is any of the alphavirus vectors, described in, for example, U.S. Pat. Nos. 6,342,372; 6,329,201 and International Publication WO 01/92552.

A number of viral based systems have been developed for gene transfer into mammalian cells. For example, retroviruses provide a convenient platform for gene delivery systems. Selected sequences can be inserted into a vector and packaged in retroviral particles using techniques known in the art. The recombinant virus can then be isolated and delivered to cells of the subject either in vivo or ex vivo. A number of retroviral systems have been described (U.S. Pat. No. 5,219,740; Miller and Rosman, BioTechniques (1989) 7:980-990; Miller, A. D., Human Gene Therapy (1990) 1:5-14; Scarpa et al., Virology (1991) 180:849-852; Burns et al., Proc. Natl. Acad. Sci. USA (1993) 90:8033-8037; and Boris-Lawrie and Temin, Cur. Opin. Genet. Develop. (1993) 3:102-109.

A number of adenovirus vectors have also been described. Unlike retroviruses which integrate into the host genome, adenoviruses persist extrachromosomally thus minimizing the risks associated with insertional mutagenesis (Haj-Ahmad and Graham, J. Virol. (1986) 57:267-274; Bett et al., J. Virol. (1993) 67:5911-5921; Mittereder et al., Human Gene Therapy (1994) 5:717-729; Seth et al., J. Virol. (1994) 68:933-940; Barr et al., Gene Therapy (1994) 1:51-58; Berkner, K. L. BioTechniques (1988) 6:616-629; and Rich et al., Human Gene Therapy (1993) 4:461-476).

Additionally, various adeno-associated virus (AAV) vector systems, have been developed for gene delivery. AAV vectors can be readily constructed using techniques well known in the art. See, e.g., U.S. Pat. Nos. 5,173,414 and 5,139,941; International Publication Nos. WO 92/01070 (published 23 Jan. 1992) and WO 93/03769 (published 4 Mar. 1993); Lebkowski et al., Molec. Cell. Biol. (1988) 8:3988-3996; Vincent et al. Vaccines 90 (1990) (Cold Spring Harbor Laboratory Press); Carter, B. J. Current Opinion in Biotechnology (1992) 3:533-539; Muzyczka, N. Current Topics in Microbiol. and Immunol. (1992) 158:97-129; Kotin, R. M. Human Gene Therapy (1994) 5:793-801; Shelling and Smith, Gene Therapy (1994) 1:165-169; and Zhou et al., J. Exp. Med. (1994) 179:1867-1875.

Another vector system useful for delivering polynucleotides, mucosally and otherwise, is the enterically administered recombinant poxvirus vaccines described by Small, Jr., P. A., et al. (U.S. Pat. No. 5,676,950, issued Oct. 14, 1997 as well as the vaccinia virus and avian poxviruses. By way of example, vaccinia virus recombinants expressing the genes can be constructed as follows. The DNA encoding the antigens and/or adjuvants of the invention is first inserted into an appropriate vector so that it is adjacent to a vaccinia promoter and flanking vaccinia DNA sequences, such as the sequence encoding thymidine kinase (TK). This vector is then used to transfect cells that are simultaneously infected with vaccinia. Homologous recombination serves to insert the vaccinia promoter plus the gene encoding the coding sequences of interest into the viral genome. The resulting TK recombinant can be selected by culturing the cells in the presence of 5-bromodeoxyuridine and picking viral plaques resistant thereto.

Alternatively, avipoxviruses, such as the fowlpox and canarypox viruses, can also be used to deliver genes encoding the antigens and/or adjuvants of the invention. Recombinant avipox viruses, expressing immunogens from mammalian pathogens, are known to confer protective immunity when administered to non-avian species; The use of an avipox vector is particularly desirable in human and other mammalian species since members of the avipox genus can only productively replicate in susceptible avian species and therefore are not infective in mammalian cells. Methods for producing recombinant avipoxviruses are known in the art and employ genetic recombination, as described above with respect to the production of vaccinia viruses. See, e.g., WO 91/12882; WO 89/03429; and WO 92/03545. Picornavirus-derived vectors can also be used. (See, e.g., U.S. Pat. Nos. 5,614,413 and 6,063,384). Molecular conjugate vectors, such as the adenovirus chimeric vectors described in Michael et al., J. Biol. Chem. (1993) 268:6866-6869 and Wagner et al., Proc. Natl. Acad. Sci. USA (1992) 89:6099-6103, can also be used for gene delivery.

A vaccinia based infection/transfection system can be conveniently used to provide for inducible, transient expression of the coding sequences of interest (for example, sequences encoding the antigens or adjuvants of the invention) in a host cell. In this system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide of interest, driven by a T7 promoter. The polymerase expressed in the cytoplasm from the vaccinia virus recombinant transcribes the transfected DNA into RNA that is then translated into protein by the host translational machinery. The method provides for high level, transient, cytoplasmic production of large quantities of RNA and its translation products. See, e.g., Elroy-Stein and Moss, Proc. Natl. Acad. Sci. USA (1990) 87:6743-6747; Fuerst et al., Proc. Natl. Acad. Sci. USA (1986) 83:8122-8126.

As an alternative approach to infection with vaccinia or avipox virus recombinants, or to the delivery of genes using other viral vectors, an amplification system can be used that will lead to high level expression following introduction into host cells. Specifically, a T7 RNA polymerase promoter preceding the coding region for T7 RNA polymerase can be engineered. Translation of RNA derived from this template will generate T7 DNA polymerase that in turn will transcribe more template. Concomitantly, there will be a cDNA whose expression is under the control of the T7 promoter. Thus, some of the T7 RNA polymerase generated from translation of the amplification template RNA will lead to transcription of the desired gene. Because some T7 RNA polymerase is required to initiate the amplification, T7 RNA polymerase can be introduced into cells along with the template(s) to prime the transcription reaction. The polymerase can be introduced as a protein or on a plasmid encoding the RNA polymerase. For a further discussion of T7 systems and their use for transforming cells, see, e.g., International Publication No. WO 94/26911; Studier and Moffatt, J. Mol. BioL (1986) 189:113-130; Deng and Wolff, Gene (1994) 143:245-249; Gao et al., Biochem. Biophys. Res. Commun. (1994) 200:1201-1206; Gao and Huang, Nuc. Acids Res. (1993) 21:2867-2872; Chen et al., Nuc. Acids Res. (1994) 22:2114-2120; and U.S. Pat. No. 5,135,855.

One or more additional HIV antigens can be used in the composition of this invention. For instance, the HIV clade C env antigens can be used with additional HIV antigens. The additional HIV antigens may be administer simultaneously, prior or after administration of the HIV clad C env antigen.

In addition, an immuno-modulatory factor may be added to the pharmaceutical composition. An "immuno-modulatory factor" refers to a molecule, for example a protein that is capable of modulating an immune response or DNA vectors encoding a given imuno-modulatory factor. Non-limiting examples of immunomodulatory factors include lymphokines (also known as cytokines), such as IL-6, TGF-beta, IL-1, IL-2, IL-3, etc.); and chemokines (e.g., secreted proteins such as macrophage inhibiting factor). Certain cytokines, for example TRANCE, flt-3L, and a secreted form of CD40L are capable of enhancing the immunostimulatory capacity of APCs. Non-limiting examples of cytokines which may be used alone or in combination in the practice of the present invention include, interleukin-2 (IL-2), stem cell factor (SCF), interleukin 3 (IL-3), interleukin 6 (IL-6), interleukin 12 (IL-12), G-CSF, granulocyte macropliage-colony stimulating factor (GM-CSF), interleukin-1 alpha (IL-1.alpha), interleukin-11 (IL-11), MIP-1,gamma., leukemia inhibitory factor (LIF), c-kit ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme (Framingham, Mass.), Amgen (Thousand Oaks, Calif.), R&D Systems and Immunex (Seattle, Wash.). The sequences ofmany of these molecules are also available, for example, from the GenBank database. It is intended, although not always explicitly stated, that molecules having similar biological activity as wild-type or purified cytokines (e.g., recombinantly produced or mutants thereof) and nucleic acid encoding these molecules are intended to be used.

The compositions of the invention will typically be formulated with pharmaceutically acceptable carriers or diluents. As used herein, the term "pharmaceutically acceptable carrier" refers to a carrier for administration of the antigens which does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee et al. (1997) J Microencapsul. 14(2):197-210; O'Hagan et al. (1993) Vaccine 1 (2):149-54. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from E. coli.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of acceptable excipients is available in the well-known Remington's Pharmaceutical Sciences.

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

### Administration

The compositions disclosed herein can be administered to a subject to generate an immune response. Preferably, the composition can be used as a vaccine to treat or prevent HIV infection.

As used herein, "subject" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

The compositions will include "immunologically effective amounts" of HIV antigen i.e. amounts sufficient to raise a specific immune response or, more preferably, to treat, reduce, or prevent HIV infection. An immune response can be detected by looking for antibodies to the HIV antigen used (e.g. IgG or IgA) in patient samples (e.g. in blood or serum, in mesenteric lymph nodes, in spleen, in gastric mucosa, and/or in feces). The precise effective amount for a given patient will depend upon the patient's age, size, healtb, the nature and extent of the condition, the precise composition selected for administration, the patient's taxonomic group, the capacity of the patient's immune system to synthesize antibodies, the degree of protection desired, the formulation of the vaccine, the treating physician's assessment of the medical situation, and other relevant factors. Thus, it is not useful to specify an exact effective amount in advance, but the amount will fall in a relatively broad range that can be determined through routine trials, and is within the judgment of the clinician. An effective dose will typically be from about 0.01 mg/kg to 50 mg/kg in the individual to which it is administered.

### EXAMPLES

### Example 1. V-loop deleted SHIVenvC induces cross-clade nAb responses in mice.

A V-loop deleted **SHIV-1157ip*env*** expression vector was prepared as follows: the entire SHIV-1157ip gp160 sequence was codon-optimized and cloned into the pTPS mutagenesis vector (Xu et al., 2002). Mutagenic primers were used to separately delete the V1/V2 loops and the V3 loop. The mutagenic primer were synthesized and included linker sequences GAG to maintain the Env core structure (Johnson et al., 2002). Mutagenesis was confirmed by DNA sequencing and the mutant gp160 gene was cloned into the pJW4303 expression vector. The resulting plasmid, pJW1157ip*env*Δ123 was tested for purity by restriction digest/gel electrophoresis and for in vitro expression by transfection into 293T cells followed by Western blot analysis. The Western blot analysis showed a protein band of approximately 135 kb.

A recombinant vaccinia virus was made expressing 1157ip*env*Δ123 and used to infect the human osteosarcoma cell line, 143 B. SHIV-1157ipΔ123 Env was prepared from cell lysates by size-exclusion and lentil-lectin chromatography (Earl et al.,1994).

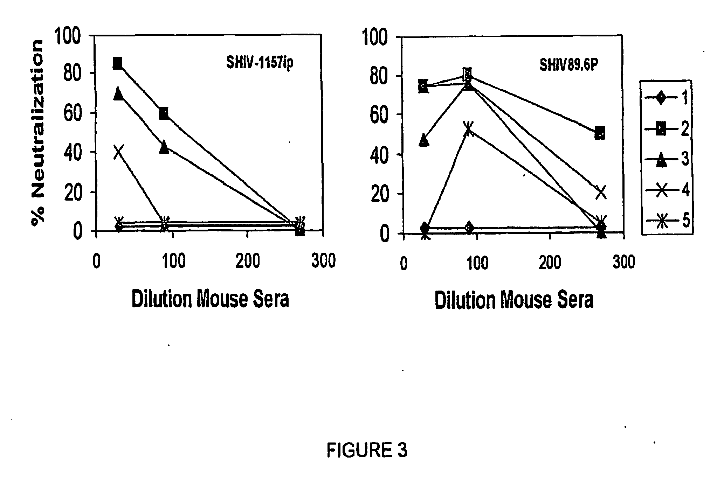

Five BALB/c mice were inoculated subcutaneously 2x with 20 ug of SHIV-1157ipΔ123 Env formulated in MPL + TDM emulsion. Sera from immunized mice were tested for neutralizing antibody activity against homologous SHIV-1157ip and against heterologous SHIV89.6P (Fig 3). Three of 5 mice showed neutralizing antibody activity against SHIV-1157ip and 4 of the 5 mice showed neutralizing antibody activity against heterologous SHIV89.6P clade B.

The results demonstrate that immunization with a multiply V-loop deleted clade C Env, prepared from the full-length gp160 genome by methodology that maintains structural integrity of the multimeric form of the Env glycoprotein, is immunogenic and can induce cross-clade nAb responses.

### Example 2: Construction and Adaptation of SHIV-1157i to Rhesus Macaques.

A biologically relevant challenge virus was created to evaluate anti-HIV clade C immune prophylaxis.

### SHIV-1157i construction.

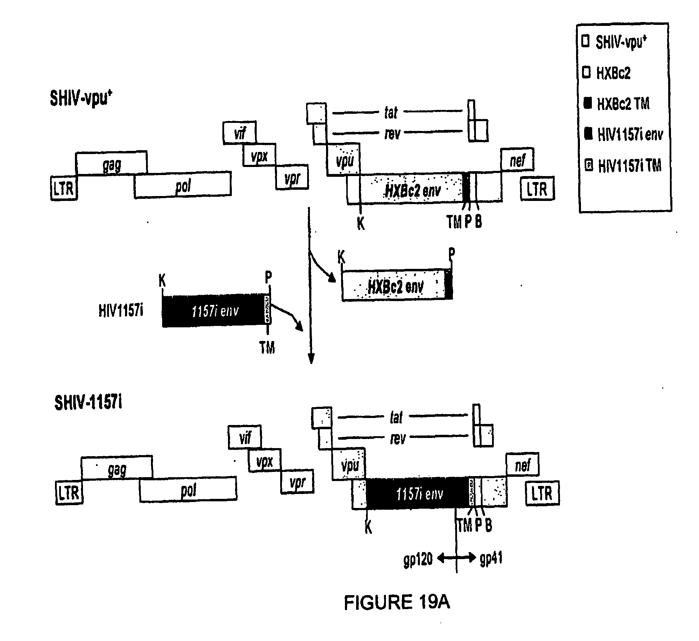

SHIV-1157i was built upon the backbone of SHIV-vpu⁺ (Li et al., J. Virol.(1995), 69; 7061-67) (Fig. 19a) and contained most of gp120 as well as the entire extracellular domain and transmembrane region of gp41 of the primary, original HIV1157i isolate. The HIV1157i isolate is a recently transmitted pediatric HIV clade C isolate from a six-month-old Zambian infant.

The initial cell-free SHIV-1157i stock was generated by transfecting 293T cells with proviral DNA and harvesting the supernatants. The virus replicated in human and rhesus monkey PBMC and in Ghost.CD4.CCR5.GFP cells but did not infect CEMx174 or CEMx174.GFP cells, which do not express CCR5, indicating that SHIV-1157i, like the original HIV1157i, uses CCR5 as coreceptor.

### Adaptation of SHIV-1157i to rhesus macaques

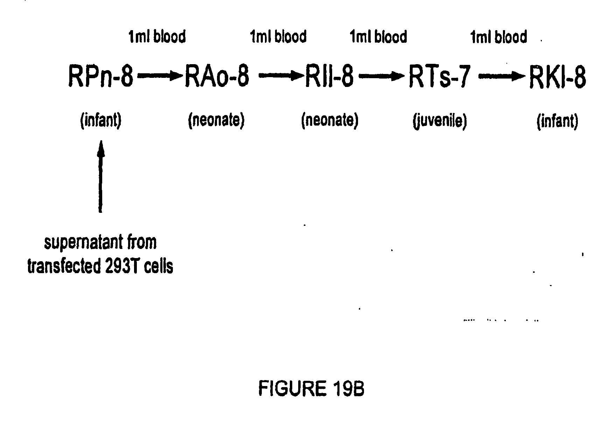

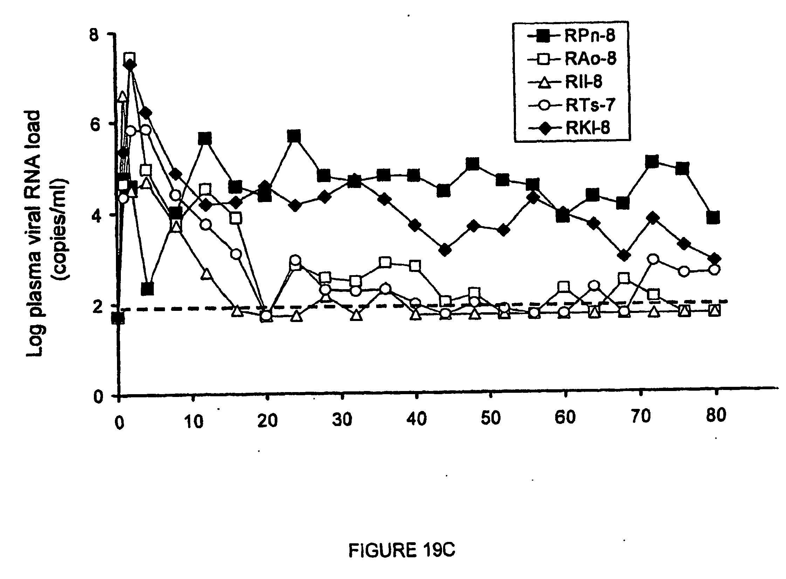

To adapt SHIV-1157i to rhesus macaques, infant rhesus monkey RPn-8 was inoculated intravenously (i.v.) with 6 ml of virus. Real-time RT-PCR revealed that SHIV-1157i replicated in this animal; peak viremia reached 6 x 10⁴ RNA copies/ml at week 4 post-inoculation (p.i.). This first animal never cleared the virus and remained viremic throughout. Four additional animals were subjected to serial blood transfer (Fig. 19b), whereby 1 ml of whole blood collected at peak viremia (week 2 p.i.) was directly transferred into the following monkey recipients: neonates RAo-8 and RIl-8, juvenile RTs-7 and infant RKl-8. Compared to the first virus recipient RPn-8, peak viral RNA loads increased between 1 to 2 logs in subsequent recipients (Fig. 19c). All five monkeys in this cohort seroconverted by week 4. At week 6 p.i., the passaged virus, termed SHIV-1157ip, was isolated from PBMC of the last recipient, RKl-8, and a large stock was grown in rhesus monkey PBMC. Virus from this stock was replication-competent in PBMC of other naive rhesus monkey donors.

SHIV-1157ip replicated neither in CEMx174 or CEMx174-GFP cells nor in U87.CD4, or U87.CD4 cells expressing CCR1, CCR2, CCR3, or CXCR4. SHIV-1157ip also failed to replicate in Ghost-BOB and Ghost-Bonzo cells. Productive infection was only observed in U87.CD4.CCR5 cells, indicating that SHIV-1157ip exclusively used CCR5 as coreceptor, even after several passages in a different species.

### Example 3. Pathogenicity of SHIV-1157ip.

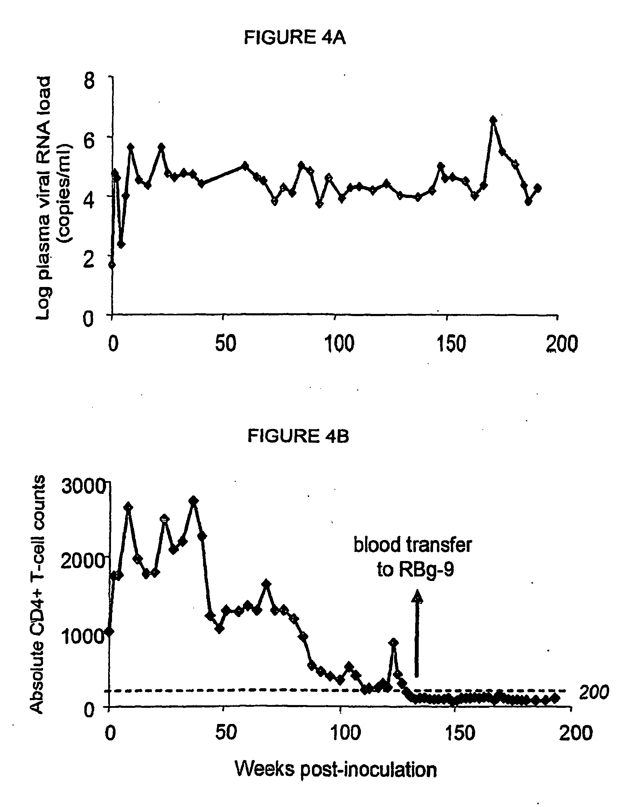

Signs of early disease (depletion of memory T cells in peripheral blood and persistently low CD4⁺/CD8⁺ ratios) were observed in two monkeys, RPn-8 and RKl-8, within the first year post-inoculation. This was followed by depletion of absolute CD4⁺ T cells in peripheral blood. In animal RPn-8, AIDS, as defined by CD4⁺ T cells persistently <200 cells/µl, developed at week 135 p.i.; in two other animals, RKl-8 and RTs-7, absolute CD4⁺ T-cell counts have persistently been <500 cells/µl (Fig. 4). As an additional sign of lentivirus-induced disease, severe, persistent thrombocytopenia has developed in animal RPn-8, although no bleeding has been observed to date.

In order to determine whether a more aggressive virus had evolved in animal RPn-8 after it had developed AIDS, 10 ml of blood was transfused into a naïve recipient, RBg-9. Three months later, this recipient animal developed thrombocytopenia and depletion of peripheral blood memory T cells. Both of these abnormalities have persisted over the ensuing 18 months. Both donor and recipient have remained persistently viremic.

### Example 4. Construction and Mucosal Trans-mission of SHIV-1157ipd3N4.

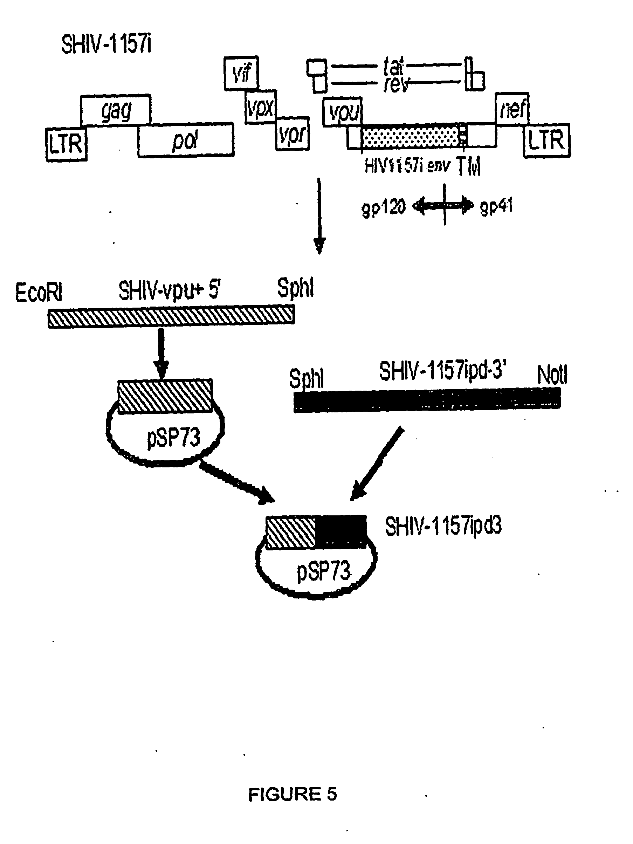

After the first SHIV-1157i-infected monkey (RPn-8) had progressed to AIDS, the virus was re-isolated in order to generate another molecular clone. The re-isolated virus, termed SHIV-1157ipd, was isolated four weeks after animal RPn-8's absolute CD4⁺ T-cell count dropped to <200 cells/µl. Clone 3 gave rise to the highest level of infectious virus and was selected for further studies (Figs. 4, 5). Genetic analysis of SHIV-1157ipd env sequences revealed a number of mutations that resulted in single amino acid substitutions in gp120, especially in V1, V2 and V4. Mutations were also observed in gp41. Five out of five clones sequenced contained a 118-bp deletion in the 3' end of env. This 118-bp deletion was located in the intracellular domain of gp41 and led to a loss of 35 of the original amino acids (aa), and due to a frame shift, a gain of 57 aa. The envelope remained infectious.

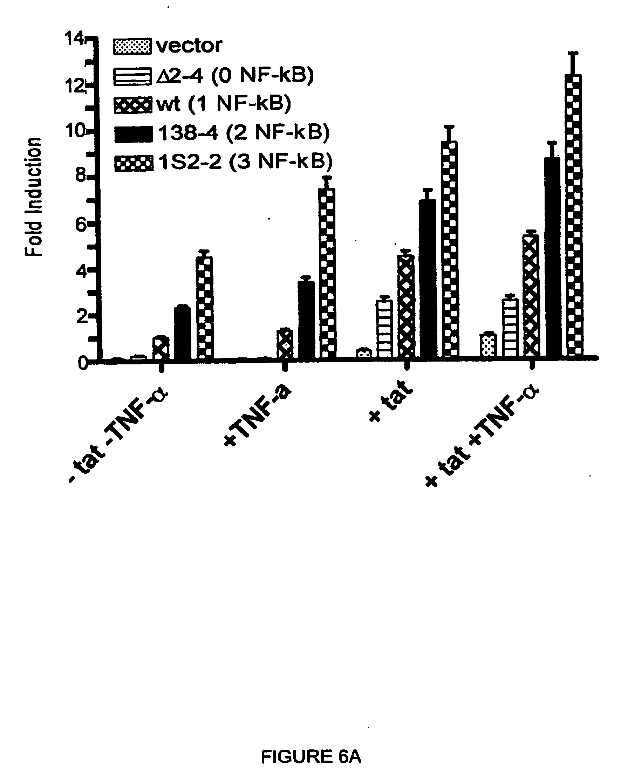

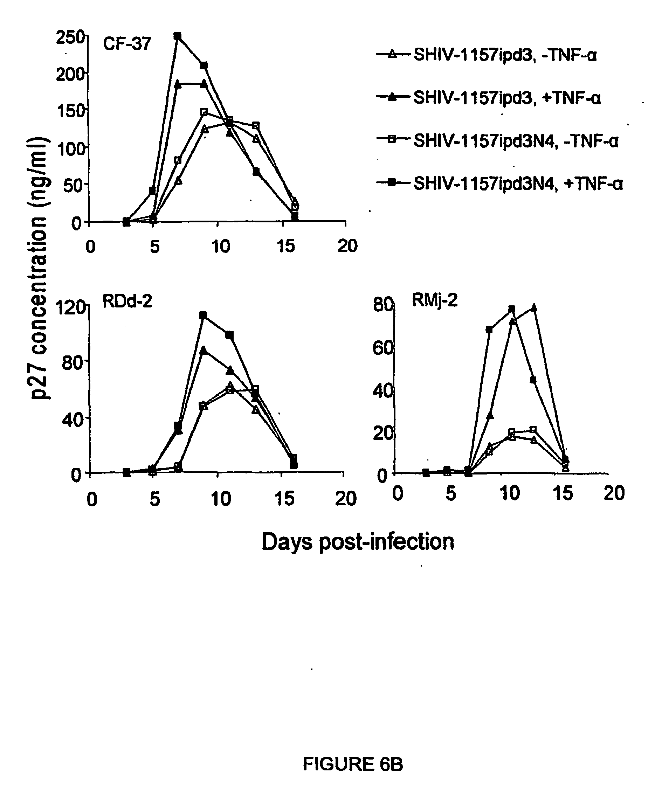

In order to maximize the replicative capacity and enhance viral loads in vivo, transcriptional control elements in the lentiviral LTR were added. Thus, the LTR of SHIV-1157ipd3 was modified to increase the number of NF-κB sites. In a set of solo-LTR constructs linked to the luciferase reporter gene, a direct correlation between LTR-mediated gene expression and the number of NF-κB sites in transient transfection assays was demonstrated (Fig. 6a). The effect was especially pronounced when a tat expression plasmid was cotransfected and TNF-a was added to the medium, consistent with the signal transduction cascade activated by this cytokine. After transfection and release of infectious virus, the modifications engineered into the 3' LTR will be copied into the 5' LTR in the subsequent viral replication cycle. The replication kinetics of late-stage viruses encoding two NF-κB sites produced higher levels of p27 Gag production and/or earlier peaks to stimulation with TNF-α than virus containing only one site (Fig. 6b).

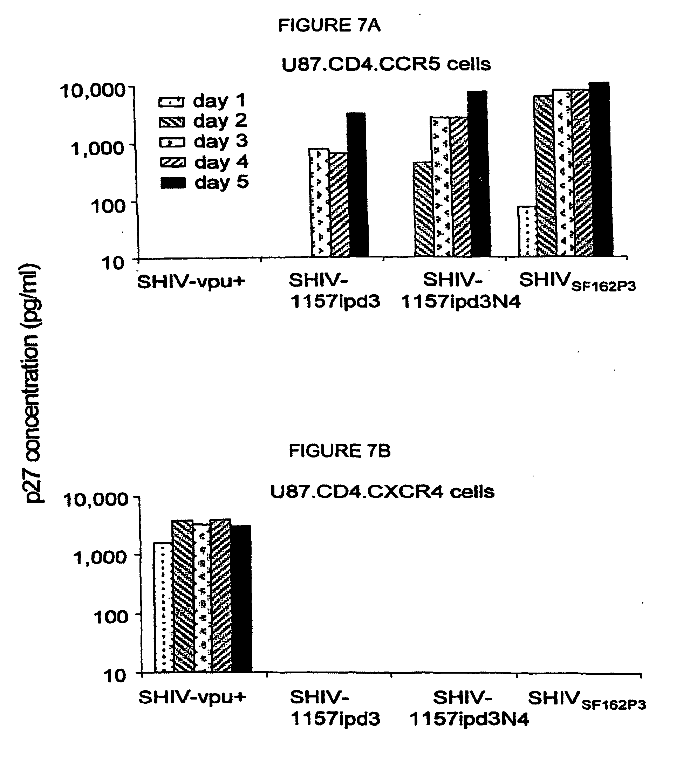

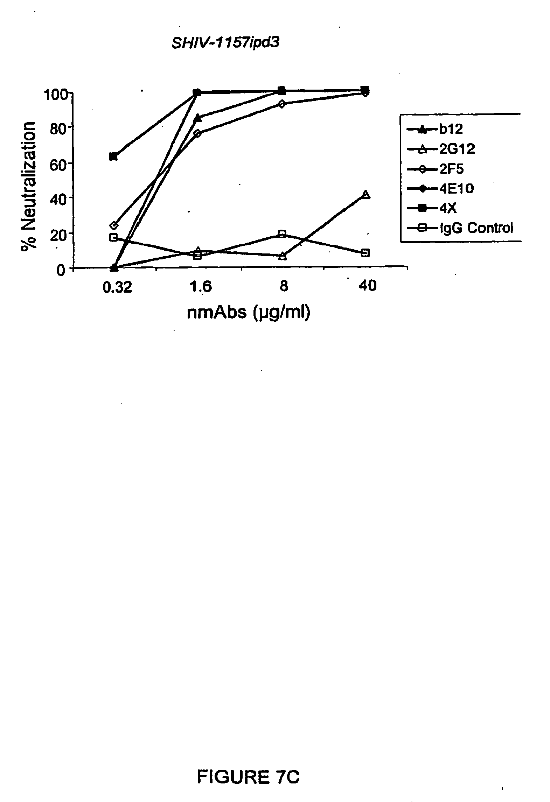

The SHIV-1157ipd3 virus replicated neither in CEMx 174 or CEMx 174-GFP cells nor in U87.CD4, U87.CD4.CCR1, U87.CD4.CCR2, U87.CD4.CCR3, U87.CD4.CXCR4, GHOST-BOB and GHOST-BONZO cells. Productive infection occurred only in U87.CD4.CCR5 cells (Fig. 7a), indicating that late-stage SHIV-1157ipd3 and its 2 NF-κB-site counterpart, SHIV-1157ipd3N4, still use CCR5 as coreceptor exclusively.

In order to determine whether late virus was susceptible to neutralization by the broadly reactive human nmAbs b12, 2G12, 2F5 and 4E10 (Fig. 7b) assays were performed in human PBMC. The late virus had lost its sensitivity to 2G12 which was correlated with the loss of a key N-linked glycosylation site at aa position 295 that is required for forming the complex 2G12 epitope (Scanlan et al., 2002); the 2G12 nmAb is unique in that it is targeted to a pure carbohydrate structure that consists of several mannan residues. Nevertheless, the late SHIV-1157ipd3N4 had not developed global neutralization resistance.

Thus, several SHIVenvC strains were prepared that encode one of various forms of the *env* gene that was originally cloned from the R5 HIV1157i, a clade C strain. This virus was isolated from a Zambian child who turned out to be a long-term non-progressor (LTNP) during the prospective follow-up. The SHIV constructs and their designations are as follows:
- SHIV-1157i infectious molecular clone, not yet adapted to rhesus monkeys; "i" designates a virus strain (or env gene) isolated from an infant
- SHIV-1157ip biological isolate obtained after passage through several rhesus monkeys; "p" designates a passaged, monkey-adapted virus
- SHIV-1157ipd biological isolate, "d" indicates that the virus was re-isolated from an infected monkey with disease
- SHIV-1157ipd3 infectious molecular clone #3. The 3' half of the provirus was derived from a monkey with virus-induced disease
- SHIV-1157ipd3N4 identical to SHIV-1157ipd3 except that each LTR has 2 instead of the usual 1 NF-κB sites.

### Example 5. Testing vaccination in animals challenged with SHIV-1157ipd3N4.

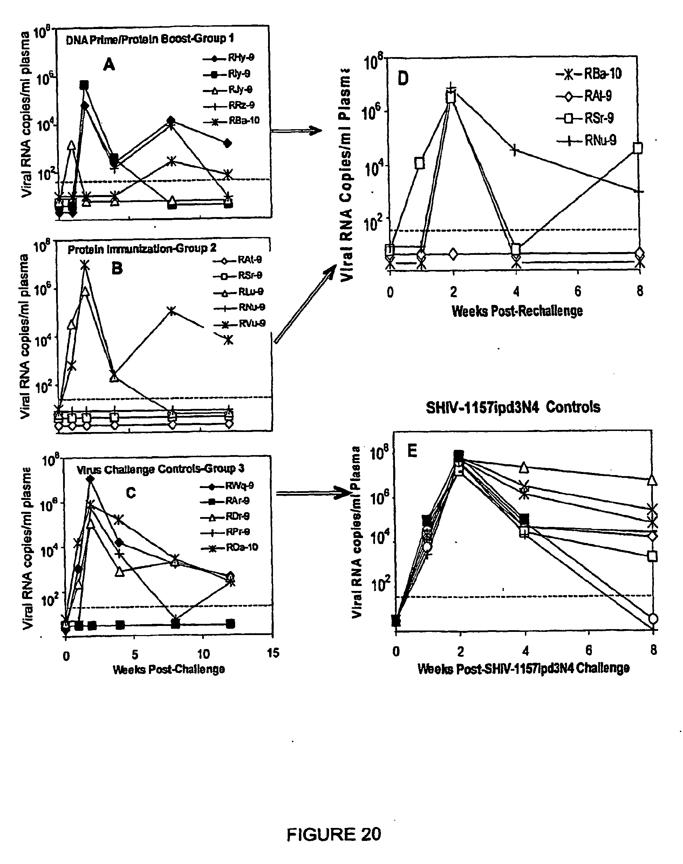

Two groups of newborn macaques were vaccinated (Group 1 and Group 3). Group 1 animals were given a combination of DNA expression plasmids encoding *SIV gag-proteasel, HIV tat* and HIV1084i*env*. Group 3 animals (controls) were given empty vector DNA. DNA was given at 2 time points - at birth and at 6 weeks. Animals were rested and then given soluble protein boosts consisting of SIV Gag-Pol particles derived from recombinant vaccinia virus infected cells, HIV Tat and HIV 1084i gp160. Age-matched Group 2 animals were given soluble proteins with no DNA priming. Proteins were given at month 9, month 11 and at month 24. Cellular and humoral immunity were assessed 2 weeks after each vaccination time point. The animals were challenged orally with SHIV-1157ip (Fig. 20, panels A, B and C). All RM received a relatively low-dose of 3.7 AID₅₀ which could account for less than 100% infection among controls. In vitro, CD8⁺ T cell-depleted PBMC from RM that had remained uninfected after oral challenge, were fully susceptible to SHIV-1157ip and SHIV-1157ipd3N4 infection, thus ruling out inherent resistance to these viruses.

Because some vaccinees appeared to have contained SHIV-1157ip, they were re-challenged i.r. with 20 AID₅₀ of SHIV-1157ipd3N4. The rechallenged animals included one animal from Group 1 (DNA prime/protein boost), 3 animals from Group 2 (protein immunization alone), and one animal from the control group, RAr-9. Plasma vRNA levels for RAr-9 after rechallenge (Fig. 20E, red) and for a group of 8 additional non-vaccinated control animals identically challenged with SHIV-1157ipd3N4 are shown. By week 1, all control animals were infected, and peak viremia levels ranged from 1.3 - 7.7 x 10⁷ vRNA copies/ml. In contrast, only 1 of 4 previously vaccinated RM had detectable virus by wk 1 (Fig. 20D). Of those that became infected, peak viremia levels were a log lower than in controls (p=0.003). Importantly, one of the protein-only vaccinated animals, RAt-9, still had no evidence of infection after rechallenge with SHIV-1157ipd3N4. At the time of rechallenge, this RM had strong cellular immunity to SIV Gag and HIV Tat.

To conserve RM, the rechallenge described was piggybacked with i.r. SHIV-1157ipd3N4 challenge of the vaccinees. All RM in the 3 vaccine groups became infected, but mean peak viremia levels for animals of the 3 vaccine groups combined were significantly less than controls (2.4 x 10⁷ vs 4.0 x 10⁷ vRNA copies/ml; p = 0.006). Mean plasma viremia levels for the Group 3 RM alone that were DNA primed with clade C *env* and boosted with haterologous clade B gp160 were the lowest at 1.5 x 10⁷ RNA copies/ml (p = 0.014).

## Claims

1. An isolated clade C HIV envelope polypeptide comprising the amino acid sequence of SEQ ID NO:2.

2. A modified clade C HIV envelope polypeptide in which the V1/V2 region has been deleted, said modified polypeptide comprising the amino acid sequence of SEQ ID NO:4.

3. A modified clade C HIV envelope polypeptide in which the V1/V2 and V3 regions have been deleted, said modified polypeptide comprising the amino acid sequence of SEQ ID NO:6.

4. A modified clade C HIV envelope polypeptide in which the V1/V2, V3, V4 regions have been deleted, said modified polypeptide comprising the amino acid sequence of SEQ ID NO:8.

5. An isolated polynucleotide comprising the nucleotide sequence of SEQ ID NO:1, which encodes the polypeptide of claim 1.

6. An isolated polynucleotide comprising the nucleotide sequence of SEQ ID NO:3, which encodes the polypeptide of claim 2.

7. An isolated polynucleotide comprising the nucleotide sequence of SEQ ID NO:5, which encodes the polypeptide of claim 3.

8. An isolated polynucleotide comprising the nucleotide sequence of SEQ ID NO:7, which encodes the polypeptide of claim 4.

9. A construct comprising the polynucleotide of anyone of claims 5-8.

10. A composition comprising the polypeptide of any one of claims 1-4 or the polynucleotide of any one of claims 5-8, and a pharmaceutically acceptable carrier.

11. A composition according to claim 10 for inducing an immune response in a subject.

12. A composition according to claim 10 for the manufacture of a medicament for inducing an immune response in a subject.

13. A composition for use as defined in claim 11 or claim 12, wherein the composition or medicament is to be administered with a second HIV antigen.

14. A composition for use as defined in claim 13, wherein said second HIV antigen is gp120 or gp160.

15. An HIV recombinant viral particle comprising an envelope region which has the amino acid sequence of SEQ ID NO:10 or is encoded by the nucleotide sequence of SEQ ID NO:9.

16. The recombinant viral particle of claim 15, wherein the viral particle further comprises the Nef amino acid sequence of SEQ ID NO:11.

17. The recombinant viral particle of claim 15 or claim 16, comprising two or more NF-kB regions.

18. The recombinant viral particle of claim 17, comprising three or more NF-kB regions.

19. A composition comprising the recombinant viral particle of any one of claims 15-18.

## Patentansprüche

1. Isoliertes Clade C HIV Hüllenpolypeptid, umfassend die Aminosäuresequenz von SEQ ID NO:2.

2. Modifiziertes Clade C HIV Hüllenpolypeptid, in dem die V1/V2-Region deletiert wurde, wobei das modifizierte Polypeptid die Aminosäuresequenz von SEQ ID NO:4 umfasst.

3. Modifiziertes Clade C HIV Hüllenpolypeptid, in dem die V1/V2- und die V3-Regionen deletiert wurden, wobei das modifizierte Polypeptid die Aminosäuresequenz von SEQ ID NO:6 umfasst.

4. Modifiziertes Clade C HIV Hüllenpolypeptid, in dem die V1/V2-, V3-, V4-Regionen deletiert wurden, wobei das modifizierte Polypeptid die Aminosäuresequenz von SEQ ID NO:8 umfasst.

5. Isoliertes Polynukleotid, umfassend die Nukleotidsequenz von SEQ ID NO:1, die das Polypeptid nach Anspruch 1 codiert.

6. Isoliertes Polynukleotid, umfassend die Nukleotidsequenz von SEQ ID NO:3, die das Polypeptid nach Anspruch 2 codiert.

7. Isoliertes Polynukleotid, umfassend die Nukleotidsequenz von SEQ ID NO:5, die das Polypeptid nach Anspruch 3 codiert.

8. Isoliertes Polynukleotid, umfassend die Nukleotidsequenz von SEQ ID NO:7, die das Polypeptid nach Anspruch 4 codiert.

9. Konstrukt, umfassend das Polynukleotid nach einem der Ansprüche 5 - 8.

10. Zusammensetzung, umfassend das Polypeptid nach einem der Ansprüche 1 - 4 oder das Polynukleotid nach einem der Ansprüche 5 - 8 und eine pharmazeutisch annehmbare Trägersubstanz.

11. Zusammensetzung nach Anspruch 10, um eine Immunreaktion in einer Person hervorzurufen.

12. Zusammensetzung nach Anspruch 10 zur Herstellung eines Arzneimittels, um eine Immunreaktion in einer Person hervorzurufen.

13. Zusammensetzung zur Verwendung wie in Anspruch 11 oder Anspruch 12 definiert, wobei die Zusammensetzung oder das Arzneimittel mit einem zweiten HIV-Antigen verabreicht werden muss.

14. Zusammensetzung zur Verwendung wie in Anspruch 13 definiert, wobei das zweite HIV-Antigen gp120 oder gp160 ist.

15. Rekombinanter viraler HIV-Partikel, umfassend einen Hüllenbereich, der die Aminosäuresequenz von SEQ ID NO:10 aufweist oder von der Nukleotidsequenz von SEQ ID NO: 9 codiert ist.

16. Rekombinanter viraler Partikel nach Anspruch 15, wobei der virale Partikel weiter die nef-Aminosäuresequenz von SEQ ID NO:11 umfasst.

17. Rekombinanter viraler Partikel nach Anspruch 15 oder Anspruch 16, umfassend zwei oder mehrere NF-kB-Regionen.

18. Rekombinanter viraler Partikel nach Anspruch 17, umfassend drei oder mehrere NF-kB-Regionen.

19. Zusammensetzung, umfassend den rekombinanten viralen Partikel nach einem der Ansprüche 15 - 18.

## Revendications

1. Polypeptide d'enveloppe HIV C à revêtement isolé comprenant la séquence d'acide aminé de la SEQ. ID N°2.

2. Polypeptide d'enveloppe HIV C à revêtement isolé, dans lequel la zone V1/V2 a été effacée, ledit polypeptide modifié comprenant la séquence d'acide aminé de la SEQ. ID N°4.

3. Polypeptide d'enveloppe HIV C à revêtement isolé, dans lequel les zones V1/V2 et V3 ont été effacées, ledit polypeptide modifié comprenant la séquence d'acide aminé de la SEQ. ID N°6.

4. Polypeptide d'enveloppe HIV C à revêtement isolé, dans lequel les zones V1/V2, V3, V4 ont été effacées, ledit polypeptide modifié comprenant la séquence d'acide aminé de la SEQ. ID N°8.

5. Polynucléotide isolé comprenant la séquence nucléotidique de la SEQ. ID N°1, qui code le polypeptide selon la revendication 1.

6. Polynucléotide isolé comprenant la séquence nucléotidique de la SEQ. ID. N°3, qui code le polypeptide selon la revendication 2.

7. Polynucléotide isolé comprenant la séquence nucléotidique de la SEQ. ID. N°5, qui code le polypeptide selon la revendication 3.

8. Polynucléotide isolé comprenant la séquence nucléotidique de la SEQ. ID. N°7, qui code le polypeptide selon la revendication 4.

9. Constituant comprenant le polynucléotide selon l'une quelconque des revendications 5-8.

10. Composition comprenant le polypeptide selon l'une quelconque des revendications 1-4 ou le polynucléotide selon l'une quelconque des revendications 5-8 et un excipient pharmaceutiquement acceptable.

11. Composition selon la revendication 10, pour induire une réponse immune chez un sujet.

12. Composition selon la revendication 10, pour la fabrication d'un médicament destiné à induire une réponse immune chez un sujet.

13. Composition à utiliser comme défini dans la revendication 11 ou la revendication 12, dans laquelle la composition ou le médicament doit être administré avec un second antigène HIV.

14. Composition à utiliser comme défini dans la revendication 13, dans laquelle ledit second antigène HIV est gp120 ou gp160.

15. Particule virale recombinante HIV comprenant une zone d'enveloppe ayant la séquence d'acide aminé de la SEQ. ID N°10 ou codée par la séquence nucléotidique de la SEQ. ID N°9.

16. Particule virale recombinante selon la revendication 15, dans laquelle la particule virale comprend en outre la séquence d'acide aminé Nef de la SEQ. ID N°11.

17. Particule virale recombinante selon la revendication 15 ou 16, comprenant deux zones NF-kB ou plus.

18. Particule virale recombinante selon la revendication 17, comprenant trois zones NF-kB ou plus.

19. Composition comprenant la particule virale recombinante selon l'une des revendications 15-18.
